# EUROPEAN PATENT SPECIFICATION

(11) **EP 3 068 877 B1**
(45) Date of publication and mention of the grant of the patent: **09.06.2021**
(21) Application number: 14799418.0
(22) Date of filing: 14.11.2014
(51) Int. Cl.: C12N 9/36, C12N 9/50

(54) **MODIFIED KZ144 ENDOLYSIN SEQUENCE**
MODIFIZIERTE KZ144-ENDOLYSINSEQUENZ
SÉQUENCE D'ENDOLYSINE KZ144 MODIFIÉE

(30) Priority: 14.11.2013 WO PCT/EP2013/073869
(43) Date of publication of application: 21.09.2016
(73) Proprietor: Lysando AG, 9497 Triesenberg (LI)
(72) Inventor: MILLER, Stefan, 93055 Regensburg (DE); STERNER, Reinhard, 93055 Regensburg (DE); STÜER, Heike, 93080 Pentling (DE)
(74) Representative: Rückerl, Florian
(86) International application number: PCT/EP2014/074671
(87) International publication number: WO 2015/071436

(56) References cited:
- WO-A2-2010/149792
- YVES BRIERS ET AL: "Muralytic activity and modular structure of the endolysins of Pseudomonas aeruginosa bacteriophages ?KZ and EL", MOLECULAR MICROBIOLOGY, vol. 65, no. 5, 1 September 2007 (2007-09-01), pages 1334-1344, XP055119325, ISSN: 0950-382X, DOI: 10.1111/j.1365-2958.2007.05870.x
- BRIERS Y ET AL: "The high-affinity peptidoglycan binding domain of Pseudomonas phage endolysin KZ144", BIOCHEMICAL AND BIOPHYSICAL RESEARCH COMMUNICATIONS, ACADEMIC PRESS INC. ORLANDO, FL, US, vol. 383, no. 2, 29 May 2009 (2009-05-29), pages 187-191, XP026057388, ISSN: 0006-291X, DOI: 10.1016/J.BBRC.2009.03.161 [retrieved on 2009-04-05]
- A. FOKINE ET AL: "Structure of the Bacteriophage KZ Lytic Transglycosylase gp144", JOURNAL OF BIOLOGICAL CHEMISTRY, vol. 283, no. 11, 14 March 2008 (2008-03-14), pages 7242-7250, XP055130395, ISSN: 0021-9258, DOI: 10.1074/jbc.M709398200
- LOPEZ R ET AL: "Enzymes for anti-infective therapy: phage lysins", DRUG DISCOVERY TODAY: THERAPEUTIC STRATEGIES, ELSEVIER, AMSTERDAM, NL, vol. 1, no. 4, 1 December 2004 (2004-12-01), pages 469-474, XP004694307, ISSN: 1740-6773, DOI: 10.1016/J.DDSTR.2004.09.002

### SEQUENCE LISTING

<110> Lysando AG
<120> Modified KZ144 endolysin sequence
<130> LYS-021 PCT 1
<150> PCT/EP2013/073869
   <151> 2013-11-14
<160> 152
<170> PatentIn version 3.5
<210> 1
   <211> 260
   <212> PRT
   <213> Artificial
<220>
   <223> Consensus Sequence
<220>
   <221> MISC_FEATURE
   <222> (1)..(1)
   <223> Xaa can be any naturally occurring amino acid or absent; in particular it can be methionine
<220>
   <221> MISC_FEATURE
   <222> (14)..(14)
   <223> Xaa can be any naturally occurring amino acid, in particular serine, arginine or aspargine
<220>
   <221> MISC_FEATURE
   <222> (23)..(23)
   <223> Xaa can be any naturally occurring amino acid, in particular serine, arginine or aspargine
<220>
   <221> MISC_FEATURE
   <222> (50)..(50)
   <223> Xaa can be any naturally occurring amino acid, in particular serine, arginine or aspargine
<220>
   <221> MISC_FEATURE
   <222> (82)..(82)
   <223> Xaa can be any naturally occurring amino acid, in particular threonine or isoleucine
<220>
   <221> MISC_FEATURE
   <222> (122)..(122)
   <223> Xaa can be any naturally occurring amino acid, in particular isoleucine or methionine
<220>
   <221> MISC_FEATURE
   <222> (149)..(149)
   <223> Xaa can be any naturally occurring amino acid, in particular methionine or proline
<220>
   <221> MISC_FEATURE
   <222> (154)..(154)
   <223> Xaa can be any naturally occurring amino acid, in particular leucine or threonine
<220>
   <221> MISC_FEATURE
   <222> (160)..(160)
   <223> Xaa can be any naturally occurring amino acid, in particular alanine or threonine
<220>
   <221> MISC_FEATURE
   <222> (167)..(167)
   <223> Xaa can be any naturally occurring amino acid, in particular isoleucine or leucine
<220>
   <221> MISC_FEATURE
   <222> (179)..(179)
   <223> Xaa can be any naturally occurring amino acid, in particular asparagine or phenylalanine
<220>
   <221> MISC_FEATURE
   <222> (180)..(180)
   <223> Xaa can be any naturally occurring amino acid, in particular methionine or glutamic acid
<220>
   <221> MISC_FEATURE
   <222> (186)..(186)
   <223> Xaa can be any naturally occurring amino acid, in particular valine or tyrosine
<220>
   <221> MISC_FEATURE
   <222> (206)..(206)
   <223> Xaa can be any naturally occurring amino acid, in particular alanine, asparagine or valine
<220>
   <221> MISC_FEATURE
   <222> (212)..(212)
   <223> Xaa can be any naturally occurring amino acid, in particular threonine or asparagine
<220>
   <221> MISC_FEATURE
   <222> (224)..(224)
   <223> Xaa can be any naturally occurring amino acid, in particular proline or glutamine
<220>
   <221> MISC_FEATURE
   <222> (230)..(230)
   <223> Xaa can be any naturally occurring amino acid, in particular asparagine or tyrosine
<220>
   <221> MISC_FEATURE
   <222> (232)..(232)
   <223> Xaa can be any naturally occurring amino acid, in particular serine or threonine
<400> 1
   Xaa Lys Val Leu Arg Lys Gly Asp Arg Gly Asp Glu Val Xaa Gln Leu
   1               5                   10                  15
   Gln Thr Leu Leu Asn Leu Xaa Gly Tyr Asp Val Gly Lys Pro Asp Gly
               20                  25                  30
   Ile Phe Gly Asn Asn Thr Phe Asn Gln Val Val Lys Phe Gln Lys Asp
           35                  40                  45
   Asn Xaa Leu Asp Ser Asp Gly Ile Val Gly Lys Asn Thr Trp Ala Glu
       50                  55                  60
   Leu Phe Ser Lys Tyr Ser Pro Pro Ile Pro Tyr Lys Thr Ile Pro Met
   65                  70                  75                  80
   Pro Xaa Ala Asn Lys Ser Arg Ala Ala Ala Thr Pro Val Met Asn Ala
                   85                  90                  95
   Val Glu Asn Ala Thr Gly Val Arg Ser Gln Leu Leu Leu Thr Phe Ala
               100                 105                 110
   Ser Ile Glu Ser Ala Phe Asp Tyr Glu Xaa Lys Ala Lys Thr Ser Ser
           115                 120                 125
   Ala Thr Gly Trp Phe Gln Phe Leu Thr Gly Thr Trp Lys Thr Met Ile
       130                 135                 140
   Glu Asn Tyr Gly Xaa Lys Tyr Gly Val Xaa Thr Asp Pro Thr Gly Xaa
   145                 150                 155                 160
   Leu Arg Lys Asp Pro Arg Xaa Ser Ala Leu Met Gly Ala Glu Leu Ile
                   165                 170                 175
   Lys Glu Xaa Xaa Asn Ile Leu Arg Pro Xaa Leu Lys Arg Glu Pro Thr
               180                 185                 190
   Asp Thr Asp Leu Tyr Leu Ala His Phe Phe Gly Pro Gly Xaa Ala Arg
           195                 200                 205
   Arg Phe Leu Xaa Thr Gly Gln Asn Glu Leu Ala Ala Thr His Phe Xaa
       210                 215                 220
   Lys Glu Ala Gln Ala Xaa Pro Xaa Ile Phe Tyr Asn Lys Asp Gly Ser
   225 230 235 240
   Pro Lys Thr Ile Gln Glu Val Tyr Asn Leu Met Asp Gly Lys Val Ala
   245 250 255
   Ala His Arg Lys
   260
<210> 2
   <211> 259
   <212> PRT
   <213> unknown
<220>
   <223> KZ144 endolysin without N-terminal methionine
<400> 2
   Lys Val Leu Arg Lys Gly Asp Arg Gly Asp Glu Val Cys Gln Leu Gln
   1 5 10 15
   Thr Leu Leu Asn Leu Cys Gly Tyr Asp Val Gly Lys Pro Asp Gly Ile
   20 25 30
   Phe Gly Asn Asn Thr Phe Asn Gln Val Val Lys Phe Gln Lys Asp Asn
   35 40 45
   Cys Leu Asp Ser Asp Gly Ile Val Gly Lys Asn Thr Trp Ala Glu Leu
   50 55 60
   Phe Ser Lys Tyr Ser Pro Pro Ile Pro Tyr Lys Thr Ile Pro Met Pro
   65 70 75 80
   Thr Ala Asn Lys Ser Arg Ala Ala Ala Thr Pro Val Met Asn Ala Val
   85 90 95
   Glu Asn Ala Thr Gly Val Arg Ser Gln Leu Leu Leu Thr Phe Ala Ser
   100 105 110
   Ile Glu Ser Ala Phe Asp Tyr Glu Ile Lys Ala Lys Thr Ser Ser Ala
   115 120 125
   Thr Gly Trp Phe Gln Phe Leu Thr Gly Thr Trp Lys Thr Met Ile Glu
   130 135 140
   Asn Tyr Gly Met Lys Tyr Gly Val Leu Thr Asp Pro Thr Gly Ala Leu
   145 150 155 160
   Arg Lys Asp Pro Arg Ile Ser Ala Leu Met Gly Ala Glu Leu Ile Lys
                   165                 170                 175
   Glu Asn Met Asn Ile Leu Arg Pro Val Leu Lys Arg Glu Pro Thr Asp
               180                 185                 190
   Thr Asp Leu Tyr Leu Ala His Phe Phe Gly Pro Gly Ala Ala Arg Arg
           195                 200                 205
   Phe Leu Thr Thr Gly Gln Asn Glu Leu Ala Ala Thr His Phe Pro Lys
       210                 215                 220
   Glu Ala Gln Ala Asn Pro Ser Ile Phe Tyr Asn Lys Asp Gly Ser Pro
   225                 230                 235                 240
   Lys Thr Ile Gln Glu Val Tyr Asn Leu Met Asp Gly Lys Val Ala Ala
                   245                 250                 255
   His Arg Lys
<210> 3
   <211> 259
   <212> PRT
   <213> Artificial sequence
<220>
   <223> KZ144 without N-terminal methionine, with selenomethionine instead of methionine residues
<220>
   <221> misc_feature
   <222> (79)..(79)
   <223> Xaa is selenomethionine
<220>
   <221> misc_feature
   <222> (93)..(93)
   <223> Xaa is selenomethionine
<220>
   <221> misc_feature
   <222> (142)..(142)
   <223> Xaa is selenomethionine
<220>
   <221> misc_feature
   <222> (148)..(148)
   <223> Xaa is selenomethionine
<220>
   <221> misc_feature
   <222> (170)..(170)
   <223> Xaa is selenomethionine
<220>
   <221> misc_feature
   <222> (179)..(179)
   <223> Xaa is selenomethionine
<220>
   <221> misc_feature
   <222> (250)..(250)
   <223> Xaa is selenomethionine
<400> 3
   Lys Val Leu Arg Lys Gly Asp Arg Gly Asp Glu Val Cys Gln Leu Gln
   1               5                   10                  15
   Thr Leu Leu Asn Leu Cys Gly Tyr Asp Val Gly Lys Pro Asp Gly Ile
               20                  25                  30
   Phe Gly Asn Asn Thr Phe Asn Gln Val Val Lys Phe Gln Lys Asp Asn
           35                  40                  45
   Cys Leu Asp Ser Asp Gly Ile Val Gly Lys Asn Thr Trp Ala Glu Leu
       50                  55                  60
   Phe Ser Lys Tyr Ser Pro Pro Ile Pro Tyr Lys Thr Ile Pro Xaa Pro
   65                  70                  75                  80
   Thr Ala Asn Lys Ser Arg Ala Ala Ala Thr Pro Val Xaa Asn Ala Val
                   85                  90                  95
   Glu Asn Ala Thr Gly Val Arg Ser Gln Leu Leu Leu Thr Phe Ala Ser
               100                 105                 110
   Ile Glu Ser Ala Phe Asp Tyr Glu Ile Lys Ala Lys Thr Ser Ser Ala
           115                 120                 125
   Thr Gly Trp Phe Gln Phe Leu Thr Gly Thr Trp Lys Thr Xaa Ile Glu
       130                 135                 140
   Asn Tyr Gly Xaa Lys Tyr Gly Val Leu Thr Asp Pro Thr Gly Ala Leu
   145                 150                 155                 160
   Arg Lys Asp Pro Arg Ile Ser Ala Leu Xaa Gly Ala Glu Leu Ile Lys
                   165                 170                 175
   Glu Asn Xaa Asn Ile Leu Arg Pro Val Leu Lys Arg Glu Pro Thr Asp
               180                 185                 190
   Thr Asp Leu Tyr Leu Ala His Phe Phe Gly Pro Gly Ala Ala Arg Arg
           195                 200                 205
   Phe Leu Thr Thr Gly Gln Asn Glu Leu Ala Ala Thr His Phe Pro Lys
       210                 215                 220
   Glu Ala Gln Ala Asn Pro Ser Ile Phe Tyr Asn Lys Asp Gly Ser Pro
   225                 230                 235                 240
   Lys Thr Ile Gln Glu Val Tyr Asn Leu Xaa Asp Gly Lys Val Ala Ala
                   245                 250                 255
   His Arg Lys
<210> 4
   <211> 259
   <212> PRT
   <213> artificial sequence
<220>
   <223> Mutated KZ144 with E115A and without n-terminal methionine
<220>
   <221> misc
   <222> (114)..(114)
   <223> mutation corresponding to position E115A in KZ144 endolysin
<400> 4
   Lys Val Leu Arg Lys Gly Asp Arg Gly Asp Glu Val Cys Gln Leu Gln
   1               5                   10                  15
   Thr Leu Leu Asn Leu Cys Gly Tyr Asp Val Gly Lys Pro Asp Gly Ile
               20                  25                  30
   Phe Gly Asn Asn Thr Phe Asn Gln Val Val Lys Phe Gln Lys Asp Asn
           35                  40                  45
   Cys Leu Asp Ser Asp Gly Ile Val Gly Lys Asn Thr Trp Ala Glu Leu
       50                  55                  60
   Phe Ser Lys Tyr Ser Pro Pro Ile Pro Tyr Lys Thr Ile Pro Met Pro
   65                  70                  75                  80
   Thr Ala Asn Lys Ser Arg Ala Ala Ala Thr Pro Val Met Asn Ala Val
                   85                  90                  95
   Glu Asn Ala Thr Gly Val Arg Ser Gln Leu Leu Leu Thr Phe Ala Ser
               100                 105                 110
   Ile Ala Ser Ala Phe Asp Tyr Glu Ile Lys Ala Lys Thr Ser Ser Ala
           115                 120                 125
   Thr Gly Trp Phe Gln Phe Leu Thr Gly Thr Trp Lys Thr Met Ile Glu
       130                 135                 140
   Asn Tyr Gly Met Lys Tyr Gly Val Leu Thr Asp Pro Thr Gly Ala Leu
   145                 150                 155                 160
   Arg Lys Asp Pro Arg Ile Ser Ala Leu Met Gly Ala Glu Leu Ile Lys
                   165                 170                 175
   Glu Asn Met Asn Ile Leu Arg Pro Val Leu Lys Arg Glu Pro Thr Asp
               180                 185                 190
   Thr Asp Leu Tyr Leu Ala His Phe Phe Gly Pro Gly Ala Ala Arg Arg
           195                 200                 205
   Phe Leu Thr Thr Gly Gln Asn Glu Leu Ala Ala Thr His Phe Pro Lys
       210                 215                 220
   Glu Ala Gln Ala Asn Pro Ser Ile Phe Tyr Asn Lys Asp Gly Ser Pro
   225                 230                 235                 240
   Lys Thr Ile Gln Glu Val Tyr Asn Leu Met Asp Gly Lys Val Ala Ala
                   245                 250                 255
   His Arg Lys
<210> 5
   <211> 260
   <212> PRT
   <213> unknown
<220>
   <223> phiKZgp144
<400> 5
   Met Lys Val Leu Arg Lys Gly Asp Arg Gly Asp Glu Val Cys Gln Leu
   1               5                   10                  15
   Gln Thr Leu Leu Asn Leu Cys Gly Tyr Asp Val Gly Lys Pro Asp Gly
               20                  25                  30
   Ile Phe Gly Asn Asn Thr Phe Asn Gln Val Val Lys Phe Gln Lys Asp
           35                  40                  45
   Asn Cys Leu Asp Ser Asp Gly Ile Val Gly Lys Asn Thr Trp Ala Glu
       50                  55                  60
   Leu Phe Ser Lys Tyr Ser Pro Pro Ile Pro Tyr Lys Thr Ile Pro Met
   65                  70                  75                  80
   Pro Thr Ala Asn Lys Ser Arg Ala Ala Ala Thr Pro Val Met Asn Ala
                   85                  90                  95
   Val Glu Asn Ala Thr Gly Val Arg Ser Gln Leu Leu Leu Thr Phe Ala
               100                 105                 110
   Ser Ile Glu Ser Ala Phe Asp Tyr Glu Ile Lys Ala Lys Thr Ser Ser
           115                 120                 125
   Ala Thr Gly Trp Phe Gln Phe Leu Thr Gly Thr Trp Lys Thr Met Ile
       130                 135                 140
   Glu Asn Tyr Gly Met Lys Tyr Gly Val Leu Thr Asp Pro Thr Gly Ala
   145                 150                 155                 160
   Leu Arg Lys Asp Pro Arg Ile Ser Ala Leu Met Gly Ala Glu Leu Ile
                   165                 170                 175
   Lys Glu Asn Met Asn Ile Leu Arg Pro Val Leu Lys Arg Glu Pro Thr
               180                 185                 190
   Asp Thr Asp Leu Tyr Leu Ala His Phe Phe Gly Pro Gly Ala Ala Arg
           195                 200                 205
   Arg Phe Leu Thr Thr Gly Gln Asn Glu Leu Ala Ala Thr His Phe Pro
       210                 215                 220
   Lys Glu Ala Gln Ala Asn Pro Ser Ile Phe Tyr Asn Lys Asp Gly Ser
   225                 230                 235                 240
   Pro Lys Thr Ile Gln Glu Val Tyr Asn Leu Met Asp Gly Lys Val Ala
                   245                 250                 255
   Ala His Arg Lys
               260
<210> 6
   <211> 260
   <212> PRT
   <213> artificial sequence
<220>
   <223> mutated KZ144 with C14S and C50S
<400> 6
   Met Lys Val Leu Arg Lys Gly Asp Arg Gly Asp Glu Val Ser Gln Leu
   1               5                   10                  15
   Gln Thr Leu Leu Asn Leu Cys Gly Tyr Asp Val Gly Lys Pro Asp Gly
               20                  25                  30
   Ile Phe Gly Asn Asn Thr Phe Asn Gln Val Val Lys Phe Gln Lys Asp
           35                  40                  45
   Asn Ser Leu Asp Ser Asp Gly Ile Val Gly Lys Asn Thr Trp Ala Glu
       50                  55                  60
   Leu Phe Ser Lys Tyr Ser Pro Pro Ile Pro Tyr Lys Thr Ile Pro Met
   65                  70                  75                  80
   Pro Thr Ala Asn Lys Ser Arg Ala Ala Ala Thr Pro Val Met Asn Ala
                   85                  90                  95
   Val Glu Asn Ala Thr Gly Val Arg Ser Gln Leu Leu Leu Thr Phe Ala
               100                 105                 110
   Ser Ile Glu Ser Ala Phe Asp Tyr Glu Ile Lys Ala Lys Thr Ser Ser
           115                 120                 125
   Ala Thr Gly Trp Phe Gln Phe Leu Thr Gly Thr Trp Lys Thr Met Ile
       130                 135                 140
   Glu Asn Tyr Gly Met Lys Tyr Gly Val Leu Thr Asp Pro Thr Gly Ala
   145                 150                 155                 160
   Leu Arg Lys Asp Pro Arg Ile Ser Ala Leu Met Gly Ala Glu Leu Ile
                   165                 170                 175
   Lys Glu Asn Met Asn Ile Leu Arg Pro Val Leu Lys Arg Glu Pro Thr
               180                 185                 190
   Asp Thr Asp Leu Tyr Leu Ala His Phe Phe Gly Pro Gly Ala Ala Arg
           195                 200                 205
   Arg Phe Leu Thr Thr Gly Gln Asn Glu Leu Ala Ala Thr His Phe Pro
       210                 215                 220
   Lys Glu Ala Gln Ala Asn Pro Ser Ile Phe Tyr Asn Lys Asp Gly Ser
   225                 230                 235                 240
   Pro Lys Thr Ile Gln Glu Val Tyr Asn Leu Met Asp Gly Lys Val Ala
                   245                 250                 255
   Ala His Arg Lys
               260
<210> 7
   <211> 260
   <212> PRT
   <213> artificial sequence
<220>
   <223> mutated KZ144 with T82I, A206V and S232
<400> 7
   Met Lys Val Leu Arg Lys Gly Asp Arg Gly Asp Glu Val Cys Gln Leu
   1               5                   10                  15
   Gln Thr Leu Leu Asn Leu Cys Gly Tyr Asp Val Gly Lys Pro Asp Gly
               20                  25                  30
   Ile Phe Gly Asn Asn Thr Phe Asn Gln Val Val Lys Phe Gln Lys Asp
           35                  40                  45
   Asn Cys Leu Asp Ser Asp Gly Ile Val Gly Lys Asn Thr Trp Ala Glu
       50                  55                  60
   Leu Phe Ser Lys Tyr Ser Pro Pro Ile Pro Tyr Lys Thr Ile Pro Met
   65                  70                  75                  80
   Pro Ile Ala Asn Lys Ser Arg Ala Ala Ala Thr Pro Val Met Asn Ala
                   85                  90                  95
   Val Glu Asn Ala Thr Gly Val Arg Ser Gln Leu Leu Leu Thr Phe Ala
               100                 105                 110
   Ser Ile Glu Ser Ala Phe Asp Tyr Glu Ile Lys Ala Lys Thr Ser Ser
           115                 120                 125
   Ala Thr Gly Trp Phe Gln Phe Leu Thr Gly Thr Trp Lys Thr Met Ile
       130                 135                 140
   Glu Asn Tyr Gly Met Lys Tyr Gly Val Leu Thr Asp Pro Thr Gly Ala
   145                 150                 155                 160
   Leu Arg Lys Asp Pro Arg Ile Ser Ala Leu Met Gly Ala Glu Leu Ile
                   165                 170                 175
   Lys Glu Asn Met Asn Ile Leu Arg Pro Val Leu Lys Arg Glu Pro Thr
               180                 185                 190
   Asp Thr Asp Leu Tyr Leu Ala His Phe Phe Gly Pro Gly Val Ala Arg
           195                 200                 205
   Arg Phe Leu Thr Thr Gly Gln Asn Glu Leu Ala Ala Thr His Phe Pro
       210                 215                 220
   Lys Glu Ala Gln Ala Asn Pro Thr Ile Phe Tyr Asn Lys Asp Gly Ser
   225                 230                 235                 240
   Pro Lys Thr Ile Gln Glu Val Tyr Asn Leu Met Asp Gly Lys Val Ala
                   245                 250                 255
   Ala His Arg Lys
               260
<210> 8
   <211> 260
   <212> PRT
   <213> artificial sequence
<220>
   <223> mutated KZ144 with T82I, A206V, S232T, I122M and A160T
<400> 8
   Met Lys Val Leu Arg Lys Gly Asp Arg Gly Asp Glu Val Cys Gln Leu
   1               5                   10                  15
   Gln Thr Leu Leu Asn Leu Cys Gly Tyr Asp Val Gly Lys Pro Asp Gly
               20                  25                  30
   Ile Phe Gly Asn Asn Thr Phe Asn Gln Val Val Lys Phe Gln Lys Asp
           35                  40                  45
   Asn Cys Leu Asp Ser Asp Gly Ile Val Gly Lys Asn Thr Trp Ala Glu
       50                  55                  60
   Leu Phe Ser Lys Tyr Ser Pro Pro Ile Pro Tyr Lys Thr Ile Pro Met
   65                  70                  75                  80
   Pro Ile Ala Asn Lys Ser Arg Ala Ala Ala Thr Pro Val Met Asn Ala
                   85                  90                  95
   Val Glu Asn Ala Thr Gly Val Arg Ser Gln Leu Leu Leu Thr Phe Ala
               100                 105                 110
   Ser Ile Glu Ser Ala Phe Asp Tyr Glu Met Lys Ala Lys Thr Ser Ser
           115                 120                 125
   Ala Thr Gly Trp Phe Gln Phe Leu Thr Gly Thr Trp Lys Thr Met Ile
       130                 135                 140
   Glu Asn Tyr Gly Met Lys Tyr Gly Val Leu Thr Asp Pro Thr Gly Thr
   145                 150                 155                 160
   Leu Arg Lys Asp Pro Arg Ile Ser Ala Leu Met Gly Ala Glu Leu Ile
                   165                 170                 175
   Lys Glu Asn Met Asn Ile Leu Arg Pro Val Leu Lys Arg Glu Pro Thr
               180                 185                 190
   Asp Thr Asp Leu Tyr Leu Ala His Phe Phe Gly Pro Gly Val Ala Arg
           195                 200                 205
   Arg Phe Leu Thr Thr Gly Gln Asn Glu Leu Ala Ala Thr His Phe Pro
       210                 215                 220
   Lys Glu Ala Gln Ala Asn Pro Thr Ile Phe Tyr Asn Lys Asp Gly Ser
   225                 230                 235                 240
   Pro Lys Thr Ile Gln Glu Val Tyr Asn Leu Met Asp Gly Lys Val Ala
                   245                 250                 255
   Ala His Arg Lys
               260
<210> 9
   <211> 260
   <212> PRT
   <213> artificial sequence
<220>
   <223> mutated KZ144 with C14S, C50S, I122M and A160T
<400> 9
   Met Lys Val Leu Arg Lys Gly Asp Arg Gly Asp Glu Val Ser Gln Leu
   1               5                   10                  15
   Gln Thr Leu Leu Asn Leu Cys Gly Tyr Asp Val Gly Lys Pro Asp Gly
               20                  25                  30
   Ile Phe Gly Asn Asn Thr Phe Asn Gln Val Val Lys Phe Gln Lys Asp
           35                  40                  45
   Asn Ser Leu Asp Ser Asp Gly Ile Val Gly Lys Asn Thr Trp Ala Glu
       50                  55                  60
   Leu Phe Ser Lys Tyr Ser Pro Pro Ile Pro Tyr Lys Thr Ile Pro Met
   65                  70                  75                  80
   Pro Thr Ala Asn Lys Ser Arg Ala Ala Ala Thr Pro Val Met Asn Ala
                   85                  90                  95
   Val Glu Asn Ala Thr Gly Val Arg Ser Gln Leu Leu Leu Thr Phe Ala
               100                 105                 110
   Ser Ile Glu Ser Ala Phe Asp Tyr Glu Met Lys Ala Lys Thr Ser Ser
           115                 120                 125
   Ala Thr Gly Trp Phe Gln Phe Leu Thr Gly Thr Trp Lys Thr Met Ile
       130                 135                 140
   Glu Asn Tyr Gly Met Lys Tyr Gly Val Leu Thr Asp Pro Thr Gly Thr
   145                 150                 155                 160
   Leu Arg Lys Asp Pro Arg Ile Ser Ala Leu Met Gly Ala Glu Leu Ile
                   165                 170                 175
   Lys Glu Asn Met Asn Ile Leu Arg Pro Val Leu Lys Arg Glu Pro Thr
               180                 185                 190
   Asp Thr Asp Leu Tyr Leu Ala His Phe Phe Gly Pro Gly Ala Ala Arg
           195                 200                 205
   Arg Phe Leu Thr Thr Gly Gln Asn Glu Leu Ala Ala Thr His Phe Pro
       210                 215                 220
   Lys Glu Ala Gln Ala Asn Pro Ser Ile Phe Tyr Asn Lys Asp Gly Ser
   225                 230                 235                 240
   Pro Lys Thr Ile Gln Glu Val Tyr Asn Leu Met Asp Gly Lys Val Ala
                   245                 250                 255
   Ala His Arg Lys
               260
<210> 10
   <211> 260
   <212> PRT
   <213> artificial sequence
<220>
   <223> mutated KZ144 with C14S, C23S and C50S
<400> 10
   Met Lys Val Leu Arg Lys Gly Asp Arg Gly Asp Glu Val Ser Gln Leu
   1               5                   10                  15
   Gln Thr Leu Leu Asn Leu Ser Gly Tyr Asp Val Gly Lys Pro Asp Gly
               20                  25                  30
   Ile Phe Gly Asn Asn Thr Phe Asn Gln Val Val Lys Phe Gln Lys Asp
           35                  40                  45
   Asn Ser Leu Asp Ser Asp Gly Ile Val Gly Lys Asn Thr Trp Ala Glu
       50                  55                  60
   Leu Phe Ser Lys Tyr Ser Pro Pro Ile Pro Tyr Lys Thr Ile Pro Met
   65                  70                  75                  80
   Pro Thr Ala Asn Lys Ser Arg Ala Ala Ala Thr Pro Val Met Asn Ala
                   85                  90                  95
   Val Glu Asn Ala Thr Gly Val Arg Ser Gln Leu Leu Leu Thr Phe Ala
               100                 105                 110
   Ser Ile Glu Ser Ala Phe Asp Tyr Glu Ile Lys Ala Lys Thr Ser Ser
           115                 120                 125
   Ala Thr Gly Trp Phe Gln Phe Leu Thr Gly Thr Trp Lys Thr Met Ile
       130                 135                 140
   Glu Asn Tyr Gly Met Lys Tyr Gly Val Leu Thr Asp Pro Thr Gly Ala
   145                 150                 155                 160
   Leu Arg Lys Asp Pro Arg Ile Ser Ala Leu Met Gly Ala Glu Leu Ile
                   165                 170                 175
   Lys Glu Asn Met Asn Ile Leu Arg Pro Val Leu Lys Arg Glu Pro Thr
               180                 185                 190
   Asp Thr Asp Leu Tyr Leu Ala His Phe Phe Gly Pro Gly Ala Ala Arg
           195                 200                 205
   Arg Phe Leu Thr Thr Gly Gln Asn Glu Leu Ala Ala Thr His Phe Pro
       210                 215                 220
   Lys Glu Ala Gln Ala Asn Pro Ser Ile Phe Tyr Asn Lys Asp Gly Ser
   225                 230                 235                 240
   Pro Lys Thr Ile Gln Glu Val Tyr Asn Leu Met Asp Gly Lys Val Ala
                   245                 250                 255
   Ala His Arg Lys
               260
<210> 11
   <211> 260
   <212> PRT
   <213> artificial sequence
<220>
   <223> mutated KZ144 with T82I, A206V, S232T, I122M, A160T, C14S and C50S
<400> 11
   Met Lys Val Leu Arg Lys Gly Asp Arg Gly Asp Glu Val Ser Gln Leu
   1               5                   10                  15
   Gln Thr Leu Leu Asn Leu Cys Gly Tyr Asp Val Gly Lys Pro Asp Gly
               20                  25                  30
   Ile Phe Gly Asn Asn Thr Phe Asn Gln Val Val Lys Phe Gln Lys Asp
           35                  40                  45
   Asn Ser Leu Asp Ser Asp Gly Ile Val Gly Lys Asn Thr Trp Ala Glu
       50                  55                  60
   Leu Phe Ser Lys Tyr Ser Pro Pro Ile Pro Tyr Lys Thr Ile Pro Met
   65                  70                  75                  80
   Pro Ile Ala Asn Lys Ser Arg Ala Ala Ala Thr Pro Val Met Asn Ala
                   85                  90                  95
   Val Glu Asn Ala Thr Gly Val Arg Ser Gln Leu Leu Leu Thr Phe Ala
               100                 105                 110
   Ser Ile Glu Ser Ala Phe Asp Tyr Glu Met Lys Ala Lys Thr Ser Ser
           115                 120                 125
   Ala Thr Gly Trp Phe Gln Phe Leu Thr Gly Thr Trp Lys Thr Met Ile
       130                 135                 140
   Glu Asn Tyr Gly Met Lys Tyr Gly Val Leu Thr Asp Pro Thr Gly Thr
   145                 150                 155                 160
   Leu Arg Lys Asp Pro Arg Ile Ser Ala Leu Met Gly Ala Glu Leu Ile
                   165                 170                 175
   Lys Glu Asn Met Asn Ile Leu Arg Pro Val Leu Lys Arg Glu Pro Thr
               180                 185                 190
   Asp Thr Asp Leu Tyr Leu Ala His Phe Phe Gly Pro Gly Val Ala Arg
           195                 200                 205
   Arg Phe Leu Thr Thr Gly Gln Asn Glu Leu Ala Ala Thr His Phe Pro
       210                 215                 220
   Lys Glu Ala Gln Ala Asn Pro Thr Ile Phe Tyr Asn Lys Asp Gly Ser
   225                 230                 235                 240
   Pro Lys Thr Ile Gln Glu Val Tyr Asn Leu Met Asp Gly Lys Val Ala
                   245                 250                 255
   Ala His Arg Lys
               260
<210> 12
   <211> 260
   <212> PRT
   <213> artificial sequence
<220>
   <223> mutated KZ144 with T82I, A206N, S232T, I122M, A160T, C14S and C50S
<400> 12
   Met Lys Val Leu Arg Lys Gly Asp Arg Gly Asp Glu Val Ser Gln Leu
   1               5                   10                  15
   Gln Thr Leu Leu Asn Leu Cys Gly Tyr Asp Val Gly Lys Pro Asp Gly
               20                  25                  30
   Ile Phe Gly Asn Asn Thr Phe Asn Gln Val Val Lys Phe Gln Lys Asp
           35                  40                  45
   Asn Ser Leu Asp Ser Asp Gly Ile Val Gly Lys Asn Thr Trp Ala Glu
       50                  55                  60
   Leu Phe Ser Lys Tyr Ser Pro Pro Ile Pro Tyr Lys Thr Ile Pro Met
   65                  70                  75                  80
   Pro Ile Ala Asn Lys Ser Arg Ala Ala Ala Thr Pro Val Met Asn Ala
                   85                  90                  95
   Val Glu Asn Ala Thr Gly Val Arg Ser Gln Leu Leu Leu Thr Phe Ala
               100                 105                 110
   Ser Ile Glu Ser Ala Phe Asp Tyr Glu Met Lys Ala Lys Thr Ser Ser
           115                 120                 125
   Ala Thr Gly Trp Phe Gln Phe Leu Thr Gly Thr Trp Lys Thr Met Ile
       130                 135                 140
   Glu Asn Tyr Gly Met Lys Tyr Gly Val Leu Thr Asp Pro Thr Gly Thr
   145 150 155 160
   Leu Arg Lys Asp Pro Arg Ile Ser Ala Leu Met Gly Ala Glu Leu Ile
   165 170 175
   Lys Glu Asn Met Asn Ile Leu Arg Pro Val Leu Lys Arg Glu Pro Thr
   180 185 190
   Asp Thr Asp Leu Tyr Leu Ala His Phe Phe Gly Pro Gly Asn Ala Arg
   195 200 205
   Arg Phe Leu Thr Thr Gly Gln Asn Glu Leu Ala Ala Thr His Phe Pro
   210 215 220
   Lys Glu Ala Gln Ala Asn Pro Thr Ile Phe Tyr Asn Lys Asp Gly Ser
   225 230 235 240
   Pro Lys Thr Ile Gln Glu Val Tyr Asn Leu Met Asp Gly Lys Val Ala
   245 250 255
   Ala His Arg Lys
   260
<210> 13
   <211> 260
   <212> PRT
   <213> artificial sequence
<220>
   <223> mutated KZ144 with N230Y, T82I, A206V, S232T, I122M, A160T, C14S and C50S
<400> 13
   Met Lys Val Leu Arg Lys Gly Asp Arg Gly Asp Glu Val Ser Gln Leu
   1 5 10 15
   Gln Thr Leu Leu Asn Leu Cys Gly Tyr Asp Val Gly Lys Pro Asp Gly
   20 25 30
   Ile Phe Gly Asn Asn Thr Phe Asn Gln Val Val Lys Phe Gln Lys Asp
   35 40 45
   Asn Ser Leu Asp Ser Asp Gly Ile Val Gly Lys Asn Thr Trp Ala Glu
   50 55 60
   Leu Phe Ser Lys Tyr Ser Pro Pro Ile Pro Tyr Lys Thr Ile Pro Met
   65 70 75 80
   Pro Ile Ala Asn Lys Ser Arg Ala Ala Ala Thr Pro Val Met Asn Ala
                   85                  90                  95
   Val Glu Asn Ala Thr Gly Val Arg Ser Gln Leu Leu Leu Thr Phe Ala
               100                 105                 110
   Ser Ile Glu Ser Ala Phe Asp Tyr Glu Met Lys Ala Lys Thr Ser Ser
           115                 120                 125
   Ala Thr Gly Trp Phe Gln Phe Leu Thr Gly Thr Trp Lys Thr Met Ile
       130                 135                 140
   Glu Asn Tyr Gly Met Lys Tyr Gly Val Leu Thr Asp Pro Thr Gly Thr
   145                 150                 155                 160
   Leu Arg Lys Asp Pro Arg Ile Ser Ala Leu Met Gly Ala Glu Leu Ile
                   165                 170                 175
   Lys Glu Asn Met Asn Ile Leu Arg Pro Val Leu Lys Arg Glu Pro Thr
               180                 185                 190
   Asp Thr Asp Leu Tyr Leu Ala His Phe Phe Gly Pro Gly Val Ala Arg
           195                 200                 205
   Arg Phe Leu Thr Thr Gly Gln Asn Glu Leu Ala Ala Thr His Phe Pro
       210                 215                 220
   Lys Glu Ala Gln Ala Tyr Pro Thr Ile Phe Tyr Asn Lys Asp Gly Ser
   225                 230                 235                 240
   Pro Lys Thr Ile Gln Glu Val Tyr Asn Leu Met Asp Gly Lys Val Ala
                   245                 250                 255
   Ala His Arg Lys
               260
<210> 14
   <211> 260
   <212> PRT
   <213> artificial sequence
<220>
   <223> mutated KZ144 with M180E, T82I, A206V, S232T, I122M, A160T, C14S and C50S
<400> 14 260
<210> 15
   <211> 260
   <212> PRT
   <213> artificial sequence
<220>
   <223> mutated KZ144 with M149P, T82I, A206V, S232T, I122M, A160T, C14S and C50S
<400> 15
   Met Lys Val Leu Arg Lys Gly Asp Arg Gly Asp Glu Val Ser Gln Leu
   1               5                   10                  15
   Gln Thr Leu Leu Asn Leu Cys Gly Tyr Asp Val Gly Lys Pro Asp Gly
               20                  25                  30
   Ile Phe Gly Asn Asn Thr Phe Asn Gln Val Val Lys Phe Gln Lys Asp
           35                  40                  45
   Asn Ser Leu Asp Ser Asp Gly Ile Val Gly Lys Asn Thr Trp Ala Glu
       50                  55                  60
   Leu Phe Ser Lys Tyr Ser Pro Pro Ile Pro Tyr Lys Thr Ile Pro Met
   65                  70                  75                  80
   Pro Ile Ala Asn Lys Ser Arg Ala Ala Ala Thr Pro Val Met Asn Ala
                   85                  90                  95
   Val Glu Asn Ala Thr Gly Val Arg Ser Gln Leu Leu Leu Thr Phe Ala
               100                 105                 110
   Ser Ile Glu Ser Ala Phe Asp Tyr Glu Met Lys Ala Lys Thr Ser Ser
           115                 120                 125
   Ala Thr Gly Trp Phe Gln Phe Leu Thr Gly Thr Trp Lys Thr Met Ile
       130                 135                 140
   Glu Asn Tyr Gly Pro Lys Tyr Gly Val Leu Thr Asp Pro Thr Gly Thr
   145                 150                 155                 160
   Leu Arg Lys Asp Pro Arg Ile Ser Ala Leu Met Gly Ala Glu Leu Ile
                   165                 170                 175
   Lys Glu Asn Met Asn Ile Leu Arg Pro Val Leu Lys Arg Glu Pro Thr
               180                 185                 190
   Asp Thr Asp Leu Tyr Leu Ala His Phe Phe Gly Pro Gly Val Ala Arg
               195                 200                 205
   Arg Phe Leu Thr Thr Gly Gln Asn Glu Leu Ala Ala Thr His Phe Pro
       210                 215                 220
   Lys Glu Ala Gln Ala Asn Pro Thr Ile Phe Tyr Asn Lys Asp Gly Ser
   225                 230                 235                 240
   Pro Lys Thr Ile Gln Glu Val Tyr Asn Leu Met Asp Gly Lys Val Ala
                   245                 250                 255
   Ala His Arg Lys
               260
<210> 16
   <211> 260
   <212> PRT
   <213> Artificial Sequence
<220>
   <223> mutated KZ144 with V186Y, T82I, A206V, S232T, I122M, A160T, C14S and C50S
<400> 16
   Met Lys Val Leu Arg Lys Gly Asp Arg Gly Asp Glu Val Ser Gln Leu
   1               5                   10                  15
   Gln Thr Leu Leu Asn Leu Cys Gly Tyr Asp Val Gly Lys Pro Asp Gly
               20                  25                  30
   Ile Phe Gly Asn Asn Thr Phe Asn Gln Val Val Lys Phe Gln Lys Asp
           35                  40                  45
   Asn Ser Leu Asp Ser Asp Gly Ile Val Gly Lys Asn Thr Trp Ala Glu
       50                  55                  60
   Leu Phe Ser Lys Tyr Ser Pro Pro Ile Pro Tyr Lys Thr Ile Pro Met
   65                  70                  75                  80
   Pro Ile Ala Asn Lys Ser Arg Ala Ala Ala Thr Pro Val Met Asn Ala
                   85                  90                  95
   Val Glu Asn Ala Thr Gly Val Arg Ser Gln Leu Leu Leu Thr Phe Ala
               100                 105                 110
   Ser Ile Glu Ser Ala Phe Asp Tyr Glu Met Lys Ala Lys Thr Ser Ser
           115                 120                 125
   Ala Thr Gly Trp Phe Gln Phe Leu Thr Gly Thr Trp Lys Thr Met Ile
               130                 135                 140
   Glu Asn Tyr Gly Met Lys Tyr Gly Val Leu Thr Asp Pro Thr Gly Thr
   145                 150                 155                 160
   Leu Arg Lys Asp Pro Arg Ile Ser Ala Leu Met Gly Ala Glu Leu Ile
                   165                 170                 175
   Lys Glu Asn Met Asn Ile Leu Arg Pro Tyr Leu Lys Arg Glu Pro Thr
               180                 185                 190
   Asp Thr Asp Leu Tyr Leu Ala His Phe Phe Gly Pro Gly Val Ala Arg
           195                 200                 205
   Arg Phe Leu Thr Thr Gly Gln Asn Glu Leu Ala Ala Thr His Phe Pro
       210                 215                 220
   Lys Glu Ala Gln Ala Asn Pro Thr Ile Phe Tyr Asn Lys Asp Gly Ser
   225                 230                 235                 240
   Pro Lys Thr Ile Gln Glu Val Tyr Asn Leu Met Asp Gly Lys Val Ala
                   245                 250                 255
   Ala His Arg Lys
               260
<210> 17
   <211> 260
   <212> PRT
   <213> Artificial Sequence
<220>
   <223> mutated KZ144 with T82I, A206V, S232T, I122M, A160T, C14R and C50S
<400> 17
   Met Lys Val Leu Arg Lys Gly Asp Arg Gly Asp Glu Val Arg Gln Leu
   1               5                   10                  15
   Gln Thr Leu Leu Asn Leu Cys Gly Tyr Asp Val Gly Lys Pro Asp Gly
               20                  25                  30
   Ile Phe Gly Asn Asn Thr Phe Asn Gln Val Val Lys Phe Gln Lys Asp
           35                  40                  45
   Asn Ser Leu Asp Ser Asp Gly Ile Val Gly Lys Asn Thr Trp Ala Glu
       50                  55                  60
   Leu Phe Ser Lys Tyr Ser Pro Pro Ile Pro Tyr Lys Thr Ile Pro Met
   65                  70                  75                  80
   Pro Ile Ala Asn Lys Ser Arg Ala Ala Ala Thr Pro Val Met Asn Ala
                   85                  90                  95
   Val Glu Asn Ala Thr Gly Val Arg Ser Gln Leu Leu Leu Thr Phe Ala
               100                 105                 110
   Ser Ile Glu Ser Ala Phe Asp Tyr Glu Met Lys Ala Lys Thr Ser Ser
           115                 120                 125
   Ala Thr Gly Trp Phe Gln Phe Leu Thr Gly Thr Trp Lys Thr Met Ile
       130                 135                 140
   Glu Asn Tyr Gly Met Lys Tyr Gly Val Leu Thr Asp Pro Thr Gly Thr
   145                 150                 155                 160
   Leu Arg Lys Asp Pro Arg Ile Ser Ala Leu Met Gly Ala Glu Leu Ile
                   165                 170                 175
   Lys Glu Asn Met Asn Ile Leu Arg Pro Val Leu Lys Arg Glu Pro Thr
               180                 185                 190
   Asp Thr Asp Leu Tyr Leu Ala His Phe Phe Gly Pro Gly Val Ala Arg
           195                 200                 205
   Arg Phe Leu Thr Thr Gly Gln Asn Glu Leu Ala Ala Thr His Phe Pro
       210                 215                 220
   Lys Glu Ala Gln Ala Asn Pro Thr Ile Phe Tyr Asn Lys Asp Gly Ser
   225                 230                 235                 240
   Pro Lys Thr Ile Gln Glu Val Tyr Asn Leu Met Asp Gly Lys Val Ala
                   245                 250                 255
   Ala His Arg Lys
               260
<210> 18
   <211> 260
   <212> PRT
   <213> Artificial Sequence
<220>
   <223> mutated KZ144 with T82I, A206V, S232T, I122M, C14S and C50S
<400> 18
   Met Lys Val Leu Arg Lys Gly Asp Arg Gly Asp Glu Val Cys Gln Leu
   1               5                   10                  15
   Gln Thr Leu Leu Asn Leu Cys Gly Tyr Asp Val Gly Lys Pro Asp Gly
               20                  25                  30
   Ile Phe Gly Asn Asn Thr Phe Asn Gln Val Val Lys Phe Gln Lys Asp
           35                  40                  45
   Asn Ser Leu Asp Ser Asp Gly Ile Val Gly Lys Asn Thr Trp Ala Glu
       50                  55                  60
   Leu Phe Ser Lys Tyr Ser Pro Pro Ile Pro Tyr Lys Thr Ile Pro Met
   65                  70                  75                  80
   Pro Ile Ala Asn Lys Ser Arg Ala Ala Ala Thr Pro Val Met Asn Ala
                   85                  90                  95
   Val Glu Asn Ala Thr Gly Val Arg Ser Gln Leu Leu Leu Thr Phe Ala
               100                 105                 110
   Ser Ile Glu Ser Ala Phe Asp Tyr Glu Met Lys Ala Lys Thr Ser Ser
           115                 120                 125
   Ala Thr Gly Trp Phe Gln Phe Leu Thr Gly Thr Trp Lys Thr Met Ile
       130                 135                 140
   Glu Asn Tyr Gly Met Lys Tyr Gly Val Leu Thr Asp Pro Thr Gly Ala
   145                 150                 155                 160
   Leu Arg Lys Asp Pro Arg Ile Ser Ala Leu Met Gly Ala Glu Leu Ile
                   165                 170                 175
   Lys Glu Asn Met Asn Ile Leu Arg Pro Val Leu Lys Arg Glu Pro Thr
               180                 185                 190
   Asp Thr Asp Leu Tyr Leu Ala His Phe Phe Gly Pro Gly Val Ala Arg
           195                 200                 205
   Arg Phe Leu Thr Thr Gly Gln Asn Glu Leu Ala Ala Thr His Phe Pro
       210                 215                 220
   Lys Glu Ala Gln Ala Asn Pro Thr Ile Phe Tyr Asn Lys Asp Gly Ser
   225                 230                 235                 240
   Pro Lys Thr Ile Gln Glu Val Tyr Asn Leu Met Asp Gly Lys Val Ala
                   245                 250                 255
   Ala His Arg Lys
   260
<210> 19
   <211> 260
   <212> PRT
   <213> Artificial Sequence
<220>
   <223> mutated KZ144 with T82I, A206V, S232T, I122M, A160T, C14S and C50N
<400> 19
   Met Lys Val Leu Arg Lys Gly Asp Arg Gly Asp Glu Val Ser Gln Leu
   1               5                   10                  15
   Gln Thr Leu Leu Asn Leu Cys Gly Tyr Asp Val Gly Lys Pro Asp Gly
               20                  25                  30
   Ile Phe Gly Asn Asn Thr Phe Asn Gln Val Val Lys Phe Gln Lys Asp
           35                  40                  45
   Asn Asn Leu Asp Ser Asp Gly Ile Val Gly Lys Asn Thr Trp Ala Glu
       50                  55                  60
   Leu Phe Ser Lys Tyr Ser Pro Pro Ile Pro Tyr Lys Thr Ile Pro Met
   65                  70                  75                  80
   Pro Ile Ala Asn Lys Ser Arg Ala Ala Ala Thr Pro Val Met Asn Ala
                   85                  90                  95
   Val Glu Asn Ala Thr Gly Val Arg Ser Gln Leu Leu Leu Thr Phe Ala
               100                 105                 110
   Ser Ile Glu Ser Ala Phe Asp Tyr Glu Met Lys Ala Lys Thr Ser Ser
           115                 120                 125
   Ala Thr Gly Trp Phe Gln Phe Leu Thr Gly Thr Trp Lys Thr Met Ile
       130                 135                 140
   Glu Asn Tyr Gly Met Lys Tyr Gly Val Leu Thr Asp Pro Thr Gly Thr
   145                 150                 155                 160
   Leu Arg Lys Asp Pro Arg Ile Ser Ala Leu Met Gly Ala Glu Leu Ile
                   165                 170                 175
   Lys Glu Asn Met Asn Ile Leu Arg Pro Val Leu Lys Arg Glu Pro Thr
               180                 185                 190
   Asp Thr Asp Leu Tyr Leu Ala His Phe Phe Gly Pro Gly Val Ala Arg
   195 200 205
   Arg Phe Leu Thr Thr Gly Gln Asn Glu Leu Ala Ala Thr His Phe Pro
   210 215 220
   Lys Glu Ala Gln Ala Asn Pro Thr Ile Phe Tyr Asn Lys Asp Gly Ser
   225 230 235 240
   Pro Lys Thr Ile Gln Glu Val Tyr Asn Leu Met Asp Gly Lys Val Ala
   245 250 255
   Ala His Arg Lys
   260
<210> 20
   <211> 260
   <212> PRT
   <213> Artificial Sequence
<220>
   <223> mutated KZ144 with C14R, C50S, T82I, I122M, M149P, A206V and S232T
<400> 20
   Met Lys Val Leu Arg Lys Gly Asp Arg Gly Asp Glu Val Arg Gln Leu
   1               5                   10                  15
   Gln Thr Leu Leu Asn Leu Cys Gly Tyr Asp Val Gly Lys Pro Asp Gly
               20                  25                  30
   Ile Phe Gly Asn Asn Thr Phe Asn Gln Val Val Lys Phe Gln Lys Asp
           35                  40                  45
   Asn Ser Leu Asp Ser Asp Gly Ile Val Gly Lys Asn Thr Trp Ala Glu
       50                  55                  60
   Leu Phe Ser Lys Tyr Ser Pro Pro Ile Pro Tyr Lys Thr Ile Pro Met
   65                  70                  75                  80
   Pro Ile Ala Asn Lys Ser Arg Ala Ala Ala Thr Pro Val Met Asn Ala
                   85                  90                  95
   Val Glu Asn Ala Thr Gly Val Arg Ser Gln Leu Leu Leu Thr Phe Ala
               100                 105                 110
   Ser Ile Glu Ser Ala Phe Asp Tyr Glu Met Lys Ala Lys Thr Ser Ser
           115                 120                 125
   Ala Thr Gly Trp Phe Gln Phe Leu Thr Gly Thr Trp Lys Thr Met Ile
           130                 135                 140
   Glu Asn Tyr Gly Pro Lys Tyr Gly Val Leu Thr Asp Pro Thr Gly Ala
   145                 150                 155                 160
   Leu Arg Lys Asp Pro Arg Ile Ser Ala Leu Met Gly Ala Glu Leu Ile
                   165                 170                 175
   Lys Glu Asn Met Asn Ile Leu Arg Pro Val Leu Lys Arg Glu Pro Thr
               180                 185                 190
   Asp Thr Asp Leu Tyr Leu Ala His Phe Phe Gly Pro Gly Val Ala Arg
           195                 200                 205
   Arg Phe Leu Thr Thr Gly Gln Asn Glu Leu Ala Ala Thr His Phe Pro
       210                 215                 220
   Lys Glu Ala Gln Ala Asn Pro Thr Ile Phe Tyr Asn Lys Asp Gly Ser
   225                 230                 235                 240
   Pro Lys Thr Ile Gln Glu Val Tyr Asn Leu Met Asp Gly Lys Val Ala
                   245                 250                 255
   Ala His Arg Lys
               260
<210> 21
   <211> 260
   <212> PRT
   <213> Artificial Sequence
<220>
   <223> mutated KZ144 with C14R, C50S, T82I, I122M, M149P, A160T, A206V and S232T
<400> 21
   Met Lys Val Leu Arg Lys Gly Asp Arg Gly Asp Glu Val Arg Gln Leu
   1               5                   10                  15
   Gln Thr Leu Leu Asn Leu Cys Gly Tyr Asp Val Gly Lys Pro Asp Gly
               20                  25                  30
   Ile Phe Gly Asn Asn Thr Phe Asn Gln Val Val Lys Phe Gln Lys Asp
           35                  40                  45
   Asn Ser Leu Asp Ser Asp Gly Ile Val Gly Lys Asn Thr Trp Ala Glu
       50                  55                  60
   Leu Phe Ser Lys Tyr Ser Pro Pro Ile Pro Tyr Lys Thr Ile Pro Met
   65                  70                  75                  80
   Pro Ile Ala Asn Lys Ser Arg Ala Ala Ala Thr Pro Val Met Asn Ala
                   85                  90                  95
   Val Glu Asn Ala Thr Gly Val Arg Ser Gln Leu Leu Leu Thr Phe Ala
               100                 105                 110
   Ser Ile Glu Ser Ala Phe Asp Tyr Glu Met Lys Ala Lys Thr Ser Ser
           115                 120                 125
   Ala Thr Gly Trp Phe Gln Phe Leu Thr Gly Thr Trp Lys Thr Met Ile
       130                 135                 140
   Glu Asn Tyr Gly Pro Lys Tyr Gly Val Leu Thr Asp Pro Thr Gly Thr
   145                 150                 155                 160
   Leu Arg Lys Asp Pro Arg Ile Ser Ala Leu Met Gly Ala Glu Leu Ile
                   165                 170                 175
   Lys Glu Asn Met Asn Ile Leu Arg Pro Val Leu Lys Arg Glu Pro Thr
               180                 185                 190
   Asp Thr Asp Leu Tyr Leu Ala His Phe Phe Gly Pro Gly Val Ala Arg
           195                 200                 205
   Arg Phe Leu Thr Thr Gly Gln Asn Glu Leu Ala Ala Thr His Phe Pro
       210                 215                 220
   Lys Glu Ala Gln Ala Asn Pro Thr Ile Phe Tyr Asn Lys Asp Gly Ser
   225                 230                 235                 240
   Pro Lys Thr Ile Gln Glu Val Tyr Asn Leu Met Asp Gly Lys Val Ala
                   245                 250                 255
   Ala His Arg Lys
               260
<210> 22
   <211> 260
   <212> PRT
   <213> Artificial Sequence
<220>
   <223> mutated KZ144 with T82I, I122M, M149P, A206V, S232T, C14R and C50N
<400> 22
   Met Lys Val Leu Arg Lys Gly Asp Arg Gly Asp Glu Val Arg Gln Leu
   1               5                   10                  15
   Gln Thr Leu Leu Asn Leu Cys Gly Tyr Asp Val Gly Lys Pro Asp Gly
               20                  25                  30
   Ile Phe Gly Asn Asn Thr Phe Asn Gln Val Val Lys Phe Gln Lys Asp
           35                  40                  45
   Asn Asn Leu Asp Ser Asp Gly Ile Val Gly Lys Asn Thr Trp Ala Glu
       50                  55                  60
   Leu Phe Ser Lys Tyr Ser Pro Pro Ile Pro Tyr Lys Thr Ile Pro Met
   65                  70                  75                  80
   Pro Ile Ala Asn Lys Ser Arg Ala Ala Ala Thr Pro Val Met Asn Ala
                   85                  90                  95
   Val Glu Asn Ala Thr Gly Val Arg Ser Gln Leu Leu Leu Thr Phe Ala
               100                 105                 110
   Ser Ile Glu Ser Ala Phe Asp Tyr Glu Met Lys Ala Lys Thr Ser Ser
           115                 120                 125
   Ala Thr Gly Trp Phe Gln Phe Leu Thr Gly Thr Trp Lys Thr Met Ile
       130                 135                 140
   Glu Asn Tyr Gly Pro Lys Tyr Gly Val Leu Thr Asp Pro Thr Gly Ala
   145                 150                 155                 160
   Leu Arg Lys Asp Pro Arg Ile Ser Ala Leu Met Gly Ala Glu Leu Ile
                   165                 170                 175
   Lys Glu Asn Met Asn Ile Leu Arg Pro Val Leu Lys Arg Glu Pro Thr
               180                 185                 190
   Asp Thr Asp Leu Tyr Leu Ala His Phe Phe Gly Pro Gly Val Ala Arg
           195                 200                 205
   Arg Phe Leu Thr Thr Gly Gln Asn Glu Leu Ala Ala Thr His Phe Pro
       210                 215                 220
   Lys Glu Ala Gln Ala Asn Pro Thr Ile Phe Tyr Asn Lys Asp Gly Ser
   225                 230                 235                 240
   Pro Lys Thr Ile Gln Glu Val Tyr Asn Leu Met Asp Gly Lys Val Ala
                   245                 250                 255
   Ala His Arg Lys
   260
<210> 23
   <211> 260
   <212> PRT
   <213> Artificial Sequence
<220>
   <223> mutated KZ144 with T82I, I122M, M149P, I167L, A206V, S232T, C14R and C50N
<400> 23
   Met Lys Val Leu Arg Lys Gly Asp Arg Gly Asp Glu Val Arg Gln Leu
   1               5                   10                  15
   Gln Thr Leu Leu Asn Leu Cys Gly Tyr Asp Val Gly Lys Pro Asp Gly
               20                  25                  30
   Ile Phe Gly Asn Asn Thr Phe Asn Gln Val Val Lys Phe Gln Lys Asp
           35                  40                  45
   Asn Asn Leu Asp Ser Asp Gly Ile Val Gly Lys Asn Thr Trp Ala Glu
       50                  55                  60
   Leu Phe Ser Lys Tyr Ser Pro Pro Ile Pro Tyr Lys Thr Ile Pro Met
   65                  70                  75                  80
   Pro Ile Ala Asn Lys Ser Arg Ala Ala Ala Thr Pro Val Met Asn Ala
                   85                  90                  95
   Val Glu Asn Ala Thr Gly Val Arg Ser Gln Leu Leu Leu Thr Phe Ala
               100                 105                 110
   Ser Ile Glu Ser Ala Phe Asp Tyr Glu Met Lys Ala Lys Thr Ser Ser
           115                 120                 125
   Ala Thr Gly Trp Phe Gln Phe Leu Thr Gly Thr Trp Lys Thr Met Ile
       130                 135                 140
   Glu Asn Tyr Gly Pro Lys Tyr Gly Val Leu Thr Asp Pro Thr Gly Ala
   145                 150                 155                 160
   Leu Arg Lys Asp Pro Arg Leu Ser Ala Leu Met Gly Ala Glu Leu Ile
                   165                 170                 175
   Lys Glu Asn Met Asn Ile Leu Arg Pro Val Leu Lys Arg Glu Pro Thr
               180                 185                 190
   Asp Thr Asp Leu Tyr Leu Ala His Phe Phe Gly Pro Gly Val Ala Arg
   195 200 205
   Arg Phe Leu Thr Thr Gly Gln Asn Glu Leu Ala Ala Thr His Phe Pro
   210 215 220
   Lys Glu Ala Gln Ala Asn Pro Thr Ile Phe Tyr Asn Lys Asp Gly Ser
   225 230 235 240
   Pro Lys Thr Ile Gln Glu Val Tyr Asn Leu Met Asp Gly Lys Val Ala
   245 250 255
   Ala His Arg Lys
   260
<210> 24
   <211> 260
   <212> PRT
   <213> Artificial Sequence
<220>
   <223> mutated KZ144 with T82I, I122M, M149P, N179F, A206V, S232T, C14R and C50N
<400> 24
   Met Lys Val Leu Arg Lys Gly Asp Arg Gly Asp Glu Val Arg Gln Leu
   1 5 10 15
   Gln Thr Leu Leu Asn Leu Cys Gly Tyr Asp Val Gly Lys Pro Asp Gly
   20 25 30
   Ile Phe Gly Asn Asn Thr Phe Asn Gln Val Val Lys Phe Gln Lys Asp
   35 40 45
   Asn Asn Leu Asp Ser Asp Gly Ile Val Gly Lys Asn Thr Trp Ala Glu
   50 55 60
   Leu Phe Ser Lys Tyr Ser Pro Pro Ile Pro Tyr Lys Thr Ile Pro Met
   65 70 75 80
   Pro Ile Ala Asn Lys Ser Arg Ala Ala Ala Thr Pro Val Met Asn Ala
   85 90 95
   Val Glu Asn Ala Thr Gly Val Arg Ser Gln Leu Leu Leu Thr Phe Ala
   100 105 110
   Ser Ile Glu Ser Ala Phe Asp Tyr Glu Met Lys Ala Lys Thr Ser Ser
   115 120 125
   Ala Thr Gly Trp Phe Gln Phe Leu Thr Gly Thr Trp Lys Thr Met Ile
       130                 135                 140
   Glu Asn Tyr Gly Pro Lys Tyr Gly Val Leu Thr Asp Pro Thr Gly Ala
   145                 150                 155                 160
   Leu Arg Lys Asp Pro Arg Leu Ser Ala Leu Met Gly Ala Glu Leu Ile
                   165                 170                 175
   Lys Glu Phe Met Asn Ile Leu Arg Pro Val Leu Lys Arg Glu Pro Thr
               180                 185                 190
   Asp Thr Asp Leu Tyr Leu Ala His Phe Phe Gly Pro Gly Val Ala Arg
           195                 200                 205
   Arg Phe Leu Thr Thr Gly Gln Asn Glu Leu Ala Ala Thr His Phe Pro
       210                 215                 220
   Lys Glu Ala Gln Ala Asn Pro Thr Ile Phe Tyr Asn Lys Asp Gly Ser
   225                 230                 235                 240
   Pro Lys Thr Ile Gln Glu Val Tyr Asn Leu Met Asp Gly Lys Val Ala
                   245                 250                 255
   Ala His Arg Lys
               260
<210> 25
   <211> 260
   <212> PRT
   <213> Artificial Sequence
<220>
   <223> mutated KZ144 with T82I, I122M, M149P, A206V, T212N, S232T, C14R and C50N
<400> 25
   Met Lys Val Leu Arg Lys Gly Asp Arg Gly Asp Glu Val Arg Gln Leu
   1               5                   10                  15
   Gln Thr Leu Leu Asn Leu Cys Gly Tyr Asp Val Gly Lys Pro Asp Gly
               20                  25                  30
   Ile Phe Gly Asn Asn Thr Phe Asn Gln Val Val Lys Phe Gln Lys Asp
           35                  40                  45
   Asn Asn Leu Asp Ser Asp Gly Ile Val Gly Lys Asn Thr Trp Ala Glu
       50                  55                  60
   Leu Phe Ser Lys Tyr Ser Pro Pro Ile Pro Tyr Lys Thr Ile Pro Met
   65                  70                  75                  80
   Pro Ile Ala Asn Lys Ser Arg Ala Ala Ala Thr Pro Val Met Asn Ala
                   85                  90                  95
   Val Glu Asn Ala Thr Gly Val Arg Ser Gln Leu Leu Leu Thr Phe Ala
               100                 105                 110
   Ser Ile Glu Ser Ala Phe Asp Tyr Glu Met Lys Ala Lys Thr Ser Ser
           115                 120                 125
   Ala Thr Gly Trp Phe Gln Phe Leu Thr Gly Thr Trp Lys Thr Met Ile
       130                 135                 140
   Glu Asn Tyr Gly Pro Lys Tyr Gly Val Leu Thr Asp Pro Thr Gly Ala
   145                 150                 155                 160
   Leu Arg Lys Asp Pro Arg Ile Ser Ala Leu Met Gly Ala Glu Leu Ile
                   165                 170                 175
   Lys Glu Asn Met Asn Ile Leu Arg Pro Val Leu Lys Arg Glu Pro Thr
               180                 185                 190
   Asp Thr Asp Leu Tyr Leu Ala His Phe Phe Gly Pro Gly Val Ala Arg
           195                 200                 205
   Arg Phe Leu Asn Thr Gly Gln Asn Glu Leu Ala Ala Thr His Phe Pro
       210                 215                 220
   Lys Glu Ala Gln Ala Asn Pro Thr Ile Phe Tyr Asn Lys Asp Gly Ser
   225                 230                 235                 240
   Pro Lys Thr Ile Gln Glu Val Tyr Asn Leu Met Asp Gly Lys Val Ala
                   245                 250                 255
   Ala His Arg Lys
               260
<210> 26
   <211> 260
   <212> PRT
   <213> Artificial Sequence
<220>
   <223> mutated KZ144 with T82I, I122M, M149P, A206V, P224Q, S232T, C14R and C50N
<400> 26
   Met Lys Val Leu Arg Lys Gly Asp Arg Gly Asp Glu Val Arg Gln Leu
   1               5                   10                  15
   Gln Thr Leu Leu Asn Leu Cys Gly Tyr Asp Val Gly Lys Pro Asp Gly
               20                  25                  30
   Ile Phe Gly Asn Asn Thr Phe Asn Gln Val Val Lys Phe Gln Lys Asp
           35                  40                  45
   Asn Asn Leu Asp Ser Asp Gly Ile Val Gly Lys Asn Thr Trp Ala Glu
       50                  55                  60
   Leu Phe Ser Lys Tyr Ser Pro Pro Ile Pro Tyr Lys Thr Ile Pro Met
   65                  70                  75                  80
   Pro Ile Ala Asn Lys Ser Arg Ala Ala Ala Thr Pro Val Met Asn Ala
                   85                  90                  95
   Val Glu Asn Ala Thr Gly Val Arg Ser Gln Leu Leu Leu Thr Phe Ala
               100                 105                 110
   Ser Ile Glu Ser Ala Phe Asp Tyr Glu Met Lys Ala Lys Thr Ser Ser
           115                 120                 125
   Ala Thr Gly Trp Phe Gln Phe Leu Thr Gly Thr Trp Lys Thr Met Ile
       130                 135                 140
   Glu Asn Tyr Gly Pro Lys Tyr Gly Val Leu Thr Asp Pro Thr Gly Ala
   145                 150                 155                 160
   Leu Arg Lys Asp Pro Arg Ile Ser Ala Leu Met Gly Ala Glu Leu Ile
                   165                 170                 175
   Lys Glu Asn Met Asn Ile Leu Arg Pro Val Leu Lys Arg Glu Pro Thr
               180                 185                 190
   Asp Thr Asp Leu Tyr Leu Ala His Phe Phe Gly Pro Gly Val Ala Arg
           195                 200                 205
   Arg Phe Leu Thr Thr Gly Gln Asn Glu Leu Ala Ala Thr His Phe Gln
       210                 215                 220
   Lys Glu Ala Gln Ala Asn Pro Thr Ile Phe Tyr Asn Lys Asp Gly Ser
   225                 230                 235                 240
   Pro Lys Thr Ile Gln Glu Val Tyr Asn Leu Met Asp Gly Lys Val Ala
                   245                 250                 255
   Ala His Arg Lys
               260
<210> 27
   <211> 260
   <212> PRT
   <213> Artificial Sequence
<220>
   <223> mutated KZ144 with T82I, I122M, M149P, L154T, A206V, S232T, C14R and C50N
<400> 27
   Met Lys Val Leu Arg Lys Gly Asp Arg Gly Asp Glu Val Arg Gln Leu
   1               5                   10                  15
   Gln Thr Leu Leu Asn Leu Cys Gly Tyr Asp Val Gly Lys Pro Asp Gly
               20                  25                  30
   Ile Phe Gly Asn Asn Thr Phe Asn Gln Val Val Lys Phe Gln Lys Asp
           35                  40                  45
   Asn Asn Leu Asp Ser Asp Gly Ile Val Gly Lys Asn Thr Trp Ala Glu
       50                  55                  60
   Leu Phe Ser Lys Tyr Ser Pro Pro Ile Pro Tyr Lys Thr Ile Pro Met
   65                  70                  75                  80
   Pro Ile Ala Asn Lys Ser Arg Ala Ala Ala Thr Pro Val Met Asn Ala
                   85                  90                  95
   Val Glu Asn Ala Thr Gly Val Arg Ser Gln Leu Leu Leu Thr Phe Ala
               100                 105                 110
   Ser Ile Glu Ser Ala Phe Asp Tyr Glu Met Lys Ala Lys Thr Ser Ser
           115                 120                 125
   Ala Thr Gly Trp Phe Gln Phe Leu Thr Gly Thr Trp Lys Thr Met Ile
       130                 135                 140
   Glu Asn Tyr Gly Pro Lys Tyr Gly Val Thr Thr Asp Pro Thr Gly Ala
   145                 150                 155                 160
   Leu Arg Lys Asp Pro Arg Ile Ser Ala Leu Met Gly Ala Glu Leu Ile
                   165                 170                 175
   Lys Glu Asn Met Asn Ile Leu Arg Pro Val Leu Lys Arg Glu Pro Thr
               180                 185                 190
   Asp Thr Asp Leu Tyr Leu Ala His Phe Phe Gly Pro Gly Val Ala Arg
           195                 200                 205
   Arg Phe Leu Thr Thr Gly Gln Asn Glu Leu Ala Ala Thr His Phe Pro
       210                 215                 220
   Lys Glu Ala Gln Ala Asn Pro Thr Ile Phe Tyr Asn Lys Asp Gly Ser
   225                 230                 235                 240
   Pro Lys Thr Ile Gln Glu Val Tyr Asn Leu Met Asp Gly Lys Val Ala
                   245                 250                 255
   Ala His Arg Lys
               260
<210> 28
   <211> 259
   <212> PRT
   <213> artificial sequence
<220>
   <223> mutated KZ144 with C14S and C50S, without N-terminal methionine
<400> 28
   Lys Val Leu Arg Lys Gly Asp Arg Gly Asp Glu Val Ser Gln Leu Gln
   1               5                   10                  15
   Thr Leu Leu Asn Leu Cys Gly Tyr Asp Val Gly Lys Pro Asp Gly Ile
               20                  25                  30
   Phe Gly Asn Asn Thr Phe Asn Gln Val Val Lys Phe Gln Lys Asp Asn
           35                  40                  45
   Ser Leu Asp Ser Asp Gly Ile Val Gly Lys Asn Thr Trp Ala Glu Leu
       50                  55                  60
   Phe Ser Lys Tyr Ser Pro Pro Ile Pro Tyr Lys Thr Ile Pro Met Pro
   65                  70                  75                  80
   Thr Ala Asn Lys Ser Arg Ala Ala Ala Thr Pro Val Met Asn Ala Val
                   85                  90                  95
   Glu Asn Ala Thr Gly Val Arg Ser Gln Leu Leu Leu Thr Phe Ala Ser
               100                 105                 110
   Ile Glu Ser Ala Phe Asp Tyr Glu Ile Lys Ala Lys Thr Ser Ser Ala
           115                 120                 125
   Thr Gly Trp Phe Gln Phe Leu Thr Gly Thr Trp Lys Thr Met Ile Glu
   130 135 140
   Asn Tyr Gly Met Lys Tyr Gly Val Leu Thr Asp Pro Thr Gly Ala Leu
   145 150 155 160
   Arg Lys Asp Pro Arg Ile Ser Ala Leu Met Gly Ala Glu Leu Ile Lys
   165 170 175
   Glu Asn Met Asn Ile Leu Arg Pro Val Leu Lys Arg Glu Pro Thr Asp
   180 185 190
   Thr Asp Leu Tyr Leu Ala His Phe Phe Gly Pro Gly Ala Ala Arg Arg
   195 200 205
   Phe Leu Thr Thr Gly Gln Asn Glu Leu Ala Ala Thr His Phe Pro Lys
   210 215 220
   Glu Ala Gln Ala Asn Pro Ser Ile Phe Tyr Asn Lys Asp Gly Ser Pro
   225 230 235 240
   Lys Thr Ile Gln Glu Val Tyr Asn Leu Met Asp Gly Lys Val Ala Ala
   245 250 255
   His Arg Lys
<210> 29
   <211> 259
   <212> PRT
   <213> artificial sequence
<220>
   <223> mutated KZ144 with T82I, A206V and S232, without N-terminal methionine
<400> 29
   Lys Val Leu Arg Lys Gly Asp Arg Gly Asp Glu Val Cys Gln Leu Gln
   1 5 10 15
   Thr Leu Leu Asn Leu Cys Gly Tyr Asp Val Gly Lys Pro Asp Gly Ile
   20 25 30
   Phe Gly Asn Asn Thr Phe Asn Gln Val Val Lys Phe Gln Lys Asp Asn
   35 40 45
   Cys Leu Asp Ser Asp Gly Ile Val Gly Lys Asn Thr Trp Ala Glu Leu
   50 55 60
   Phe Ser Lys Tyr Ser Pro Pro Ile Pro Tyr Lys Thr Ile Pro Met Pro
   65 70 75 80
   Ile Ala Asn Lys Ser Arg Ala Ala Ala Thr Pro Val Met Asn Ala Val
   85 90 95
   Glu Asn Ala Thr Gly Val Arg Ser Gln Leu Leu Leu Thr Phe Ala Ser
   100 105 110
   Ile Glu Ser Ala Phe Asp Tyr Glu Ile Lys Ala Lys Thr Ser Ser Ala
   115 120 125
   Thr Gly Trp Phe Gln Phe Leu Thr Gly Thr Trp Lys Thr Met Ile Glu
   130 135 140
   Asn Tyr Gly Met Lys Tyr Gly Val Leu Thr Asp Pro Thr Gly Ala Leu
   145 150 155 160
   Arg Lys Asp Pro Arg Ile Ser Ala Leu Met Gly Ala Glu Leu Ile Lys
   165 170 175
   Glu Asn Met Asn Ile Leu Arg Pro Val Leu Lys Arg Glu Pro Thr Asp
   180 185 190
   Thr Asp Leu Tyr Leu Ala His Phe Phe Gly Pro Gly Val Ala Arg Arg
   195 200 205
   Phe Leu Thr Thr Gly Gln Asn Glu Leu Ala Ala Thr His Phe Pro Lys
   210 215 220
   Glu Ala Gln Ala Asn Pro Thr Ile Phe Tyr Asn Lys Asp Gly Ser Pro
   225 230 235 240
   Lys Thr Ile Gln Glu Val Tyr Asn Leu Met Asp Gly Lys Val Ala Ala
   245 250 255
   His Arg Lys
<210> 30
   <211> 259
   <212> PRT
   <213> artificial sequence
<220>
   <223> mutated KZ144 with T82I, A206V, S232T, I122M and A160T, without N-terminal methionine
<400> 30
   Lys Val Leu Arg Lys Gly Asp Arg Gly Asp Glu Val Cys Gln Leu Gln
   1               5                   10                  15
   Thr Leu Leu Asn Leu Cys Gly Tyr Asp Val Gly Lys Pro Asp Gly Ile
               20                  25                  30
   Phe Gly Asn Asn Thr Phe Asn Gln Val Val Lys Phe Gln Lys Asp Asn
           35                  40                  45
   Cys Leu Asp Ser Asp Gly Ile Val Gly Lys Asn Thr Trp Ala Glu Leu
       50                  55                  60
   Phe Ser Lys Tyr Ser Pro Pro Ile Pro Tyr Lys Thr Ile Pro Met Pro
   65                  70                  75                  80
   Ile Ala Asn Lys Ser Arg Ala Ala Ala Thr Pro Val Met Asn Ala Val
                   85                  90                  95
   Glu Asn Ala Thr Gly Val Arg Ser Gln Leu Leu Leu Thr Phe Ala Ser
               100                 105                 110
   Ile Glu Ser Ala Phe Asp Tyr Glu Met Lys Ala Lys Thr Ser Ser Ala
           115                 120                 125
   Thr Gly Trp Phe Gln Phe Leu Thr Gly Thr Trp Lys Thr Met Ile Glu
       130                 135                 140
   Asn Tyr Gly Met Lys Tyr Gly Val Leu Thr Asp Pro Thr Gly Thr Leu
   145                 150                 155                 160
   Arg Lys Asp Pro Arg Ile Ser Ala Leu Met Gly Ala Glu Leu Ile Lys
                   165                 170                 175
   Glu Asn Met Asn Ile Leu Arg Pro Val Leu Lys Arg Glu Pro Thr Asp
               180                 185                 190
   Thr Asp Leu Tyr Leu Ala His Phe Phe Gly Pro Gly Val Ala Arg Arg
           195                 200                 205
   Phe Leu Thr Thr Gly Gln Asn Glu Leu Ala Ala Thr His Phe Pro Lys
       210                 215                 220
   Glu Ala Gln Ala Asn Pro Thr Ile Phe Tyr Asn Lys Asp Gly Ser Pro
   225                 230                 235                 240
   Lys Thr Ile Gln Glu Val Tyr Asn Leu Met Asp Gly Lys Val Ala Ala
                   245                 250                 255
   His Arg Lys
<210> 31
   <211> 259
   <212> PRT
   <213> artificial sequence
<220>
   <223> mutated KZ144 with C14S, C50S, I122M and A160T, without N-terminal methionine
<400> 31
   Lys Val Leu Arg Lys Gly Asp Arg Gly Asp Glu Val Ser Gln Leu Gln
   1 5 10 15
   Thr Leu Leu Asn Leu Cys Gly Tyr Asp Val Gly Lys Pro Asp Gly Ile
   20 25 30
   Phe Gly Asn Asn Thr Phe Asn Gln Val Val Lys Phe Gln Lys Asp Asn
   35 40 45
   Ser Leu Asp Ser Asp Gly Ile Val Gly Lys Asn Thr Trp Ala Glu Leu
   50 55 60
   Phe Ser Lys Tyr Ser Pro Pro Ile Pro Tyr Lys Thr Ile Pro Met Pro
   65 70 75 80
   Thr Ala Asn Lys Ser Arg Ala Ala Ala Thr Pro Val Met Asn Ala Val
   85 90 95
   Glu Asn Ala Thr Gly Val Arg Ser Gln Leu Leu Leu Thr Phe Ala Ser
   100 105 110
   Ile Glu Ser Ala Phe Asp Tyr Glu Met Lys Ala Lys Thr Ser Ser Ala
   115 120 125
   Thr Gly Trp Phe Gln Phe Leu Thr Gly Thr Trp Lys Thr Met Ile Glu
   130 135 140
   Asn Tyr Gly Met Lys Tyr Gly Val Leu Thr Asp Pro Thr Gly Thr Leu
   145 150 155 160
   Arg Lys Asp Pro Arg Ile Ser Ala Leu Met Gly Ala Glu Leu Ile Lys
   165 170 175
   Glu Asn Met Asn Ile Leu Arg Pro Val Leu Lys Arg Glu Pro Thr Asp
   180 185 190
   Thr Asp Leu Tyr Leu Ala His Phe Phe Gly Pro Gly Ala Ala Arg Arg
           195                 200                 205
   Phe Leu Thr Thr Gly Gln Asn Glu Leu Ala Ala Thr His Phe Pro Lys
       210                 215                 220
   Glu Ala Gln Ala Asn Pro Ser Ile Phe Tyr Asn Lys Asp Gly Ser Pro
   225                 230                 235                 240
   Lys Thr Ile Gln Glu Val Tyr Asn Leu Met Asp Gly Lys Val Ala Ala
                   245                 250                 255
   His Arg Lys
<210> 32
   <211> 259
   <212> PRT
   <213> artificial sequence
<220>
   <223> mutated KZ144 with C14S, C23S and C50S, without N-terminal methionine
<400> 32
   Lys Val Leu Arg Lys Gly Asp Arg Gly Asp Glu Val Ser Gln Leu Gln
   1               5                   10                  15
   Thr Leu Leu Asn Leu Ser Gly Tyr Asp Val Gly Lys Pro Asp Gly Ile
               20                  25                  30
   Phe Gly Asn Asn Thr Phe Asn Gln Val Val Lys Phe Gln Lys Asp Asn
           35                  40                  45
   Ser Leu Asp Ser Asp Gly Ile Val Gly Lys Asn Thr Trp Ala Glu Leu
       50                  55                  60
   Phe Ser Lys Tyr Ser Pro Pro Ile Pro Tyr Lys Thr Ile Pro Met Pro
   65                  70                  75                  80
   Thr Ala Asn Lys Ser Arg Ala Ala Ala Thr Pro Val Met Asn Ala Val
                   85                  90                  95
   Glu Asn Ala Thr Gly Val Arg Ser Gln Leu Leu Leu Thr Phe Ala Ser
               100                 105                 110
   Ile Glu Ser Ala Phe Asp Tyr Glu Ile Lys Ala Lys Thr Ser Ser Ala
           115                 120                 125
   Thr Gly Trp Phe Gln Phe Leu Thr Gly Thr Trp Lys Thr Met Ile Glu
       130                 135                 140
   Asn Tyr Gly Met Lys Tyr Gly Val Leu Thr Asp Pro Thr Gly Ala Leu
   145                 150                 155                 160
   Arg Lys Asp Pro Arg Ile Ser Ala Leu Met Gly Ala Glu Leu Ile Lys
                   165                 170                 175
   Glu Asn Met Asn Ile Leu Arg Pro Val Leu Lys Arg Glu Pro Thr Asp
               180                 185                 190
   Thr Asp Leu Tyr Leu Ala His Phe Phe Gly Pro Gly Ala Ala Arg Arg
           195                 200                 205
   Phe Leu Thr Thr Gly Gln Asn Glu Leu Ala Ala Thr His Phe Pro Lys
       210                 215                 220
   Glu Ala Gln Ala Asn Pro Ser Ile Phe Tyr Asn Lys Asp Gly Ser Pro
   225                 230                 235                 240
   Lys Thr Ile Gln Glu Val Tyr Asn Leu Met Asp Gly Lys Val Ala Ala
                   245                 250                 255
   His Arg Lys
<210> 33
   <211> 259
   <212> PRT
   <213> artificial sequence
<220>
   <223> mutated KZ144 with T82I, A206V, S232T, I122M, A160T, C14S and C50S, without N-terminal methionine
<400> 33
   Lys Val Leu Arg Lys Gly Asp Arg Gly Asp Glu Val Ser Gln Leu Gln
   1               5                   10                  15
   Thr Leu Leu Asn Leu Cys Gly Tyr Asp Val Gly Lys Pro Asp Gly Ile
               20                  25                  30
   Phe Gly Asn Asn Thr Phe Asn Gln Val Val Lys Phe Gln Lys Asp Asn
           35                  40                  45
   Ser Leu Asp Ser Asp Gly Ile Val Gly Lys Asn Thr Trp Ala Glu Leu
       50                  55                  60
   Phe Ser Lys Tyr Ser Pro Pro Ile Pro Tyr Lys Thr Ile Pro Met Pro
   65                  70                  75                  80
   Ile Ala Asn Lys Ser Arg Ala Ala Ala Thr Pro Val Met Asn Ala Val
                   85                  90                  95
   Glu Asn Ala Thr Gly Val Arg Ser Gln Leu Leu Leu Thr Phe Ala Ser
               100                 105                 110
   Ile Glu Ser Ala Phe Asp Tyr Glu Met Lys Ala Lys Thr Ser Ser Ala
           115                 120                 125
   Thr Gly Trp Phe Gln Phe Leu Thr Gly Thr Trp Lys Thr Met Ile Glu
       130                 135                 140
   Asn Tyr Gly Met Lys Tyr Gly Val Leu Thr Asp Pro Thr Gly Thr Leu
   145                 150                 155                 160
   Arg Lys Asp Pro Arg Ile Ser Ala Leu Met Gly Ala Glu Leu Ile Lys
                   165                 170                 175
   Glu Asn Met Asn Ile Leu Arg Pro Val Leu Lys Arg Glu Pro Thr Asp
               180                 185                 190
   Thr Asp Leu Tyr Leu Ala His Phe Phe Gly Pro Gly Val Ala Arg Arg
           195                 200                 205
   Phe Leu Thr Thr Gly Gln Asn Glu Leu Ala Ala Thr His Phe Pro Lys
       210                 215                 220
   Glu Ala Gln Ala Asn Pro Thr Ile Phe Tyr Asn Lys Asp Gly Ser Pro
   225                 230                 235                 240
   Lys Thr Ile Gln Glu Val Tyr Asn Leu Met Asp Gly Lys Val Ala Ala
                   245                 250                 255
   His Arg Lys
<210> 34
   <211> 259
   <212> PRT
   <213> artificial sequence
<220>
   <223> mutated KZ144 with T82I, A206N, S232T, I122M, A160T, C14S and C50S, without N-terminal methionine
<400> 34
   Lys Val Leu Arg Lys Gly Asp Arg Gly Asp Glu Val Ser Gln Leu Gln
   1               5                   10                  15
   Thr Leu Leu Asn Leu Cys Gly Tyr Asp Val Gly Lys Pro Asp Gly Ile
               20                  25                  30
   Phe Gly Asn Asn Thr Phe Asn Gln Val Val Lys Phe Gln Lys Asp Asn
           35                  40                  45
   Ser Leu Asp Ser Asp Gly Ile Val Gly Lys Asn Thr Trp Ala Glu Leu
       50                  55                  60
   Phe Ser Lys Tyr Ser Pro Pro Ile Pro Tyr Lys Thr Ile Pro Met Pro
   65                  70                  75                  80
   Ile Ala Asn Lys Ser Arg Ala Ala Ala Thr Pro Val Met Asn Ala Val
                   85                  90                  95
   Glu Asn Ala Thr Gly Val Arg Ser Gln Leu Leu Leu Thr Phe Ala Ser
               100                 105                 110
   Ile Glu Ser Ala Phe Asp Tyr Glu Met Lys Ala Lys Thr Ser Ser Ala
           115                 120                 125
   Thr Gly Trp Phe Gln Phe Leu Thr Gly Thr Trp Lys Thr Met Ile Glu
       130                 135                 140
   Asn Tyr Gly Met Lys Tyr Gly Val Leu Thr Asp Pro Thr Gly Thr Leu
   145                 150                 155                 160
   Arg Lys Asp Pro Arg Ile Ser Ala Leu Met Gly Ala Glu Leu Ile Lys
                   165                 170                 175
   Glu Asn Met Asn Ile Leu Arg Pro Val Leu Lys Arg Glu Pro Thr Asp
               180                 185                 190
   Thr Asp Leu Tyr Leu Ala His Phe Phe Gly Pro Gly Asn Ala Arg Arg
           195                 200                 205
   Phe Leu Thr Thr Gly Gln Asn Glu Leu Ala Ala Thr His Phe Pro Lys
       210                 215                 220
   Glu Ala Gln Ala Asn Pro Thr Ile Phe Tyr Asn Lys Asp Gly Ser Pro
   225                 230                 235                 240
   Lys Thr Ile Gln Glu Val Tyr Asn Leu Met Asp Gly Lys Val Ala Ala
                   245                 250                 255
   His Arg Lys
<210> 35
   <211> 259
   <212> PRT
   <213> Artificial Sequence
<220>
   <223> mutated KZ144 with N230Y, T82I, A206V, S232T, I122M, A160T, C14S and C50S, without N-terminal methionine
<400> 35
   Lys Val Leu Arg Lys Gly Asp Arg Gly Asp Glu Val Ser Gln Leu Gln
   1               5                   10                  15
   Thr Leu Leu Asn Leu Cys Gly Tyr Asp Val Gly Lys Pro Asp Gly Ile
               20                  25                  30
   Phe Gly Asn Asn Thr Phe Asn Gln Val Val Lys Phe Gln Lys Asp Asn
           35                  40                  45
   Ser Leu Asp Ser Asp Gly Ile Val Gly Lys Asn Thr Trp Ala Glu Leu
       50                  55                  60
   Phe Ser Lys Tyr Ser Pro Pro Ile Pro Tyr Lys Thr Ile Pro Met Pro
   65                  70                  75                  80
   Ile Ala Asn Lys Ser Arg Ala Ala Ala Thr Pro Val Met Asn Ala Val
                   85                  90                  95
   Glu Asn Ala Thr Gly Val Arg Ser Gln Leu Leu Leu Thr Phe Ala Ser
               100                 105                 110
   Ile Glu Ser Ala Phe Asp Tyr Glu Met Lys Ala Lys Thr Ser Ser Ala
           115                 120                 125
   Thr Gly Trp Phe Gln Phe Leu Thr Gly Thr Trp Lys Thr Met Ile Glu
       130                 135                 140
   Asn Tyr Gly Met Lys Tyr Gly Val Leu Thr Asp Pro Thr Gly Thr Leu
   145                 150                 155                 160
   Arg Lys Asp Pro Arg Ile Ser Ala Leu Met Gly Ala Glu Leu Ile Lys
                   165                 170                 175
   Glu Asn Met Asn Ile Leu Arg Pro Val Leu Lys Arg Glu Pro Thr Asp
               180                 185                 190
   Thr Asp Leu Tyr Leu Ala His Phe Phe Gly Pro Gly Val Ala Arg Arg
   195 200 205
   Phe Leu Thr Thr Gly Gln Asn Glu Leu Ala Ala Thr His Phe Pro Lys
   210 215 220
   Glu Ala Gln Ala Tyr Pro Thr Ile Phe Tyr Asn Lys Asp Gly Ser Pro
   225 230 235 240
   Lys Thr Ile Gln Glu Val Tyr Asn Leu Met Asp Gly Lys Val Ala Ala
   245 250 255
   His Arg Lys
<210> 36
   <211> 259
   <212> PRT
   <213> Artificial Sequence
<220>
   <223> mutated KZ144 with M180E, T82I, A206V, S232T, I122M, A160T, C14S and C50S, without N-terminal methionine
<400> 36
   Lys Val Leu Arg Lys Gly Asp Arg Gly Asp Glu Val Ser Gln Leu Gln
   1 5 10 15
   Thr Leu Leu Asn Leu Cys Gly Tyr Asp Val Gly Lys Pro Asp Gly Ile
   20 25 30
   Phe Gly Asn Asn Thr Phe Asn Gln Val Val Lys Phe Gln Lys Asp Asn
   35 40 45
   Ser Leu Asp Ser Asp Gly Ile Val Gly Lys Asn Thr Trp Ala Glu Leu
   50 55 60
   Phe Ser Lys Tyr Ser Pro Pro Ile Pro Tyr Lys Thr Ile Pro Met Pro
   65 70 75 80
   Ile Ala Asn Lys Ser Arg Ala Ala Ala Thr Pro Val Met Asn Ala Val
   85 90 95
   Glu Asn Ala Thr Gly Val Arg Ser Gln Leu Leu Leu Thr Phe Ala Ser
   100 105 110
   Ile Glu Ser Ala Phe Asp Tyr Glu Met Lys Ala Lys Thr Ser Ser Ala
   115 120 125
   Thr Gly Trp Phe Gln Phe Leu Thr Gly Thr Trp Lys Thr Met Ile Glu
       130                 135                 140
   Asn Tyr Gly Met Lys Tyr Gly Val Leu Thr Asp Pro Thr Gly Thr Leu
   145                 150                 155                 160
   Arg Lys Asp Pro Arg Ile Ser Ala Leu Met Gly Ala Glu Leu Ile Lys
                   165                 170                 175
   Glu Asn Glu Asn Ile Leu Arg Pro Val Leu Lys Arg Glu Pro Thr Asp
               180                 185                 190
   Thr Asp Leu Tyr Leu Ala His Phe Phe Gly Pro Gly Val Ala Arg Arg
           195                 200                 205
   Phe Leu Thr Thr Gly Gln Asn Glu Leu Ala Ala Thr His Phe Pro Lys
       210                 215                 220
   Glu Ala Gln Ala Asn Pro Thr Ile Phe Tyr Asn Lys Asp Gly Ser Pro
   225                 230                 235                 240
   Lys Thr Ile Gln Glu Val Tyr Asn Leu Met Asp Gly Lys Val Ala Ala
                   245                 250                 255
   His Arg Lys
<210> 37
   <211> 259
   <212> PRT
   <213> Artificial Sequence
<220>
   <223> mutated KZ144 with M149P, T82I, A206V, S232T, I122M, A160T, C14S and C50S, without N-terminal methionine
<400> 37
   Lys Val Leu Arg Lys Gly Asp Arg Gly Asp Glu Val Ser Gln Leu Gln
   1               5                   10                  15
   Thr Leu Leu Asn Leu Cys Gly Tyr Asp Val Gly Lys Pro Asp Gly Ile
               20                  25                  30
   Phe Gly Asn Asn Thr Phe Asn Gln Val Val Lys Phe Gln Lys Asp Asn
           35                  40                  45
   Ser Leu Asp Ser Asp Gly Ile Val Gly Lys Asn Thr Trp Ala Glu Leu
       50                  55                  60
   Phe Ser Lys Tyr Ser Pro Pro Ile Pro Tyr Lys Thr Ile Pro Met Pro
   65 70 75 80
   Ile Ala Asn Lys Ser Arg Ala Ala Ala Thr Pro Val Met Asn Ala Val
   85 90 95
   Glu Asn Ala Thr Gly Val Arg Ser Gln Leu Leu Leu Thr Phe Ala Ser
   100 105 110
   Ile Glu Ser Ala Phe Asp Tyr Glu Met Lys Ala Lys Thr Ser Ser Ala
   115 120 125
   Thr Gly Trp Phe Gln Phe Leu Thr Gly Thr Trp Lys Thr Met Ile Glu
   130 135 140
   Asn Tyr Gly Pro Lys Tyr Gly Val Leu Thr Asp Pro Thr Gly Thr Leu
   145 150 155 160
   Arg Lys Asp Pro Arg Ile Ser Ala Leu Met Gly Ala Glu Leu Ile Lys
   165 170 175
   Glu Asn Met Asn Ile Leu Arg Pro Val Leu Lys Arg Glu Pro Thr Asp
   180 185 190
   Thr Asp Leu Tyr Leu Ala His Phe Phe Gly Pro Gly Val Ala Arg Arg
   195 200 205
   Phe Leu Thr Thr Gly Gln Asn Glu Leu Ala Ala Thr His Phe Pro Lys
   210 215 220
   Glu Ala Gln Ala Asn Pro Thr Ile Phe Tyr Asn Lys Asp Gly Ser Pro
   225 230 235 240
   Lys Thr Ile Gln Glu Val Tyr Asn Leu Met Asp Gly Lys Val Ala Ala
   245 250 255
   His Arg Lys
<210> 38
   <211> 259
   <212> PRT
   <213> Artificial Sequence
<220>
   <223> mutated KZ144 with V186Y, T82I, A206V, S232T, I122M, A160T, C14S and C50S, without N-terminal methionine
<400> 38
   Lys Val Leu Arg Lys Gly Asp Arg Gly Asp Glu Val Ser Gln Leu Gln
   1               5                   10                  15
   Thr Leu Leu Asn Leu Cys Gly Tyr Asp Val Gly Lys Pro Asp Gly Ile
               20                  25                  30
   Phe Gly Asn Asn Thr Phe Asn Gln Val Val Lys Phe Gln Lys Asp Asn
           35                  40                  45
   Ser Leu Asp Ser Asp Gly Ile Val Gly Lys Asn Thr Trp Ala Glu Leu
       50                  55                  60
   Phe Ser Lys Tyr Ser Pro Pro Ile Pro Tyr Lys Thr Ile Pro Met Pro
   65                  70                  75                  80
   Ile Ala Asn Lys Ser Arg Ala Ala Ala Thr Pro Val Met Asn Ala Val
                   85                  90                  95
   Glu Asn Ala Thr Gly Val Arg Ser Gln Leu Leu Leu Thr Phe Ala Ser
               100                 105                 110
   Ile Glu Ser Ala Phe Asp Tyr Glu Met Lys Ala Lys Thr Ser Ser Ala
           115                 120                 125
   Thr Gly Trp Phe Gln Phe Leu Thr Gly Thr Trp Lys Thr Met Ile Glu
       130                 135                 140
   Asn Tyr Gly Met Lys Tyr Gly Val Leu Thr Asp Pro Thr Gly Thr Leu
   145                 150                 155                 160
   Arg Lys Asp Pro Arg Ile Ser Ala Leu Met Gly Ala Glu Leu Ile Lys
                   165                 170                 175
   Glu Asn Met Asn Ile Leu Arg Pro Tyr Leu Lys Arg Glu Pro Thr Asp
               180                 185                 190
   Thr Asp Leu Tyr Leu Ala His Phe Phe Gly Pro Gly Val Ala Arg Arg
           195                 200                 205
   Phe Leu Thr Thr Gly Gln Asn Glu Leu Ala Ala Thr His Phe Pro Lys
       210                 215                 220
   Glu Ala Gln Ala Asn Pro Thr Ile Phe Tyr Asn Lys Asp Gly Ser Pro
   225                 230                 235                 240
   Lys Thr Ile Gln Glu Val Tyr Asn Leu Met Asp Gly Lys Val Ala Ala
   245 250 255
   His Arg Lys
<210> 39
   <211> 259
   <212> PRT
   <213> Artificial Sequence
<220>
   <223> mutated KZ144 with T82I, A206V, S232T, I122M, A160T, C14R and C50S, without N-terminal methionine
<400> 39
   Lys Val Leu Arg Lys Gly Asp Arg Gly Asp Glu Val Arg Gln Leu Gln
   1               5                   10                  15
   Thr Leu Leu Asn Leu Cys Gly Tyr Asp Val Gly Lys Pro Asp Gly Ile
               20                  25                  30
   Phe Gly Asn Asn Thr Phe Asn Gln Val Val Lys Phe Gln Lys Asp Asn
           35                  40                  45
   Ser Leu Asp Ser Asp Gly Ile Val Gly Lys Asn Thr Trp Ala Glu Leu
       50                  55                  60
   Phe Ser Lys Tyr Ser Pro Pro Ile Pro Tyr Lys Thr Ile Pro Met Pro
   65                  70                  75                  80
   Ile Ala Asn Lys Ser Arg Ala Ala Ala Thr Pro Val Met Asn Ala Val
                   85                  90                  95
   Glu Asn Ala Thr Gly Val Arg Ser Gln Leu Leu Leu Thr Phe Ala Ser
               100                 105                 110
   Ile Glu Ser Ala Phe Asp Tyr Glu Met Lys Ala Lys Thr Ser Ser Ala
           115                 120                 125
   Thr Gly Trp Phe Gln Phe Leu Thr Gly Thr Trp Lys Thr Met Ile Glu
       130                 135                 140
   Asn Tyr Gly Met Lys Tyr Gly Val Leu Thr Asp Pro Thr Gly Thr Leu
   145                 150                 155                 160
   Arg Lys Asp Pro Arg Ile Ser Ala Leu Met Gly Ala Glu Leu Ile Lys
                   165                 170                 175
   Glu Asn Met Asn Ile Leu Arg Pro Val Leu Lys Arg Glu Pro Thr Asp
   180 185 190
   Thr Asp Leu Tyr Leu Ala His Phe Phe Gly Pro Gly Val Ala Arg Arg
   195 200 205
   Phe Leu Thr Thr Gly Gln Asn Glu Leu Ala Ala Thr His Phe Pro Lys
   210 215 220
   Glu Ala Gln Ala Asn Pro Thr Ile Phe Tyr Asn Lys Asp Gly Ser Pro
   225 230 235 240
   Lys Thr Ile Gln Glu Val Tyr Asn Leu Met Asp Gly Lys Val Ala Ala
   245 250 255
   His Arg Lys
<210> 40
   <211> 259
   <212> PRT
   <213> Artificial Sequence
<220>
   <223> mutated KZ144 with T82I, A206V, S232T, I122M, C14S and C50S, without N-terminal methionine
<400> 40
   Lys Val Leu Arg Lys Gly Asp Arg Gly Asp Glu Val Cys Gln Leu Gln
   1 5 10 15
   Thr Leu Leu Asn Leu Cys Gly Tyr Asp Val Gly Lys Pro Asp Gly Ile
   20 25 30
   Phe Gly Asn Asn Thr Phe Asn Gln Val Val Lys Phe Gln Lys Asp Asn
   35 40 45
   Ser Leu Asp Ser Asp Gly Ile Val Gly Lys Asn Thr Trp Ala Glu Leu
   50 55 60
   Phe Ser Lys Tyr Ser Pro Pro Ile Pro Tyr Lys Thr Ile Pro Met Pro
   65 70 75 80
   Ile Ala Asn Lys Ser Arg Ala Ala Ala Thr Pro Val Met Asn Ala Val
   85 90 95
   Glu Asn Ala Thr Gly Val Arg Ser Gln Leu Leu Leu Thr Phe Ala Ser
   100 105 110
   Ile Glu Ser Ala Phe Asp Tyr Glu Met Lys Ala Lys Thr Ser Ser Ala
   115 120 125
   Thr Gly Trp Phe Gln Phe Leu Thr Gly Thr Trp Lys Thr Met Ile Glu
   130 135 140
   Asn Tyr Gly Met Lys Tyr Gly Val Leu Thr Asp Pro Thr Gly Ala Leu
   145 150 155 160
   Arg Lys Asp Pro Arg Ile Ser Ala Leu Met Gly Ala Glu Leu Ile Lys
   165 170 175
   Glu Asn Met Asn Ile Leu Arg Pro Val Leu Lys Arg Glu Pro Thr Asp
   180 185 190
   Thr Asp Leu Tyr Leu Ala His Phe Phe Gly Pro Gly Val Ala Arg Arg
   195 200 205
   Phe Leu Thr Thr Gly Gln Asn Glu Leu Ala Ala Thr His Phe Pro Lys
   210 215 220
   Glu Ala Gln Ala Asn Pro Thr Ile Phe Tyr Asn Lys Asp Gly Ser Pro
   225 230 235 240
   Lys Thr Ile Gln Glu Val Tyr Asn Leu Met Asp Gly Lys Val Ala Ala
   245 250 255
   His Arg Lys
<210> 41
   <211> 259
   <212> PRT
   <213> Artificial Sequence
<220>
   <223> mutated KZ144 with T82I, A206V, S232T, I122M, A160T, C14S and C50N, without N-terminal methionine
<400> 41
   Lys Val Leu Arg Lys Gly Asp Arg Gly Asp Glu Val Ser Gln Leu Gln
   1 5 10 15
   Thr Leu Leu Asn Leu Cys Gly Tyr Asp Val Gly Lys Pro Asp Gly Ile
   20 25 30
   Phe Gly Asn Asn Thr Phe Asn Gln Val Val Lys Phe Gln Lys Asp Asn
   35 40 45
   Asn Leu Asp Ser Asp Gly Ile Val Gly Lys Asn Thr Trp Ala Glu Leu
       50                  55                  60
   Phe Ser Lys Tyr Ser Pro Pro Ile Pro Tyr Lys Thr Ile Pro Met Pro
   65                  70                  75                  80
   Ile Ala Asn Lys Ser Arg Ala Ala Ala Thr Pro Val Met Asn Ala Val
                   85                  90                  95
   Glu Asn Ala Thr Gly Val Arg Ser Gln Leu Leu Leu Thr Phe Ala Ser
               100                 105                 110
   Ile Glu Ser Ala Phe Asp Tyr Glu Met Lys Ala Lys Thr Ser Ser Ala
           115                 120                 125
   Thr Gly Trp Phe Gln Phe Leu Thr Gly Thr Trp Lys Thr Met Ile Glu
       130                 135                 140
   Asn Tyr Gly Met Lys Tyr Gly Val Leu Thr Asp Pro Thr Gly Thr Leu
   145                 150                 155                 160
   Arg Lys Asp Pro Arg Ile Ser Ala Leu Met Gly Ala Glu Leu Ile Lys
                   165                 170                 175
   Glu Asn Met Asn Ile Leu Arg Pro Val Leu Lys Arg Glu Pro Thr Asp
               180                 185                 190
   Thr Asp Leu Tyr Leu Ala His Phe Phe Gly Pro Gly Val Ala Arg Arg
           195                 200                 205
   Phe Leu Thr Thr Gly Gln Asn Glu Leu Ala Ala Thr His Phe Pro Lys
       210                 215                 220
   Glu Ala Gln Ala Asn Pro Thr Ile Phe Tyr Asn Lys Asp Gly Ser Pro
   225                 230                 235                 240
   Lys Thr Ile Gln Glu Val Tyr Asn Leu Met Asp Gly Lys Val Ala Ala
                   245                 250                 255
   His Arg Lys
<210> 42
   <211> 259
   <212> PRT
   <213> Artificial Sequence
<220>
   <223> mutated KZ144 with C14R, C50S, T82I, I122M, M149P, A206V and S232T; without N-terminal methionine
<400> 42
   Lys Val Leu Arg Lys Gly Asp Arg Gly Asp Glu Val Arg Gln Leu Gln
   1               5                   10                  15
   Thr Leu Leu Asn Leu Cys Gly Tyr Asp Val Gly Lys Pro Asp Gly Ile
               20                  25                  30
   Phe Gly Asn Asn Thr Phe Asn Gln Val Val Lys Phe Gln Lys Asp Asn
           35                  40                  45
   Ser Leu Asp Ser Asp Gly Ile Val Gly Lys Asn Thr Trp Ala Glu Leu
       50                  55                  60
   Phe Ser Lys Tyr Ser Pro Pro Ile Pro Tyr Lys Thr Ile Pro Met Pro
   65                  70                  75                  80
   Ile Ala Asn Lys Ser Arg Ala Ala Ala Thr Pro Val Met Asn Ala Val
                   85                  90                  95
   Glu Asn Ala Thr Gly Val Arg Ser Gln Leu Leu Leu Thr Phe Ala Ser
               100                 105                 110
   Ile Glu Ser Ala Phe Asp Tyr Glu Met Lys Ala Lys Thr Ser Ser Ala
           115                 120                 125
   Thr Gly Trp Phe Gln Phe Leu Thr Gly Thr Trp Lys Thr Met Ile Glu
       130                 135                 140
   Asn Tyr Gly Pro Lys Tyr Gly Val Leu Thr Asp Pro Thr Gly Ala Leu
   145                 150                 155                 160
   Arg Lys Asp Pro Arg Ile Ser Ala Leu Met Gly Ala Glu Leu Ile Lys
                   165                 170                 175
   Glu Asn Met Asn Ile Leu Arg Pro Val Leu Lys Arg Glu Pro Thr Asp
               180                 185                 190
   Thr Asp Leu Tyr Leu Ala His Phe Phe Gly Pro Gly Val Ala Arg Arg
           195                 200                 205
   Phe Leu Thr Thr Gly Gln Asn Glu Leu Ala Ala Thr His Phe Pro Lys
       210                 215                 220
   Glu Ala Gln Ala Asn Pro Thr Ile Phe Tyr Asn Lys Asp Gly Ser Pro
   225                 230                 235                 240
   Lys Thr Ile Gln Glu Val Tyr Asn Leu Met Asp Gly Lys Val Ala Ala
                   245                 250                 255
   His Arg Lys
<210> 43
   <211> 259
   <212> PRT
   <213> Artificial Sequence
<220>
   <223> mutated KZ144 with C14R, C50S, T82I, I122M, M149P, A160T, A206V and S232T; without N-terminal methionine
<400> 43
   Lys Val Leu Arg Lys Gly Asp Arg Gly Asp Glu Val Arg Gln Leu Gln
   1               5                   10                  15
   Thr Leu Leu Asn Leu Cys Gly Tyr Asp Val Gly Lys Pro Asp Gly Ile
               20                  25                  30
   Phe Gly Asn Asn Thr Phe Asn Gln Val Val Lys Phe Gln Lys Asp Asn
           35                  40                  45
   Ser Leu Asp Ser Asp Gly Ile Val Gly Lys Asn Thr Trp Ala Glu Leu
       50                  55                  60
   Phe Ser Lys Tyr Ser Pro Pro Ile Pro Tyr Lys Thr Ile Pro Met Pro
   65                  70                  75                  80
   Ile Ala Asn Lys Ser Arg Ala Ala Ala Thr Pro Val Met Asn Ala Val
                   85                  90                  95
   Glu Asn Ala Thr Gly Val Arg Ser Gln Leu Leu Leu Thr Phe Ala Ser
               100                 105                 110
   Ile Glu Ser Ala Phe Asp Tyr Glu Met Lys Ala Lys Thr Ser Ser Ala
           115                 120                 125
   Thr Gly Trp Phe Gln Phe Leu Thr Gly Thr Trp Lys Thr Met Ile Glu
       130                 135                 140
   Asn Tyr Gly Pro Lys Tyr Gly Val Leu Thr Asp Pro Thr Gly Thr Leu
   145                 150                 155                 160
   Arg Lys Asp Pro Arg Ile Ser Ala Leu Met Gly Ala Glu Leu Ile Lys
                   165                 170                 175
   Glu Asn Met Asn Ile Leu Arg Pro Val Leu Lys Arg Glu Pro Thr Asp
   180 185 190
   Thr Asp Leu Tyr Leu Ala His Phe Phe Gly Pro Gly Val Ala Arg Arg
   195 200 205
   Phe Leu Thr Thr Gly Gln Asn Glu Leu Ala Ala Thr His Phe Pro Lys
   210 215 220
   Glu Ala Gln Ala Asn Pro Thr Ile Phe Tyr Asn Lys Asp Gly Ser Pro
   225 230 235 240
   Lys Thr Ile Gln Glu Val Tyr Asn Leu Met Asp Gly Lys Val Ala Ala
   245 250 255
   His Arg Lys
<210> 44
   <211> 259
   <212> PRT
   <213> Artificial Sequence
<220>
   <223> mutated KZ144 with T82I, I122M, M149P, A206V, S232T, C14R and C50N, without N-terminal methionine
<400> 44
   Lys Val Leu Arg Lys Gly Asp Arg Gly Asp Glu Val Arg Gln Leu Gln
   1 5 10 15
   Thr Leu Leu Asn Leu Cys Gly Tyr Asp Val Gly Lys Pro Asp Gly Ile
   20 25 30
   Phe Gly Asn Asn Thr Phe Asn Gln Val Val Lys Phe Gln Lys Asp Asn
   35 40 45
   Asn Leu Asp Ser Asp Gly Ile Val Gly Lys Asn Thr Trp Ala Glu Leu
   50 55 60
   Phe Ser Lys Tyr Ser Pro Pro Ile Pro Tyr Lys Thr Ile Pro Met Pro
   65 70 75 80
   Ile Ala Asn Lys Ser Arg Ala Ala Ala Thr Pro Val Met Asn Ala Val
   85 90 95
   Glu Asn Ala Thr Gly Val Arg Ser Gln Leu Leu Leu Thr Phe Ala Ser
   100 105 110
   Ile Glu Ser Ala Phe Asp Tyr Glu Met Lys Ala Lys Thr Ser Ser Ala
           115                 120                 125
   Thr Gly Trp Phe Gln Phe Leu Thr Gly Thr Trp Lys Thr Met Ile Glu
       130                 135                 140
   Asn Tyr Gly Pro Lys Tyr Gly Val Leu Thr Asp Pro Thr Gly Ala Leu
   145                 150                 155                 160
   Arg Lys Asp Pro Arg Ile Ser Ala Leu Met Gly Ala Glu Leu Ile Lys
                   165                 170                 175
   Glu Asn Met Asn Ile Leu Arg Pro Val Leu Lys Arg Glu Pro Thr Asp
               180                 185                 190
   Thr Asp Leu Tyr Leu Ala His Phe Phe Gly Pro Gly Val Ala Arg Arg
           195                 200                 205
   Phe Leu Thr Thr Gly Gln Asn Glu Leu Ala Ala Thr His Phe Pro Lys
       210                 215                 220
   Glu Ala Gln Ala Asn Pro Thr Ile Phe Tyr Asn Lys Asp Gly Ser Pro
   225                 230                 235                 240
   Lys Thr Ile Gln Glu Val Tyr Asn Leu Met Asp Gly Lys Val Ala Ala
                   245                 250                 255
   His Arg Lys
<210> 45
   <211> 259
   <212> PRT
   <213> Artificial Sequence
<220>
   <223> mutated KZ144 with T82I, I122M, M149P, I167L, A206V, S232T, C14R and C50N, without N-terminal methionine
<400> 45
   Lys Val Leu Arg Lys Gly Asp Arg Gly Asp Glu Val Arg Gln Leu Gln
   1               5                   10                  15
   Thr Leu Leu Asn Leu Cys Gly Tyr Asp Val Gly Lys Pro Asp Gly Ile
               20                  25                  30
   Phe Gly Asn Asn Thr Phe Asn Gln Val Val Lys Phe Gln Lys Asp Asn
           35                  40                  45
   Asn Leu Asp Ser Asp Gly Ile Val Gly Lys Asn Thr Trp Ala Glu Leu
       50                  55                  60
   Phe Ser Lys Tyr Ser Pro Pro Ile Pro Tyr Lys Thr Ile Pro Met Pro
   65                  70                  75                  80
   Ile Ala Asn Lys Ser Arg Ala Ala Ala Thr Pro Val Met Asn Ala Val
                   85                  90                  95
   Glu Asn Ala Thr Gly Val Arg Ser Gln Leu Leu Leu Thr Phe Ala Ser
               100                 105                 110
   Ile Glu Ser Ala Phe Asp Tyr Glu Met Lys Ala Lys Thr Ser Ser Ala
           115                 120                 125
   Thr Gly Trp Phe Gln Phe Leu Thr Gly Thr Trp Lys Thr Met Ile Glu
       130                 135                 140
   Asn Tyr Gly Pro Lys Tyr Gly Val Leu Thr Asp Pro Thr Gly Ala Leu
   145                 150                 155                 160
   Arg Lys Asp Pro Arg Leu Ser Ala Leu Met Gly Ala Glu Leu Ile Lys
                   165                 170                 175
   Glu Asn Met Asn Ile Leu Arg Pro Val Leu Lys Arg Glu Pro Thr Asp
               180                 185                 190
   Thr Asp Leu Tyr Leu Ala His Phe Phe Gly Pro Gly Val Ala Arg Arg
           195                 200                 205
   Phe Leu Thr Thr Gly Gln Asn Glu Leu Ala Ala Thr His Phe Pro Lys
       210                 215                 220
   Glu Ala Gln Ala Asn Pro Thr Ile Phe Tyr Asn Lys Asp Gly Ser Pro
   225                 230                 235                 240
   Lys Thr Ile Gln Glu Val Tyr Asn Leu Met Asp Gly Lys Val Ala Ala
                   245                 250                 255
   His Arg Lys
<210> 46
   <211> 259
   <212> PRT
   <213> Artificial Sequence
<220>
   <223> mutated KZ144 with T82I, I122M, M149P, N179F, A206V, S232T, C14R and C50N, without N-terminal methionine
<400> 46
   Lys Val Leu Arg Lys Gly Asp Arg Gly Asp Glu Val Arg Gln Leu Gln
   1               5                   10                  15
   Thr Leu Leu Asn Leu Cys Gly Tyr Asp Val Gly Lys Pro Asp Gly Ile
               20                  25                  30
   Phe Gly Asn Asn Thr Phe Asn Gln Val Val Lys Phe Gln Lys Asp Asn
           35                  40                  45
   Asn Leu Asp Ser Asp Gly Ile Val Gly Lys Asn Thr Trp Ala Glu Leu
       50                  55                  60
   Phe Ser Lys Tyr Ser Pro Pro Ile Pro Tyr Lys Thr Ile Pro Met Pro
   65                  70                  75                  80
   Ile Ala Asn Lys Ser Arg Ala Ala Ala Thr Pro Val Met Asn Ala Val
                   85                  90                  95
   Glu Asn Ala Thr Gly Val Arg Ser Gln Leu Leu Leu Thr Phe Ala Ser
               100                 105                 110
   Ile Glu Ser Ala Phe Asp Tyr Glu Met Lys Ala Lys Thr Ser Ser Ala
           115                 120                 125
   Thr Gly Trp Phe Gln Phe Leu Thr Gly Thr Trp Lys Thr Met Ile Glu
       130                 135                 140
   Asn Tyr Gly Pro Lys Tyr Gly Val Leu Thr Asp Pro Thr Gly Ala Leu
   145                 150                 155                 160
   Arg Lys Asp Pro Arg Leu Ser Ala Leu Met Gly Ala Glu Leu Ile Lys
                   165                 170                 175
   Glu Phe Met Asn Ile Leu Arg Pro Val Leu Lys Arg Glu Pro Thr Asp
               180                 185                 190
   Thr Asp Leu Tyr Leu Ala His Phe Phe Gly Pro Gly Val Ala Arg Arg
           195                 200                 205
   Phe Leu Thr Thr Gly Gln Asn Glu Leu Ala Ala Thr His Phe Pro Lys
       210                 215                 220
   Glu Ala Gln Ala Asn Pro Thr Ile Phe Tyr Asn Lys Asp Gly Ser Pro
   225                 230                 235                 240
   Lys Thr Ile Gln Glu Val Tyr Asn Leu Met Asp Gly Lys Val Ala Ala
   245 250 255
   His Arg Lys
<210> 47
   <211> 259
   <212> PRT
   <213> Artificial Sequence
<220>
   <223> mutated KZ144 with T82I, I122M, M149P, A206V, T212N, S232T, C14R and C50N, without N-terminal methionine
<400> 47
   Lys Val Leu Arg Lys Gly Asp Arg Gly Asp Glu Val Arg Gln Leu Gln
   1 5 10 15
   Thr Leu Leu Asn Leu Cys Gly Tyr Asp Val Gly Lys Pro Asp Gly Ile
   20 25 30
   Phe Gly Asn Asn Thr Phe Asn Gln Val Val Lys Phe Gln Lys Asp Asn
   35 40 45
   Asn Leu Asp Ser Asp Gly Ile Val Gly Lys Asn Thr Trp Ala Glu Leu
   50 55 60
   Phe Ser Lys Tyr Ser Pro Pro Ile Pro Tyr Lys Thr Ile Pro Met Pro
   65 70 75 80
   Ile Ala Asn Lys Ser Arg Ala Ala Ala Thr Pro Val Met Asn Ala Val
   85 90 95
   Glu Asn Ala Thr Gly Val Arg Ser Gln Leu Leu Leu Thr Phe Ala Ser
   100 105 110
   Ile Glu Ser Ala Phe Asp Tyr Glu Met Lys Ala Lys Thr Ser Ser Ala
   115 120 125
   Thr Gly Trp Phe Gln Phe Leu Thr Gly Thr Trp Lys Thr Met Ile Glu
   130 135 140
   Asn Tyr Gly Pro Lys Tyr Gly Val Leu Thr Asp Pro Thr Gly Ala Leu
   145 150 155 160
   Arg Lys Asp Pro Arg Ile Ser Ala Leu Met Gly Ala Glu Leu Ile Lys
   165 170 175
   Glu Asn Met Asn Ile Leu Arg Pro Val Leu Lys Arg Glu Pro Thr Asp
   180 185 190
   Thr Asp Leu Tyr Leu Ala His Phe Phe Gly Pro Gly Val Ala Arg Arg
   195 200 205
   Phe Leu Asn Thr Gly Gln Asn Glu Leu Ala Ala Thr His Phe Pro Lys
   210 215 220
   Glu Ala Gln Ala Asn Pro Thr Ile Phe Tyr Asn Lys Asp Gly Ser Pro
   225 230 235 240
   Lys Thr Ile Gln Glu Val Tyr Asn Leu Met Asp Gly Lys Val Ala Ala
   245 250 255
   His Arg Lys
<210> 48
   <211> 259
   <212> PRT
   <213> Artificial Sequence
<220>
   <223> mutated KZ144 with T82I, I122M, M149P, A206V, P224Q, S232T, C14R and C50N, without N-terminal methionine
<400> 48
   Lys Val Leu Arg Lys Gly Asp Arg Gly Asp Glu Val Arg Gln Leu Gln
   1 5 10 15
   Thr Leu Leu Asn Leu Cys Gly Tyr Asp Val Gly Lys Pro Asp Gly Ile
   20 25 30
   Phe Gly Asn Asn Thr Phe Asn Gln Val Val Lys Phe Gln Lys Asp Asn
   35 40 45
   Asn Leu Asp Ser Asp Gly Ile Val Gly Lys Asn Thr Trp Ala Glu Leu
   50 55 60
   Phe Ser Lys Tyr Ser Pro Pro Ile Pro Tyr Lys Thr Ile Pro Met Pro
   65 70 75 80
   Ile Ala Asn Lys Ser Arg Ala Ala Ala Thr Pro Val Met Asn Ala Val
   85 90 95
   Glu Asn Ala Thr Gly Val Arg Ser Gln Leu Leu Leu Thr Phe Ala Ser
   100 105 110
   Ile Glu Ser Ala Phe Asp Tyr Glu Met Lys Ala Lys Thr Ser Ser Ala
           115                 120                 125
   Thr Gly Trp Phe Gln Phe Leu Thr Gly Thr Trp Lys Thr Met Ile Glu
       130                 135                 140
   Asn Tyr Gly Pro Lys Tyr Gly Val Leu Thr Asp Pro Thr Gly Ala Leu
   145                 150                 155                 160
   Arg Lys Asp Pro Arg Ile Ser Ala Leu Met Gly Ala Glu Leu Ile Lys
                   165                 170                 175
   Glu Asn Met Asn Ile Leu Arg Pro Val Leu Lys Arg Glu Pro Thr Asp
               180                 185                 190
   Thr Asp Leu Tyr Leu Ala His Phe Phe Gly Pro Gly Val Ala Arg Arg
           195                 200                 205
   Phe Leu Thr Thr Gly Gln Asn Glu Leu Ala Ala Thr His Phe Gln Lys
       210                 215                 220
   Glu Ala Gln Ala Asn Pro Thr Ile Phe Tyr Asn Lys Asp Gly Ser Pro
   225                 230                 235                 240
   Lys Thr Ile Gln Glu Val Tyr Asn Leu Met Asp Gly Lys Val Ala Ala
                   245                 250                 255
   His Arg Lys
<210> 49
   <211> 259
   <212> PRT
   <213> Artificial Sequence
<220>
   <223> mutated KZ144 with T82I, I122M, M149P, L154T, A206V, S232T, C14R and C50N, without N-terminal methionine
<400> 49
   Lys Val Leu Arg Lys Gly Asp Arg Gly Asp Glu Val Arg Gln Leu Gln
   1               5                   10                  15
   Thr Leu Leu Asn Leu Cys Gly Tyr Asp Val Gly Lys Pro Asp Gly Ile
               20                  25                  30
   Phe Gly Asn Asn Thr Phe Asn Gln Val Val Lys Phe Gln Lys Asp Asn
           35                  40                  45
   Asn Leu Asp Ser Asp Gly Ile Val Gly Lys Asn Thr Trp Ala Glu Leu
   50 55 60
   Phe Ser Lys Tyr Ser Pro Pro Ile Pro Tyr Lys Thr Ile Pro Met Pro
   65 70 75 80
   Ile Ala Asn Lys Ser Arg Ala Ala Ala Thr Pro Val Met Asn Ala Val
   85 90 95
   Glu Asn Ala Thr Gly Val Arg Ser Gln Leu Leu Leu Thr Phe Ala Ser
   100 105 110
   Ile Glu Ser Ala Phe Asp Tyr Glu Met Lys Ala Lys Thr Ser Ser Ala
   115 120 125
   Thr Gly Trp Phe Gln Phe Leu Thr Gly Thr Trp Lys Thr Met Ile Glu
   130 135 140
   Asn Tyr Gly Pro Lys Tyr Gly Val Thr Thr Asp Pro Thr Gly Ala Leu
   145 150 155 160
   Arg Lys Asp Pro Arg Ile Ser Ala Leu Met Gly Ala Glu Leu Ile Lys
   165 170 175
   Glu Asn Met Asn Ile Leu Arg Pro Val Leu Lys Arg Glu Pro Thr Asp
   180 185 190
   Thr Asp Leu Tyr Leu Ala His Phe Phe Gly Pro Gly Val Ala Arg Arg
   195 200 205
   Phe Leu Thr Thr Gly Gln Asn Glu Leu Ala Ala Thr His Phe Pro Lys
   210 215 220
   Glu Ala Gln Ala Asn Pro Thr Ile Phe Tyr Asn Lys Asp Gly Ser Pro
   225 230 235 240
   Lys Thr Ile Gln Glu Val Tyr Asn Leu Met Asp Gly Lys Val Ala Ala
   245 250 255
   His Arg Lys
<210> 50
   <211> 6
   <212> PRT
   <213> artificial
<220>
   <223> synthetic sequence
<400> 50
   Lys Arg Lys Lys Arg Lys
   1 5
<210> 51
   <211> 5
   <212> PRT
   <213> artificial
<220>
   <223> synethtic sequence
<220>
   <221> misc_feature
   <222> (3)..(3)
   <223> Xaa can be any naturally occurring amino acid
<400> 51
   Lys Arg Xaa Lys Arg
   1 5
<210> 52
   <211> 5
   <212> PRT
   <213> artificial
<220>
   <223> synthetic sequence
<400> 52
   Lys Arg Ser Lys Arg
   1 5
<210> 53
   <211> 5
   <212> PRT
   <213> artificial
<220>
   <223> synthetic sequence
<400> 53
   Lys Arg Gly Ser Gly
   1 5
<210> 54
   <211> 9
   <212> PRT
   <213> artificial
<220>
   <223> synthetic sequence
<400> 54
   Lys Arg Lys Lys Arg Lys Lys Arg Lys
   1 5
<210> 55
   <211> 9
   <212> PRT
   <213> artificial
<220>
   <223> synthetic sequence
<400> 55
   Arg Arg Arg Arg Arg Arg Arg Arg Arg
   1 5
<210> 56
   <211> 8
   <212> PRT
   <213> artificial
<220>
   <223> synthetic sequence
<400> 56
   Lys Lys Lys Lys Lys Lys Lys Lys
   1 5
<210> 57
   <211> 10
   <212> PRT
   <213> artificial
<220>
   <223> synthetic sequence
<400> 57
   Lys Arg Lys Lys Arg Lys Lys Arg Lys Lys
   1 5 10
<210> 58
   <211> 12
   <212> PRT
   <213> artificial
<220>
   <223> synthetic sequence
<400> 58
   Lys Arg Lys Lys Arg Lys Lys Arg Lys Lys Arg Lys
   1 5 10
<210> 59
   <211> 14
   <212> PRT
   <213> artificial
<220>
   <223> synthetic sequence
<400> 59
   Lys Arg Lys Lys Arg Lys Lys Arg Lys Lys Arg Lys Lys Arg
   1               5                   10
<210> 60
   <211> 16
   <212> PRT
   <213> artificial
<220>
   <223> synthetic sequence
<400> 60
   Lys Lys Lys Lys Lys Lys Lys Lys Lys Lys Lys Lys Lys Lys Lys Lys
   1               5                   10                  15
<210> 61
   <211> 18
   <212> PRT
   <213> artificial
<220>
   <223> synthetic sequence
<400> 61
   Lys Arg Lys Lys Arg Lys Lys Arg Lys Lys Arg Lys Lys Arg Lys Lys
   1               5                   10                  15
   Arg Lys
<210> 62
   <211> 19
   <212> PRT
   <213> artificial
<220>
   <223> synthetic sequence
<400> 62
   Lys Arg Lys Lys Arg Lys Lys Arg Lys Lys Arg Lys Lys Arg Lys Lys
   1               5                   10                  15
   Arg Lys Lys
<210> 63
   <211> 19
   <212> PRT
   <213> artificial
<220>
   <223> synthetic sequence
<400> 63
   Arg Arg Arg Arg Arg Arg Arg Arg Arg Arg Arg Arg Arg Arg Arg Arg
   1               5                   10                  15
   Arg Arg Arg
<210> 64
   <211> 19
   <212> PRT
   <213> artificial
<220>
   <223> synthetic sequence
<400> 64
   Lys Lys Lys Lys Lys Lys Lys Lys Lys Lys Lys Lys Lys Lys Lys Lys
   1               5                   10                  15
   Lys Lys Lys
<210> 65
   <211> 20
   <212> PRT
   <213> artificial
<220>
   <223> synthetic sequence
<400> 65
   Lys Arg Lys Lys Arg Lys Lys Arg Lys Arg Ser Lys Arg Lys Lys Arg
   1               5                   10                  15
   Lys Lys Arg Lys
               20
<210> 66
   <211> 21
   <212> PRT
   <213> artificial
<220>
   <223> synthetic sequence
<400> 66
   Lys Arg Lys Lys Arg Lys Lys Arg Lys Arg Ser Lys Arg Lys Lys Arg
   1 5 10 15
   Lys Lys Arg Lys Lys
   20
<210> 67
   <211> 21
   <212> PRT
   <213> artificial
<220>
   <223> synthetic sequence
<400> 67
   Lys Arg Lys Lys Arg Lys Lys Arg Lys Lys Arg Lys Lys Arg Lys Lys
   1 5 10 15
   Arg Lys Lys Arg Lys
   20
<210> 68
   <211> 22
   <212> PRT
   <213> artificial
<220>
   <223> synthetic sequence
<400> 68
   Lys Arg Lys Lys Arg Lys Lys Arg Lys Arg Gly Ser Gly Lys Arg Lys
   1 5 10 15
   Lys Arg Lys Lys Arg Lys
   20
<210> 69
   <211> 24
   <212> PRT
   <213> artificial
<220>
   <223> synthetic sequence
<400> 69
   Lys Arg Lys Lys Arg Lys Lys Arg Lys Arg Gly Ser Gly Ser Gly Lys
   1 5 10 15
   Arg Lys Lys Arg Lys Lys Arg Lys
   20
<210> 70
   <211> 25
   <212> PRT
   <213> artificial
<220>
   <223> synthetic sequence
<400> 70
   Lys Arg Lys Lys Arg Lys Lys Arg Lys Lys Arg Lys Lys Arg Lys Lys
   1               5                   10                  15
   Arg Lys Lys Arg Lys Lys Arg Lys Lys
               20                  25
<210> 71
   <211> 31
   <212> PRT
   <213> artificial
<220>
   <223> synthetic sequence
<400> 71
   Lys Arg Lys Lys Arg Lys Lys Arg Lys Arg Ser Lys Arg Lys Lys Arg
   1               5                   10                  15
   Lys Lys Arg Lys Arg Ser Lys Arg Lys Lys Arg Lys Lys Arg Lys
               20                  25                  30
<210> 72
   <211> 38
   <212> PRT
   <213> artificial
<220>
   <223> synthetic sequence
<400> 72
   Lys Arg Lys Lys Arg Lys Lys Arg Lys Arg Gly Ser Gly Ser Gly Lys
   1               5                   10                  15
   Arg Lys Lys Arg Lys Lys Arg Lys Gly Ser Gly Ser Gly Lys Arg Lys
               20                  25                  30
   Lys Arg Lys Lys Arg Lys
           35
<210> 73
   <211> 39
   <212> PRT
   <213> artificial
<220>
   <223> synthetic sequence
<400> 73
   Lys Arg Lys Lys Arg Lys Lys Arg Lys Lys Arg Lys Lys Arg Lys Lys
   1               5                   10                  15
   Arg Lys Lys Arg Lys Lys Arg Lys Lys Arg Lys Lys Arg Lys Lys Arg
               20                  25                  30
   Lys Lys Arg Lys Lys Arg Lys
           35
<210> 74
   <211> 42
   <212> PRT
   <213> artificial
<220>
   <223> synthetic sequence
<400> 74
   Lys Arg Lys Lys Arg Lys Lys Arg Lys Arg Ser Lys Arg Lys Lys Arg
   1               5                   10                  15
   Lys Lys Arg Lys Arg Ser Lys Arg Lys Lys Arg Lys Lys Arg Lys Arg
               20                  25                  30
   Ser Lys Arg Lys Lys Arg Lys Lys Arg Lys
           35                  40
<210> 75
   <211> 37
   <212> PRT
   <213> Homo sapiens
<400> 75
   Leu Leu Gly Asp Phe Phe Arg Lys Ser Lys Glu Lys Ile Gly Lys Glu
   1               5                   10                  15
   Phe Lys Arg Ile Val Gln Arg Ile Lys Asp Phe Leu Arg Asn Leu Val
               20                  25                  30
   Pro Arg Thr Glu Ser
           35
<210> 76
   <211> 29
   <212> PRT
   <213> unknown
<220>
   <223> SMAP-29 sheep
<400> 76
   Arg Gly Leu Arg Arg Leu Gly Arg Lys Ile Ala His Gly Val Lys Lys
   1               5                   10                  15
   Tyr Gly Pro Thr Val Leu Arg Ile Ile Arg Ile Ala Gly
               20                  25
<210> 77
   <211> 13
   <212> PRT
   <213> unknown
<220>
   <223> Indolicidine bovine
<400> 77
   Ile Leu Pro Trp Lys Trp Pro Trp Trp Pro Trp Arg Arg
   1               5                   10
<210> 78
   <211> 18
   <212> PRT
   <213> unknown
<220>
   <223> Protegrin Porcine
<400> 78
   Arg Gly Gly Arg Leu Cys Tyr Cys Arg Arg Arg Phe Cys Val Cys Val
   1               5                   10                  15
   Gly Arg
<210> 79
   <211> 31
   <212> PRT
   <213> unknown
<220>
   <223> Cecropin P1 Mammal (pig)
<400> 79
   Ser Trp Leu Ser Lys Thr Ala Lys Lys Leu Glu Asn Ser Ala Lys Lys
   1               5                   10                  15
   Arg Ile Ser Glu Gly Ile Ala Ile Ala Ile Gln Gly Gly Pro Arg
               20                  25                  30
<210> 80
   <211> 23
   <212> PRT
   <213> unknown
<220>
   <223> Magainin frog
<400> 80
   Gly Ile Gly Lys Phe Leu His Ser Ala Lys Lys Phe Gly Lys Ala Phe
   1               5                   10                  15
   Val Gly Glu Ile Met Asn Ser
               20
<210> 81
   <211> 25
   <212> PRT
   <213> unknown
<220>
   <223> Pleurocidin fish
<400> 81
   Gly Trp Gly Ser Phe Phe Lys Lys Ala Ala His Val Gly Lys His Val
   1               5                   10                  15
   Gly Lys Ala Ala Leu Thr His Tyr Leu
               20                  25
<210> 82
   <211> 36
   <212> PRT
   <213> Aedes aegypti
<400> 82
   Gly Gly Leu Lys Lys Leu Gly Lys Lys Leu Glu Gly Ala Gly Lys Arg
   1               5                   10                  15
   Val Phe Asn Ala Ala Glu Lys Ala Leu Pro Val Val Ala Gly Ala Lys
               20                  25                  30
   Ala Leu Arg Lys
           35
<210> 83
   <211> 40
   <212> PRT
   <213> Drosophila melanogaster
<400> 83
   Gly Trp Leu Lys Lys Ile Gly Lys Lys Ile Glu Arg Val Gly Gln His
   1               5                   10                  15
   Thr Arg Asp Ala Thr Ile Gln Gly Leu Gly Ile Pro Gln Gln Ala Ala
               20                  25                  30
   Asn Val Ala Ala Thr Ala Arg Gly
           35                  40
<210> 84
   <211> 21
   <212> PRT
   <213> unknown
<220>
   <223> Buforin II vertebrate
<400> 84
   Thr Arg Ser Ser Arg Ala Gly Leu Gln Phe Pro Val Gly Arg Val His
   1               5                   10                  15
   Arg Leu Leu Arg Lys
               20
<210> 85
   <211> 39
   <212> PRT
   <213> unknown
<220>
   <223> Sarcotoxin IA Fly
<400> 85
   Gly Trp Leu Lys Lys Ile Gly Lys Lys Ile Glu Arg Val Gly Gln His
   1               5                   10                  15
   Thr Arg Asp Ala Thr Ile Gln Gly Leu Gly Ile Ala Gln Gln Ala Ala
               20                  25                  30
   Asn Val Ala Ala Thr Ala Arg
           35
<210> 86
   <211> 17
   <212> PRT
   <213> Apis mellifera
<400> 86
   Ala Asn Arg Pro Val Tyr Ile Pro Pro Pro Arg Pro Pro His Pro Arg
   1               5                   10                  15
   Leu
<210> 87
   <211> 24
   <212> PRT
   <213> unknown
<220>
   <223> Ascaphine 5 Frog
<400> 87
   Gly Ile Lys Asp Trp Ile Lys Gly Ala Ala Lys Lys Leu Ile Lys Thr
   1               5                   10                  15
   Val Ala Ser His Ile Ala Asn Gln
               20
<210> 88
   <211> 22
   <212> PRT
   <213> unknown
<220>
   <223> Nigrocine 2 Frog
<400> 88
   Gly Leu Leu Ser Lys Val Leu Gly Val Gly Lys Lys Val Leu Cys Gly
   1               5                   10                  15
   Val Ser Gly Leu Val Cys
               20
<210> 89
   <211> 24
   <212> PRT
   <213> unknown
<220>
   <223> Pseudin 1 Rana Frog
<400> 89
   Gly Leu Asn Thr Leu Lys Lys Val Phe Gln Gly Leu His Glu Ala Ile
   1               5                   10                  15
   Lys Leu Ile Asn Asn His Val Gln
               20
<210> 90
   <211> 18
   <212> PRT
   <213> unknown
<220>
   <223> Ranalexin Frog
<400> 90
   Phe Leu Gly Gly Leu Ile Val Pro Ala Met Ile Cys Ala Val Thr Lys
   1               5                   10                  15
   Lys Cys
<210> 91
   <211> 26
   <212> PRT
   <213> unknown
<220>
   <223> Melittin bee
<400> 91
   Gly Ile Gly Ala Val Leu Lys Val Leu Thr Thr Gly Leu Pro Ala Leu
   1               5                   10                  15
   Ile Ser Trp Ile Lys Arg Lys Arg Gln Gln
               20                  25
<210> 92
   <211> 25
   <212> PRT
   <213> unknown
<220>
   <223> Lycotoxin 1 Spider
<400> 92
   Ile Trp Leu Thr Ala Leu Lys Phe Leu Gly Lys His Ala Ala Lys Lys
   1               5                   10                  15
   Leu Ala Lys Gln Gln Leu Ser Lys Leu
               20                  25
<210> 93
   <211> 19
   <212> PRT
   <213> unknown
<220>
   <223> Parasin 1 Fish
<400> 93
   Lys Gly Arg Gly Lys Gln Gly Gly Lys Val Arg Ala Lys Ala Lys Thr
   1               5                   10                  15
   Arg Ser Ser
<210> 94
   <211> 39
   <212> PRT
   <213> unknown
<220>
   <223> Buforin I Toad
<400> 94
   Ala Gly Arg Gly Lys Gln Gly Gly Lys Val Arg Ala Lys Ala Lys Thr
   1               5                   10                  15
   Arg Ser Ser Arg Ala Gly Leu Gln Phe Pro Val Gly Arg Val His Arg
               20                  25                  30
   Leu Leu Arg Lys Gly Asn Tyr
           35
<210> 95
   <211> 34
   <212> PRT
   <213> unknown
<220>
   <223> Dermaseptin 1 Frog
<400> 95
   Ala Leu Trp Lys Thr Met Leu Lys Lys Leu Gly Thr Met Ala Leu His
   1               5                   10                  15
   Ala Gly Lys Ala Ala Leu Gly Ala Ala Ala Asp Thr Ile Ser Gln Gly
               20                  25                  30
   Thr Gln
<210> 96
   <211> 12
   <212> PRT
   <213> unknown
<220>
   <223> Bactenecin 1 Cow
<400> 96
   Arg Leu Cys Arg Ile Val Val Ile Arg Val Cys Arg
   1               5                   10
<210> 97
   <211> 21
   <212> PRT
   <213> unknown
<220>
   <223> Thanatin Insect
<400> 97
   Gly Ser Lys Lys Pro Val Pro Ile Ile Tyr Cys Asn Arg Arg Thr Gly
   1               5                   10                  15
   Lys Cys Gln Arg Met
               20
<210> 98
   <211> 19
   <212> PRT
   <213> unknown
<220>
   <223> Brevinin 1T Rana frogs
<400> 98
   Val Asn Pro Ile Ile Leu Gly Val Leu Pro Lys Val Cys Leu Ile Thr
   1               5                   10                  15
   Lys Lys Cys
<210> 99
   <211> 26
   <212> PRT
   <213> unknown
<220>
   <223> Ranateurin 1 Rana frog
<400> 99
   Ser Met Leu Ser Val Leu Lys Asn Leu Gly Lys Val Gly Leu Gly Phe
   1               5                   10                  15
   Val Ala Cys Lys Ile Asn Ile Lys Gln Cys
               20                  25
<210> 100
   <211> 46
   <212> PRT
   <213> unknown
<220>
   <223> Esculentin 1 Rana frogs
<400> 100
   Gly Ile Phe Ser Lys Leu Gly Arg Lys Lys Ile Lys Asn Leu Leu Ile
   1               5                   10                  15
   Ser Gly Leu Lys Asn Val Gly Lys Glu Val Gly Met Asp Val Val Arg
               20                  25                  30
   Thr Gly Ile Lys Ile Ala Gly Cys Lys Ile Lys Gly Glu Cys
           35                  40                  45
<210> 101
   <211> 17
   <212> PRT
   <213> Limulus polyphemus
<400> 101
   Arg Trp Cys Phe Arg Val Cys Tyr Arg Gly Ile Cys Tyr Arg Lys Cys
   1               5                   10                  15
   Arg
<210> 102
   <211> 25
   <212> PRT
   <213> unknown
<220>
   <223> Androctonin Scorpion
<400> 102
   Arg Ser Val Cys Arg Gln Ile Lys Ile Cys Arg Arg Arg Gly Gly Cys
   1               5                   10                  15
   Tyr Tyr Lys Cys Thr Asn Arg Pro Tyr
               20                  25
<210> 103
   <211> 30
   <212> PRT
   <213> Homo sapiens
<400> 103
   Asp Cys Tyr Cys Arg Ile Pro Ala Cys Ile Ala Gly Glu Arg Arg Tyr
   1               5                   10                  15
   Gly Thr Cys Ile Tyr Gln Gly Arg Leu Trp Ala Phe Cys Cys
               20                  25                  30
<210> 104
   <211> 38
   <212> PRT
   <213> unknown
<220>
   <223> beta-defensin cow
<400> 104
   Asn Pro Val Ser Cys Val Arg Asn Lys Gly Ile Cys Val Pro Ile Arg
   1               5                   10                  15
   Cys Pro Gly Ser Met Lys Gln Ile Gly Thr Cys Val Gly Arg Ala Val
               20                  25                  30
   Lys Cys Cys Arg Lys Lys
           35
<210> 105
   <211> 18
   <212> PRT
   <213> unknown
<220>
   <223> theta-defensin monkey
<400> 105
   Gly Phe Cys Arg Cys Leu Cys Arg Arg Gly Val Cys Arg Cys Ile Cys
   1               5                   10                  15
   Thr Arg
<210> 106
   <211> 40
   <212> PRT
   <213> unknown
<220>
   <223> defensin (sapecin A) insect
<400> 106
   Ala Thr Cys Asp Leu Leu Ser Gly Thr Gly Ile Asn His Ser Ala Cys
   1               5                   10                  15
   Ala Ala His Cys Leu Leu Arg Gly Asn Arg Gly Gly Tyr Cys Asn Gly
               20                  25                  30
   Lys Ala Val Cys Val Cys Arg Asn
           35                  40
<210> 107
   <211> 46
   <212> PRT
   <213> unknown
<220>
   <223> Thionin (crambin) plant
<400> 107
   Thr Thr Cys Cys Pro Ser Ile Val Ala Arg Ser Asn Phe Asn Val Cys
   1               5                   10                  15
   Arg Ile Pro Gly Thr Pro Glu Ala Ile Cys Ala Thr Tyr Thr Gly Cys
               20                  25                  30
   Ile Ile Ile Pro Gly Ala Thr Cys Pro Gly Asp Tyr Ala Asn
           35                  40                  45
<210> 108
   <211> 50
   <212> PRT
   <213> unknown
<220>
   <223> defensin from radish
<400> 108
   Gln Lys Leu Cys Gln Arg Pro Ser Gly Thr Trp Ser Gly Val Cys Gly
   1               5                   10                  15
   Asn Asn Asn Ala Cys Lys Asn Gln Cys Ile Arg Leu Glu Lys Ala Arg
               20                  25                  30
   His Gly Ser Cys Asn Tyr Val Phe Pro Ala His Cys Ile Cys Tyr Phe
           35                  40                  45
   Pro Cys
       50
<210> 109
   <211> 44
   <212> PRT
   <213> Drosophila melanogaster
<400> 109
   Asp Cys Leu Ser Gly Arg Tyr Lys Gly Pro Cys Ala Val Trp Asp Asn
   1               5                   10                  15
   Glu Thr Cys Arg Arg Val Cys Lys Glu Glu Gly Arg Ser Ser Gly His
               20                  25                  30
   Cys Ser Pro Ser Leu Lys Cys Trp Cys Glu Gly Cys
           35                  40
<210> 110
   <211> 25
   <212> PRT
   <213> Homo sapiens
<400> 110
   Asp Thr His Phe Pro Ile Cys Ile Phe Cys Cys Gly Cys Cys His Arg
   1               5                   10                  15
   Ser Lys Cys Gly Met Cys Cys Lys Thr
               20                  25
<210> 111
   <211> 44
   <212> PRT
   <213> unknown
<220>
   <223> Bac 5 Cow
<400> 111
   Arg Phe Arg Pro Pro Ile Arg Arg Pro Pro Ile Arg Pro Pro Phe Tyr
   1               5                   10                  15
   Pro Pro Phe Arg Pro Pro Ile Arg Pro Pro Ile Phe Pro Pro Ile Arg
               20                  25                  30
   Pro Pro Phe Arg Pro Pro Leu Gly Arg Pro Phe Pro
           35                  40
<210> 112
   <211> 39
   <212> PRT
   <213> unknown
<220>
   <223> PR-39 Pig
<400> 112
   Arg Arg Arg Pro Arg Pro Pro Tyr Leu Pro Arg Pro Arg Pro Pro Pro
   1               5                   10                  15
   Phe Phe Pro Pro Arg Leu Pro Pro Arg Ile Pro Pro Gly Phe Pro Pro
               20                  25                  30
   Arg Phe Pro Pro Arg Phe Pro
           35
<210> 113
   <211> 20
   <212> PRT
   <213> unknown
<220>
   <223> Pyrrhocoricin Insect
<400> 113
   Val Asp Lys Gly Ser Tyr Leu Pro Arg Pro Thr Pro Pro Arg Pro Ile
   1               5                   10                  15
   Tyr Asn Arg Asn
               20
<210> 114
   <211> 24
   <212> PRT
   <213> Homo sapiens
<400> 114
   Asp Ser His Ala Lys Arg His His Gly Tyr Lys Arg Lys Phe His Glu
   1               5                   10                  15
   Lys His His Ser His Arg Gly Tyr
               20
<210> 115
   <211> 34
   <212> PRT
   <213> Limulus polyphemus
<400> 115
   Gly Phe Lys Leu Lys Gly Met Ala Arg Ile Ser Cys Leu Pro Asn Gly
   1 5 10 15
   Gln Trp Ser Asn Phe Pro Pro Lys Cys Ile Arg Glu Cys Ala Met Val
   20 25 30
   Ser Ser
<210> 116
   <211> 18
   <212> PRT
   <213> artificial
<220>
   <223> synthetic sequence
<400> 116
   Gly Phe Phe Ile Pro Ala Val Ile Leu Pro Ser Ile Ala Phe Leu Ile
   1 5 10 15
   Val Pro
<210> 117
   <211> 5
   <212> PRT
   <213> artificial
<220>
   <223> synthetic sequence
<400> 117
   Phe Phe Val Ala Pro
   1 5
<210> 118
   <211> 13
   <212> PRT
   <213> unknown
<220>
   <223> alpha4-helix of T4 lysozyme
<400> 118
   Pro Asn Arg Ala Lys Arg Val Ile Thr Thr Phe Arg Thr
   1 5 10
<210> 119
   <211> 27
   <212> PRT
   <213> artificial
<220>
   <223> synthetic sequence
<400> 119
   Lys Arg Trp Val Lys Arg Val Lys Arg Val Lys Arg Trp Val Lys Arg
   1               5                   10                  15
   Val Val Arg Val Val Lys Arg Trp Val Lys Arg
               20                  25
<210> 120
   <211> 25
   <212> PRT
   <213> artificial
<220>
   <223> synthetic sequence
<400> 120
   Gly Lys Pro Gly Trp Leu Ile Lys Lys Ala Leu Val Phe Lys Lys Leu
   1               5                   10                  15
   Ile Arg Arg Pro Leu Lys Arg Leu Ala
               20                  25
<210> 121
   <211> 291
   <212> PRT
   <213> artificial sequence
<220>
   <223> mutated KZ144 with C14S and C50S plus SMAP-29
<400> 121
   Met Arg Gly Leu Arg Arg Leu Gly Arg Lys Ile Ala His Gly Val Lys
   1               5                   10                  15
   Lys Tyr Gly Pro Thr Val Leu Arg Ile Ile Arg Ile Ala Gly Gly Ser
               20                  25                  30
   Lys Val Leu Arg Lys Gly Asp Arg Gly Asp Glu Val Ser Gln Leu Gln
           35                  40                  45
   Thr Leu Leu Asn Leu Cys Gly Tyr Asp Val Gly Lys Pro Asp Gly Ile
       50                  55                  60
   Phe Gly Asn Asn Thr Phe Asn Gln Val Val Lys Phe Gln Lys Asp Asn
   65                  70                  75                  80
   Ser Leu Asp Ser Asp Gly Ile Val Gly Lys Asn Thr Trp Ala Glu Leu
                   85                  90                  95
   Phe Ser Lys Tyr Ser Pro Pro Ile Pro Tyr Lys Thr Ile Pro Met Pro
               100                 105                 110
   Thr Ala Asn Lys Ser Arg Ala Ala Ala Thr Pro Val Met Asn Ala Val
           115                 120                 125
   Glu Asn Ala Thr Gly Val Arg Ser Gln Leu Leu Leu Thr Phe Ala Ser
       130                 135                 140
   Ile Glu Ser Ala Phe Asp Tyr Glu Ile Lys Ala Lys Thr Ser Ser Ala
   145                 150                 155                 160
   Thr Gly Trp Phe Gln Phe Leu Thr Gly Thr Trp Lys Thr Met Ile Glu
                   165                 170                 175
   Asn Tyr Gly Met Lys Tyr Gly Val Leu Thr Asp Pro Thr Gly Ala Leu
               180                 185                 190
   Arg Lys Asp Pro Arg Ile Ser Ala Leu Met Gly Ala Glu Leu Ile Lys
               195                 200                 205
   Glu Asn Met Asn Ile Leu Arg Pro Val Leu Lys Arg Glu Pro Thr Asp
       210                 215                 220
   Thr Asp Leu Tyr Leu Ala His Phe Phe Gly Pro Gly Ala Ala Arg Arg
   225                 230                 235                 240
   Phe Leu Thr Thr Gly Gln Asn Glu Leu Ala Ala Thr His Phe Pro Lys
                   245                 250                 255
   Glu Ala Gln Ala Asn Pro Ser Ile Phe Tyr Asn Lys Asp Gly Ser Pro
               260                 265                 270
   Lys Thr Ile Gln Glu Val Tyr Asn Leu Met Asp Gly Lys Val Ala Ala
           275                 280                 285
   His Arg Lys
       290
<210> 122
   <211> 291
   <212> PRT
   <213> artificial sequence
<220>
   <223> mutated KZ144 with T82I, A206V and S232 plus SMAP-29
<400> 122
   Met Arg Gly Leu Arg Arg Leu Gly Arg Lys Ile Ala His Gly Val Lys
   1               5                   10                  15
   Lys Tyr Gly Pro Thr Val Leu Arg Ile Ile Arg Ile Ala Gly Gly Ser
               20                  25                  30
   Lys Val Leu Arg Lys Gly Asp Arg Gly Asp Glu Val Cys Gln Leu Gln
           35                  40                  45
   Thr Leu Leu Asn Leu Cys Gly Tyr Asp Val Gly Lys Pro Asp Gly Ile
       50                  55                  60
   Phe Gly Asn Asn Thr Phe Asn Gln Val Val Lys Phe Gln Lys Asp Asn
   65                  70                  75                  80
   Cys Leu Asp Ser Asp Gly Ile Val Gly Lys Asn Thr Trp Ala Glu Leu
                   85                  90                  95
   Phe Ser Lys Tyr Ser Pro Pro Ile Pro Tyr Lys Thr Ile Pro Met Pro
               100                 105                 110
   Ile Ala Asn Lys Ser Arg Ala Ala Ala Thr Pro Val Met Asn Ala Val
               115                 120                 125
   Glu Asn Ala Thr Gly Val Arg Ser Gln Leu Leu Leu Thr Phe Ala Ser
       130                 135                 140
   Ile Glu Ser Ala Phe Asp Tyr Glu Ile Lys Ala Lys Thr Ser Ser Ala
   145                 150                 155                 160
   Thr Gly Trp Phe Gln Phe Leu Thr Gly Thr Trp Lys Thr Met Ile Glu
                   165                 170                 175
   Asn Tyr Gly Met Lys Tyr Gly Val Leu Thr Asp Pro Thr Gly Ala Leu
               180                 185                 190
   Arg Lys Asp Pro Arg Ile Ser Ala Leu Met Gly Ala Glu Leu Ile Lys
           195                 200                 205
   Glu Asn Met Asn Ile Leu Arg Pro Val Leu Lys Arg Glu Pro Thr Asp
       210                 215                 220
   Thr Asp Leu Tyr Leu Ala His Phe Phe Gly Pro Gly Val Ala Arg Arg
   225                 230                 235                 240
   Phe Leu Thr Thr Gly Gln Asn Glu Leu Ala Ala Thr His Phe Pro Lys
                   245                 250                 255
   Glu Ala Gln Ala Asn Pro Thr Ile Phe Tyr Asn Lys Asp Gly Ser Pro
               260                 265                 270
   Lys Thr Ile Gln Glu Val Tyr Asn Leu Met Asp Gly Lys Val Ala Ala
           275                 280                 285
   His Arg Lys
       290
<210> 123
   <211> 291
   <212> PRT
   <213> artificial sequence
<220>
   <223> mutated KZ144 with T82I, A206V, S232T, I122M and A160T plus SMAP-29
<400> 123
   Met Arg Gly Leu Arg Arg Leu Gly Arg Lys Ile Ala His Gly Val Lys
   1               5                   10                  15
   Lys Tyr Gly Pro Thr Val Leu Arg Ile Ile Arg Ile Ala Gly Gly Ser
               20                  25                  30
   Lys Val Leu Arg Lys Gly Asp Arg Gly Asp Glu Val Cys Gln Leu Gln
           35                  40                  45
   Thr Leu Leu Asn Leu Cys Gly Tyr Asp Val Gly Lys Pro Asp Gly Ile
       50                  55                  60
   Phe Gly Asn Asn Thr Phe Asn Gln Val Val Lys Phe Gln Lys Asp Asn
   65                  70                  75                  80
   Cys Leu Asp Ser Asp Gly Ile Val Gly Lys Asn Thr Trp Ala Glu Leu
                   85                  90                  95
   Phe Ser Lys Tyr Ser Pro Pro Ile Pro Tyr Lys Thr Ile Pro Met Pro
               100                 105                 110
   Ile Ala Asn Lys Ser Arg Ala Ala Ala Thr Pro Val Met Asn Ala Val
           115                 120                 125
   Glu Asn Ala Thr Gly Val Arg Ser Gln Leu Leu Leu Thr Phe Ala Ser
       130                 135                 140
   Ile Glu Ser Ala Phe Asp Tyr Glu Met Lys Ala Lys Thr Ser Ser Ala
   145                 150                 155                 160
   Thr Gly Trp Phe Gln Phe Leu Thr Gly Thr Trp Lys Thr Met Ile Glu
                   165                 170                 175
   Asn Tyr Gly Met Lys Tyr Gly Val Leu Thr Asp Pro Thr Gly Thr Leu
               180                 185                 190
   Arg Lys Asp Pro Arg Ile Ser Ala Leu Met Gly Ala Glu Leu Ile Lys
           195                 200                 205
   Glu Asn Met Asn Ile Leu Arg Pro Val Leu Lys Arg Glu Pro Thr Asp
       210                 215                 220
   Thr Asp Leu Tyr Leu Ala His Phe Phe Gly Pro Gly Val Ala Arg Arg
   225                 230                 235                 240
   Phe Leu Thr Thr Gly Gln Asn Glu Leu Ala Ala Thr His Phe Pro Lys
                   245                 250                 255
   Glu Ala Gln Ala Asn Pro Thr Ile Phe Tyr Asn Lys Asp Gly Ser Pro
   260 265 270
   Lys Thr Ile Gln Glu Val Tyr Asn Leu Met Asp Gly Lys Val Ala Ala
   275 280 285
   His Arg Lys
   290
<210> 124
   <211> 291
   <212> PRT
   <213> artificial sequence
<220>
   <223> mutated KZ144 with C14S, C50S, I122M and A160T plus SMAP-29
<400> 124
   Met Arg Gly Leu Arg Arg Leu Gly Arg Lys Ile Ala His Gly Val Lys
   1               5                   10                  15
   Lys Tyr Gly Pro Thr Val Leu Arg Ile Ile Arg Ile Ala Gly Gly Ser
               20                  25                  30
   Lys Val Leu Arg Lys Gly Asp Arg Gly Asp Glu Val Ser Gln Leu Gln
           35                  40                  45
   Thr Leu Leu Asn Leu Cys Gly Tyr Asp Val Gly Lys Pro Asp Gly Ile
       50                  55                  60
   Phe Gly Asn Asn Thr Phe Asn Gln Val Val Lys Phe Gln Lys Asp Asn
   65                  70                  75                  80
   Ser Leu Asp Ser Asp Gly Ile Val Gly Lys Asn Thr Trp Ala Glu Leu
                   85                  90                  95
   Phe Ser Lys Tyr Ser Pro Pro Ile Pro Tyr Lys Thr Ile Pro Met Pro
               100                 105                 110
   Thr Ala Asn Lys Ser Arg Ala Ala Ala Thr Pro Val Met Asn Ala Val
           115                 120                 125
   Glu Asn Ala Thr Gly Val Arg Ser Gln Leu Leu Leu Thr Phe Ala Ser
       130                 135                 140
   Ile Glu Ser Ala Phe Asp Tyr Glu Met Lys Ala Lys Thr Ser Ser Ala
   145                 150                 155                 160
   Thr Gly Trp Phe Gln Phe Leu Thr Gly Thr Trp Lys Thr Met Ile Glu
                   165                 170                 175
   Asn Tyr Gly Met Lys Tyr Gly Val Leu Thr Asp Pro Thr Gly Thr Leu
   180 185 190
   Arg Lys Asp Pro Arg Ile Ser Ala Leu Met Gly Ala Glu Leu Ile Lys
   195 200 205
   Glu Asn Met Asn Ile Leu Arg Pro Val Leu Lys Arg Glu Pro Thr Asp
   210 215 220
   Thr Asp Leu Tyr Leu Ala His Phe Phe Gly Pro Gly Ala Ala Arg Arg
   225 230 235 240
   Phe Leu Thr Thr Gly Gln Asn Glu Leu Ala Ala Thr His Phe Pro Lys
   245 250 255
   Glu Ala Gln Ala Asn Pro Ser Ile Phe Tyr Asn Lys Asp Gly Ser Pro
   260 265 270
   Lys Thr Ile Gln Glu Val Tyr Asn Leu Met Asp Gly Lys Val Ala Ala
   275 280 285
   His Arg Lys
   290
<210> 125
   <211> 291
   <212> PRT
   <213> artificial sequence
<220>
   <223> mutated KZ144 with C14S, C23S and C50S plus SMAP-29
<400> 125
   Met Arg Gly Leu Arg Arg Leu Gly Arg Lys Ile Ala His Gly Val Lys
   1 5 10 15
   Lys Tyr Gly Pro Thr Val Leu Arg Ile Ile Arg Ile Ala Gly Gly Ser
   20 25 30
   Lys Val Leu Arg Lys Gly Asp Arg Gly Asp Glu Val Ser Gln Leu Gln
   35 40 45
   Thr Leu Leu Asn Leu Ser Gly Tyr Asp Val Gly Lys Pro Asp Gly Ile
   50 55 60
   Phe Gly Asn Asn Thr Phe Asn Gln Val Val Lys Phe Gln Lys Asp Asn
   65 70 75 80
   Ser Leu Asp Ser Asp Gly Ile Val Gly Lys Asn Thr Trp Ala Glu Leu
                   85                  90                  95
   Phe Ser Lys Tyr Ser Pro Pro Ile Pro Tyr Lys Thr Ile Pro Met Pro
               100                 105                 110
   Thr Ala Asn Lys Ser Arg Ala Ala Ala Thr Pro Val Met Asn Ala Val
           115                 120                 125
   Glu Asn Ala Thr Gly Val Arg Ser Gln Leu Leu Leu Thr Phe Ala Ser
       130                 135                 140
   Ile Glu Ser Ala Phe Asp Tyr Glu Ile Lys Ala Lys Thr Ser Ser Ala
   145                 150                 155                 160
   Thr Gly Trp Phe Gln Phe Leu Thr Gly Thr Trp Lys Thr Met Ile Glu
                   165                 170                 175
   Asn Tyr Gly Met Lys Tyr Gly Val Leu Thr Asp Pro Thr Gly Ala Leu
               180                 185                 190
   Arg Lys Asp Pro Arg Ile Ser Ala Leu Met Gly Ala Glu Leu Ile Lys
           195                 200                 205
   Glu Asn Met Asn Ile Leu Arg Pro Val Leu Lys Arg Glu Pro Thr Asp
       210                 215                 220
   Thr Asp Leu Tyr Leu Ala His Phe Phe Gly Pro Gly Ala Ala Arg Arg
   225                 230                 235                 240
   Phe Leu Thr Thr Gly Gln Asn Glu Leu Ala Ala Thr His Phe Pro Lys
                   245                 250                 255
   Glu Ala Gln Ala Asn Pro Ser Ile Phe Tyr Asn Lys Asp Gly Ser Pro
               260                 265                 270
   Lys Thr Ile Gln Glu Val Tyr Asn Leu Met Asp Gly Lys Val Ala Ala
           275                 280                 285
   His Arg Lys
       290
<210> 126
   <211> 291
   <212> PRT
   <213> artificial sequence
<220>
   <223> mutated KZ144 with T82I, A206V, S232T, I122M, A160T, C14S and C50S plus SMAP-29
<400> 126
   Met Arg Gly Leu Arg Arg Leu Gly Arg Lys Ile Ala His Gly Val Lys
   1               5                   10                  15
   Lys Tyr Gly Pro Thr Val Leu Arg Ile Ile Arg Ile Ala Gly Gly Ser
               20                  25                  30
   Lys Val Leu Arg Lys Gly Asp Arg Gly Asp Glu Val Ser Gln Leu Gln
           35                  40                  45
   Thr Leu Leu Asn Leu Cys Gly Tyr Asp Val Gly Lys Pro Asp Gly Ile
       50                  55                  60
   Phe Gly Asn Asn Thr Phe Asn Gln Val Val Lys Phe Gln Lys Asp Asn
   65                  70                  75                  80
   Ser Leu Asp Ser Asp Gly Ile Val Gly Lys Asn Thr Trp Ala Glu Leu
                   85                  90                  95
   Phe Ser Lys Tyr Ser Pro Pro Ile Pro Tyr Lys Thr Ile Pro Met Pro
               100                 105                 110
   Ile Ala Asn Lys Ser Arg Ala Ala Ala Thr Pro Val Met Asn Ala Val
           115                 120                 125
   Glu Asn Ala Thr Gly Val Arg Ser Gln Leu Leu Leu Thr Phe Ala Ser
       130                 135                 140
   Ile Glu Ser Ala Phe Asp Tyr Glu Met Lys Ala Lys Thr Ser Ser Ala
   145                 150                 155                 160
   Thr Gly Trp Phe Gln Phe Leu Thr Gly Thr Trp Lys Thr Met Ile Glu
                   165                 170                 175
   Asn Tyr Gly Met Lys Tyr Gly Val Leu Thr Asp Pro Thr Gly Thr Leu
               180                 185                 190
   Arg Lys Asp Pro Arg Ile Ser Ala Leu Met Gly Ala Glu Leu Ile Lys
           195                 200                 205
   Glu Asn Met Asn Ile Leu Arg Pro Val Leu Lys Arg Glu Pro Thr Asp
       210                 215                 220
   Thr Asp Leu Tyr Leu Ala His Phe Phe Gly Pro Gly Val Ala Arg Arg
   225                 230                 235                 240
   Phe Leu Thr Thr Gly Gln Asn Glu Leu Ala Ala Thr His Phe Pro Lys
   245 250 255
   Glu Ala Gln Ala Asn Pro Thr Ile Phe Tyr Asn Lys Asp Gly Ser Pro
   260 265 270
   Lys Thr Ile Gln Glu Val Tyr Asn Leu Met Asp Gly Lys Val Ala Ala
   275 280 285
   His Arg Lys
   290
<210> 127
   <211> 291
   <212> PRT
   <213> artificial sequence
<220>
   <223> mutated KZ144 with T82I, A206N, S232T, I122M, A160T, C14S and C50S plus SMAP-29
<400> 127
   Met Arg Gly Leu Arg Arg Leu Gly Arg Lys Ile Ala His Gly Val Lys
   1 5 10 15
   Lys Tyr Gly Pro Thr Val Leu Arg Ile Ile Arg Ile Ala Gly Gly Ser
   20 25 30
   Lys Val Leu Arg Lys Gly Asp Arg Gly Asp Glu Val Ser Gln Leu Gln
   35 40 45
   Thr Leu Leu Asn Leu Cys Gly Tyr Asp Val Gly Lys Pro Asp Gly Ile
   50 55 60
   Phe Gly Asn Asn Thr Phe Asn Gln Val Val Lys Phe Gln Lys Asp Asn
   65 70 75 80
   Ser Leu Asp Ser Asp Gly Ile Val Gly Lys Asn Thr Trp Ala Glu Leu
   85 90 95
   Phe Ser Lys Tyr Ser Pro Pro Ile Pro Tyr Lys Thr Ile Pro Met Pro
   100 105 110
   Ile Ala Asn Lys Ser Arg Ala Ala Ala Thr Pro Val Met Asn Ala Val
   115 120 125
   Glu Asn Ala Thr Gly Val Arg Ser Gln Leu Leu Leu Thr Phe Ala Ser
   130 135 140
   Ile Glu Ser Ala Phe Asp Tyr Glu Met Lys Ala Lys Thr Ser Ser Ala
   145 150 155 160
   Thr Gly Trp Phe Gln Phe Leu Thr Gly Thr Trp Lys Thr Met Ile Glu
   165 170 175
   Asn Tyr Gly Met Lys Tyr Gly Val Leu Thr Asp Pro Thr Gly Thr Leu
   180 185 190
   Arg Lys Asp Pro Arg Ile Ser Ala Leu Met Gly Ala Glu Leu Ile Lys
   195 200 205
   Glu Asn Met Asn Ile Leu Arg Pro Val Leu Lys Arg Glu Pro Thr Asp
   210 215 220
   Thr Asp Leu Tyr Leu Ala His Phe Phe Gly Pro Gly Asn Ala Arg Arg
   225 230 235 240
   Phe Leu Thr Thr Gly Gln Asn Glu Leu Ala Ala Thr His Phe Pro Lys
   245 250 255
   Glu Ala Gln Ala Asn Pro Thr Ile Phe Tyr Asn Lys Asp Gly Ser Pro
   260 265 270
   Lys Thr Ile Gln Glu Val Tyr Asn Leu Met Asp Gly Lys Val Ala Ala
   275 280 285
   His Arg Lys
   290
<210> 128
   <211> 290
   <212> PRT
   <213> artificial sequence
<220>
   <223> mutated KZ144 with C14S and C50S plus SMAP-29 without Met
<400> 128
   Arg Gly Leu Arg Arg Leu Gly Arg Lys Ile Ala His Gly Val Lys Lys
   1 5 10 15
   Tyr Gly Pro Thr Val Leu Arg Ile Ile Arg Ile Ala Gly Gly Ser Lys
   20 25 30
   Val Leu Arg Lys Gly Asp Arg Gly Asp Glu Val Ser Gln Leu Gln Thr
   35 40 45
   Leu Leu Asn Leu Cys Gly Tyr Asp Val Gly Lys Pro Asp Gly Ile Phe
       50                  55                  60
   Gly Asn Asn Thr Phe Asn Gln Val Val Lys Phe Gln Lys Asp Asn Ser
   65                  70                  75                  80
   Leu Asp Ser Asp Gly Ile Val Gly Lys Asn Thr Trp Ala Glu Leu Phe
                   85                  90                  95
   Ser Lys Tyr Ser Pro Pro Ile Pro Tyr Lys Thr Ile Pro Met Pro Thr
               100                 105                 110
   Ala Asn Lys Ser Arg Ala Ala Ala Thr Pro Val Met Asn Ala Val Glu
           115                 120                 125
   Asn Ala Thr Gly Val Arg Ser Gln Leu Leu Leu Thr Phe Ala Ser Ile
       130                 135                 140
   Glu Ser Ala Phe Asp Tyr Glu Ile Lys Ala Lys Thr Ser Ser Ala Thr
   145                 150                 155                 160
   Gly Trp Phe Gln Phe Leu Thr Gly Thr Trp Lys Thr Met Ile Glu Asn
                   165                 170                 175
   Tyr Gly Met Lys Tyr Gly Val Leu Thr Asp Pro Thr Gly Ala Leu Arg
               180                 185                 190
   Lys Asp Pro Arg Ile Ser Ala Leu Met Gly Ala Glu Leu Ile Lys Glu
           195                 200                 205
   Asn Met Asn Ile Leu Arg Pro Val Leu Lys Arg Glu Pro Thr Asp Thr
       210                 215                 220
   Asp Leu Tyr Leu Ala His Phe Phe Gly Pro Gly Ala Ala Arg Arg Phe
   225                 230                 235                 240
   Leu Thr Thr Gly Gln Asn Glu Leu Ala Ala Thr His Phe Pro Lys Glu
                   245                 250                 255
   Ala Gln Ala Asn Pro Ser Ile Phe Tyr Asn Lys Asp Gly Ser Pro Lys
               260                 265                 270
   Thr Ile Gln Glu Val Tyr Asn Leu Met Asp Gly Lys Val Ala Ala His
           275                 280                 285
   Arg Lys
       290
<210> 129
   <211> 290
   <212> PRT
   <213> artificial sequence
<220>
   <223> mutated KZ144 with T82I, A206V and S232 plus SMAP-29 without Met
<400> 129
   Arg Gly Leu Arg Arg Leu Gly Arg Lys Ile Ala His Gly Val Lys Lys
   1               5                   10                  15
   Tyr Gly Pro Thr Val Leu Arg Ile Ile Arg Ile Ala Gly Gly Ser Lys
               20                  25                  30
   Val Leu Arg Lys Gly Asp Arg Gly Asp Glu Val Cys Gln Leu Gln Thr
           35                  40                  45
   Leu Leu Asn Leu Cys Gly Tyr Asp Val Gly Lys Pro Asp Gly Ile Phe
       50                  55                  60
   Gly Asn Asn Thr Phe Asn Gln Val Val Lys Phe Gln Lys Asp Asn Cys
   65                  70                  75                  80
   Leu Asp Ser Asp Gly Ile Val Gly Lys Asn Thr Trp Ala Glu Leu Phe
                   85                  90                  95
   Ser Lys Tyr Ser Pro Pro Ile Pro Tyr Lys Thr Ile Pro Met Pro Ile
               100                 105                 110
   Ala Asn Lys Ser Arg Ala Ala Ala Thr Pro Val Met Asn Ala Val Glu
           115                 120                 125
   Asn Ala Thr Gly Val Arg Ser Gln Leu Leu Leu Thr Phe Ala Ser Ile
       130                 135                 140
   Glu Ser Ala Phe Asp Tyr Glu Ile Lys Ala Lys Thr Ser Ser Ala Thr
   145                 150                 155                 160
   Gly Trp Phe Gln Phe Leu Thr Gly Thr Trp Lys Thr Met Ile Glu Asn
                   165                 170                 175
   Tyr Gly Met Lys Tyr Gly Val Leu Thr Asp Pro Thr Gly Ala Leu Arg
               180                 185                 190
   Lys Asp Pro Arg Ile Ser Ala Leu Met Gly Ala Glu Leu Ile Lys Glu
           195                 200                 205
   Asn Met Asn Ile Leu Arg Pro Val Leu Lys Arg Glu Pro Thr Asp Thr
       210                 215                 220
   Asp Leu Tyr Leu Ala His Phe Phe Gly Pro Gly Val Ala Arg Arg Phe
   225                 230                 235                 240
   Leu Thr Thr Gly Gln Asn Glu Leu Ala Ala Thr His Phe Pro Lys Glu
                   245                 250                 255
   Ala Gln Ala Asn Pro Thr Ile Phe Tyr Asn Lys Asp Gly Ser Pro Lys
               260                 265                 270
   Thr Ile Gln Glu Val Tyr Asn Leu Met Asp Gly Lys Val Ala Ala His
           275                 280                 285
   Arg Lys
       290
<210> 130
   <211> 290
   <212> PRT
   <213> artificial sequence
<220>
   <223> mutated KZ144 with T82I, A206V, S232T, I122M and A160T plus SMAP-29 without Met
<400> 130
   Arg Gly Leu Arg Arg Leu Gly Arg Lys Ile Ala His Gly Val Lys Lys
   1               5                   10                  15
   Tyr Gly Pro Thr Val Leu Arg Ile Ile Arg Ile Ala Gly Gly Ser Lys
               20                  25                  30
   Val Leu Arg Lys Gly Asp Arg Gly Asp Glu Val Cys Gln Leu Gln Thr
           35                  40                  45
   Leu Leu Asn Leu Cys Gly Tyr Asp Val Gly Lys Pro Asp Gly Ile Phe
       50                  55                  60
   Gly Asn Asn Thr Phe Asn Gln Val Val Lys Phe Gln Lys Asp Asn Cys
   65                  70                  75                  80
   Leu Asp Ser Asp Gly Ile Val Gly Lys Asn Thr Trp Ala Glu Leu Phe
                   85                  90                  95
   Ser Lys Tyr Ser Pro Pro Ile Pro Tyr Lys Thr Ile Pro Met Pro Ile
               100                 105                 110
   Ala Asn Lys Ser Arg Ala Ala Ala Thr Pro Val Met Asn Ala Val Glu
   115 120 125
   Asn Ala Thr Gly Val Arg Ser Gln Leu Leu Leu Thr Phe Ala Ser Ile
   130 135 140
   Glu Ser Ala Phe Asp Tyr Glu Met Lys Ala Lys Thr Ser Ser Ala Thr
   145 150 155 160
   Gly Trp Phe Gln Phe Leu Thr Gly Thr Trp Lys Thr Met Ile Glu Asn
   165 170 175
   Tyr Gly Met Lys Tyr Gly Val Leu Thr Asp Pro Thr Gly Thr Leu Arg
   180 185 190
   Lys Asp Pro Arg Ile Ser Ala Leu Met Gly Ala Glu Leu Ile Lys Glu
   195 200 205
   Asn Met Asn Ile Leu Arg Pro Val Leu Lys Arg Glu Pro Thr Asp Thr
   210 215 220
   Asp Leu Tyr Leu Ala His Phe Phe Gly Pro Gly Val Ala Arg Arg Phe
   225 230 235 240
   Leu Thr Thr Gly Gln Asn Glu Leu Ala Ala Thr His Phe Pro Lys Glu
   245 250 255
   Ala Gln Ala Asn Pro Thr Ile Phe Tyr Asn Lys Asp Gly Ser Pro Lys
   260 265 270
   Thr Ile Gln Glu Val Tyr Asn Leu Met Asp Gly Lys Val Ala Ala His
   275 280 285
   Arg Lys
   290
<210> 131
   <211> 290
   <212> PRT
   <213> artificial sequence
<220>
   <223> mutated KZ144 with C14S, C50S, I122M and A160T plus SMAP-29 without Met
<400> 131
   Arg Gly Leu Arg Arg Leu Gly Arg Lys Ile Ala His Gly Val Lys Lys
   1 5 10 15
   Tyr Gly Pro Thr Val Leu Arg Ile Ile Arg Ile Ala Gly Gly Ser Lys
   20 25 30
   Val Leu Arg Lys Gly Asp Arg Gly Asp Glu Val Ser Gln Leu Gln Thr
   35 40 45
   Leu Leu Asn Leu Cys Gly Tyr Asp Val Gly Lys Pro Asp Gly Ile Phe
   50 55 60
   Gly Asn Asn Thr Phe Asn Gln Val Val Lys Phe Gln Lys Asp Asn Ser
   65 70 75 80
   Leu Asp Ser Asp Gly Ile Val Gly Lys Asn Thr Trp Ala Glu Leu Phe
   85 90 95
   Ser Lys Tyr Ser Pro Pro Ile Pro Tyr Lys Thr Ile Pro Met Pro Thr
   100 105 110
   Ala Asn Lys Ser Arg Ala Ala Ala Thr Pro Val Met Asn Ala Val Glu
   115 120 125
   Asn Ala Thr Gly Val Arg Ser Gln Leu Leu Leu Thr Phe Ala Ser Ile
   130 135 140
   Glu Ser Ala Phe Asp Tyr Glu Met Lys Ala Lys Thr Ser Ser Ala Thr
   145 150 155 160
   Gly Trp Phe Gln Phe Leu Thr Gly Thr Trp Lys Thr Met Ile Glu Asn
   165 170 175
   Tyr Gly Met Lys Tyr Gly Val Leu Thr Asp Pro Thr Gly Thr Leu Arg
   180 185 190
   Lys Asp Pro Arg Ile Ser Ala Leu Met Gly Ala Glu Leu Ile Lys Glu
   195 200 205
   Asn Met Asn Ile Leu Arg Pro Val Leu Lys Arg Glu Pro Thr Asp Thr
   210 215 220
   Asp Leu Tyr Leu Ala His Phe Phe Gly Pro Gly Ala Ala Arg Arg Phe
   225 230 235 240
   Leu Thr Thr Gly Gln Asn Glu Leu Ala Ala Thr His Phe Pro Lys Glu
   245 250 255
   Ala Gln Ala Asn Pro Ser Ile Phe Tyr Asn Lys Asp Gly Ser Pro Lys
   260 265 270
   Thr Ile Gln Glu Val Tyr Asn Leu Met Asp Gly Lys Val Ala Ala His
           275                 280                 285
   Arg Lys
       290
<210> 132
   <211> 290
   <212> PRT
   <213> artificial sequence
<220>
   <223> mutated KZ144 with C14S, C23S and C50S plus SMAP-29 w/o Met
<400> 132
   Arg Gly Leu Arg Arg Leu Gly Arg Lys Ile Ala His Gly Val Lys Lys
   1               5                   10                  15
   Tyr Gly Pro Thr Val Leu Arg Ile Ile Arg Ile Ala Gly Gly Ser Lys
               20                  25                  30
   Val Leu Arg Lys Gly Asp Arg Gly Asp Glu Val Ser Gln Leu Gln Thr
           35                  40                  45
   Leu Leu Asn Leu Ser Gly Tyr Asp Val Gly Lys Pro Asp Gly Ile Phe
       50                  55                  60
   Gly Asn Asn Thr Phe Asn Gln Val Val Lys Phe Gln Lys Asp Asn Ser
   65                  70                  75                  80
   Leu Asp Ser Asp Gly Ile Val Gly Lys Asn Thr Trp Ala Glu Leu Phe
                   85                  90                  95
   Ser Lys Tyr Ser Pro Pro Ile Pro Tyr Lys Thr Ile Pro Met Pro Thr
               100                 105                 110
   Ala Asn Lys Ser Arg Ala Ala Ala Thr Pro Val Met Asn Ala Val Glu
           115                 120                 125
   Asn Ala Thr Gly Val Arg Ser Gln Leu Leu Leu Thr Phe Ala Ser Ile
       130                 135                 140
   Glu Ser Ala Phe Asp Tyr Glu Ile Lys Ala Lys Thr Ser Ser Ala Thr
   145                 150                 155                 160
   Gly Trp Phe Gln Phe Leu Thr Gly Thr Trp Lys Thr Met Ile Glu Asn
                   165                 170                 175
   Tyr Gly Met Lys Tyr Gly Val Leu Thr Asp Pro Thr Gly Ala Leu Arg
   180 185 190
   Lys Asp Pro Arg Ile Ser Ala Leu Met Gly Ala Glu Leu Ile Lys Glu
   195 200 205
   Asn Met Asn Ile Leu Arg Pro Val Leu Lys Arg Glu Pro Thr Asp Thr
   210 215 220
   Asp Leu Tyr Leu Ala His Phe Phe Gly Pro Gly Ala Ala Arg Arg Phe
   225 230 235 240
   Leu Thr Thr Gly Gln Asn Glu Leu Ala Ala Thr His Phe Pro Lys Glu
   245 250 255
   Ala Gln Ala Asn Pro Ser Ile Phe Tyr Asn Lys Asp Gly Ser Pro Lys
   260 265 270
   Thr Ile Gln Glu Val Tyr Asn Leu Met Asp Gly Lys Val Ala Ala His
   275 280 285
   Arg Lys
   290
<210> 133
   <211> 290
   <212> PRT
   <213> artificial sequence
<220>
   <223> mutated KZ144 with T82I, A206V, S232T, I122M, A160T, C14S and C50S plus SMAP-29 without Met
<400> 133
   Arg Gly Leu Arg Arg Leu Gly Arg Lys Ile Ala His Gly Val Lys Lys
   1 5 10 15
   Tyr Gly Pro Thr Val Leu Arg Ile Ile Arg Ile Ala Gly Gly Ser Lys
   20 25 30
   Val Leu Arg Lys Gly Asp Arg Gly Asp Glu Val Ser Gln Leu Gln Thr
   35 40 45
   Leu Leu Asn Leu Cys Gly Tyr Asp Val Gly Lys Pro Asp Gly Ile Phe
   50 55 60
   Gly Asn Asn Thr Phe Asn Gln Val Val Lys Phe Gln Lys Asp Asn Ser
   65 70 75 80
   Leu Asp Ser Asp Gly Ile Val Gly Lys Asn Thr Trp Ala Glu Leu Phe
   85 90 95
   Ser Lys Tyr Ser Pro Pro Ile Pro Tyr Lys Thr Ile Pro Met Pro Ile
   100 105 110
   Ala Asn Lys Ser Arg Ala Ala Ala Thr Pro Val Met Asn Ala Val Glu
   115 120 125
   Asn Ala Thr Gly Val Arg Ser Gln Leu Leu Leu Thr Phe Ala Ser Ile
   130 135 140
   Glu Ser Ala Phe Asp Tyr Glu Met Lys Ala Lys Thr Ser Ser Ala Thr
   145 150 155 160
   Gly Trp Phe Gln Phe Leu Thr Gly Thr Trp Lys Thr Met Ile Glu Asn
   165 170 175
   Tyr Gly Met Lys Tyr Gly Val Leu Thr Asp Pro Thr Gly Thr Leu Arg
   180 185 190
   Lys Asp Pro Arg Ile Ser Ala Leu Met Gly Ala Glu Leu Ile Lys Glu
   195 200 205
   Asn Met Asn Ile Leu Arg Pro Val Leu Lys Arg Glu Pro Thr Asp Thr
   210 215 220
   Asp Leu Tyr Leu Ala His Phe Phe Gly Pro Gly Val Ala Arg Arg Phe
   225 230 235 240
   Leu Thr Thr Gly Gln Asn Glu Leu Ala Ala Thr His Phe Pro Lys Glu
   245 250 255
   Ala Gln Ala Asn Pro Thr Ile Phe Tyr Asn Lys Asp Gly Ser Pro Lys
   260 265 270
   Thr Ile Gln Glu Val Tyr Asn Leu Met Asp Gly Lys Val Ala Ala His
   275 280 285
   Arg Lys
   290
<210> 134
   <211> 290
   <212> PRT
   <213> artificial sequence
<220>
   <223> mutated KZ144 with T82I, A206N, S232T, I122M, A160T, C14S and C50S plus SMAP-29 without Met
<400> 134
   Arg Gly Leu Arg Arg Leu Gly Arg Lys Ile Ala His Gly Val Lys Lys
   1               5                   10                  15
   Tyr Gly Pro Thr Val Leu Arg Ile Ile Arg Ile Ala Gly Gly Ser Lys
               20                  25                  30
   Val Leu Arg Lys Gly Asp Arg Gly Asp Glu Val Ser Gln Leu Gln Thr
           35                  40                  45
   Leu Leu Asn Leu Cys Gly Tyr Asp Val Gly Lys Pro Asp Gly Ile Phe
       50                  55                  60
   Gly Asn Asn Thr Phe Asn Gln Val Val Lys Phe Gln Lys Asp Asn Ser
   65                  70                  75                  80
   Leu Asp Ser Asp Gly Ile Val Gly Lys Asn Thr Trp Ala Glu Leu Phe
                   85                  90                  95
   Ser Lys Tyr Ser Pro Pro Ile Pro Tyr Lys Thr Ile Pro Met Pro Ile
               100                 105                 110
   Ala Asn Lys Ser Arg Ala Ala Ala Thr Pro Val Met Asn Ala Val Glu
           115                 120                 125
   Asn Ala Thr Gly Val Arg Ser Gln Leu Leu Leu Thr Phe Ala Ser Ile
       130                 135                 140
   Glu Ser Ala Phe Asp Tyr Glu Met Lys Ala Lys Thr Ser Ser Ala Thr
   145                 150                 155                 160
   Gly Trp Phe Gln Phe Leu Thr Gly Thr Trp Lys Thr Met Ile Glu Asn
                   165                 170                 175
   Tyr Gly Met Lys Tyr Gly Val Leu Thr Asp Pro Thr Gly Thr Leu Arg
               180                 185                 190
   Lys Asp Pro Arg Ile Ser Ala Leu Met Gly Ala Glu Leu Ile Lys Glu
           195                 200                 205
   Asn Met Asn Ile Leu Arg Pro Val Leu Lys Arg Glu Pro Thr Asp Thr
       210                 215                 220
   Asp Leu Tyr Leu Ala His Phe Phe Gly Pro Gly Asn Ala Arg Arg Phe
   225                 230                 235                 240
   Leu Thr Thr Gly Gln Asn Glu Leu Ala Ala Thr His Phe Pro Lys Glu
   245 250 255
   Ala Gln Ala Asn Pro Thr Ile Phe Tyr Asn Lys Asp Gly Ser Pro Lys
   260 265 270
   Thr Ile Gln Glu Val Tyr Asn Leu Met Asp Gly Lys Val Ala Ala His
   275 280 285
   Arg Lys
   290
<210> 135
   <211> 6
   <212> PRT
   <213> artificial sequence
<220>
   <223> His-Tag (6x)
<400> 135
   His His His His His His
   1 5
<210> 136
   <211> 299
   <212> PRT
   <213> artificial sequence
<220>
   <223> mutated KZ144 with C14S and C50S plus SMAP-29 and HisTag
<400> 136
   Met Arg Gly Leu Arg Arg Leu Gly Arg Lys Ile Ala His Gly Val Lys
   1 5 10 15
   Lys Tyr Gly Pro Thr Val Leu Arg Ile Ile Arg Ile Ala Gly Gly Ser
   20 25 30
   Lys Val Leu Arg Lys Gly Asp Arg Gly Asp Glu Val Ser Gln Leu Gln
   35 40 45
   Thr Leu Leu Asn Leu Cys Gly Tyr Asp Val Gly Lys Pro Asp Gly Ile
   50 55 60
   Phe Gly Asn Asn Thr Phe Asn Gln Val Val Lys Phe Gln Lys Asp Asn
   65 70 75 80
   Ser Leu Asp Ser Asp Gly Ile Val Gly Lys Asn Thr Trp Ala Glu Leu
                   85                  90                  95
   Phe Ser Lys Tyr Ser Pro Pro Ile Pro Tyr Lys Thr Ile Pro Met Pro
               100                 105                 110
   Thr Ala Asn Lys Ser Arg Ala Ala Ala Thr Pro Val Met Asn Ala Val
           115                 120                 125
   Glu Asn Ala Thr Gly Val Arg Ser Gln Leu Leu Leu Thr Phe Ala Ser
       130                 135                 140
   Ile Glu Ser Ala Phe Asp Tyr Glu Ile Lys Ala Lys Thr Ser Ser Ala
   145                 150                 155                 160
   Thr Gly Trp Phe Gln Phe Leu Thr Gly Thr Trp Lys Thr Met Ile Glu
                   165                 170                 175
   Asn Tyr Gly Met Lys Tyr Gly Val Leu Thr Asp Pro Thr Gly Ala Leu
               180                 185                 190
   Arg Lys Asp Pro Arg Ile Ser Ala Leu Met Gly Ala Glu Leu Ile Lys
           195                 200                 205
   Glu Asn Met Asn Ile Leu Arg Pro Val Leu Lys Arg Glu Pro Thr Asp
       210                 215                 220
   Thr Asp Leu Tyr Leu Ala His Phe Phe Gly Pro Gly Ala Ala Arg Arg
   225                 230                 235                 240
   Phe Leu Thr Thr Gly Gln Asn Glu Leu Ala Ala Thr His Phe Pro Lys
                   245                 250                 255
   Glu Ala Gln Ala Asn Pro Ser Ile Phe Tyr Asn Lys Asp Gly Ser Pro
               260                 265                 270
   Lys Thr Ile Gln Glu Val Tyr Asn Leu Met Asp Gly Lys Val Ala Ala
           275                 280                 285
   His Arg Lys Leu Glu His His His His His His
       290                 295
<210> 137
   <211> 299
   <212> PRT
   <213> artificial sequence
<220>
   <223> mutated KZ144 with T82I, A206V and S232 plus SMAP-29 and HisTag
<400> 137
   Met Arg Gly Leu Arg Arg Leu Gly Arg Lys Ile Ala His Gly Val Lys
   1               5                   10                  15
   Lys Tyr Gly Pro Thr Val Leu Arg Ile Ile Arg Ile Ala Gly Gly Ser
               20                  25                  30
   Lys Val Leu Arg Lys Gly Asp Arg Gly Asp Glu Val Cys Gln Leu Gln
           35                  40                  45
   Thr Leu Leu Asn Leu Cys Gly Tyr Asp Val Gly Lys Pro Asp Gly Ile
       50                  55                  60
   Phe Gly Asn Asn Thr Phe Asn Gln Val Val Lys Phe Gln Lys Asp Asn
   65                  70                  75                  80
   Cys Leu Asp Ser Asp Gly Ile Val Gly Lys Asn Thr Trp Ala Glu Leu
                   85                  90                  95
   Phe Ser Lys Tyr Ser Pro Pro Ile Pro Tyr Lys Thr Ile Pro Met Pro
               100                 105                 110
   Ile Ala Asn Lys Ser Arg Ala Ala Ala Thr Pro Val Met Asn Ala Val
           115                 120                 125
   Glu Asn Ala Thr Gly Val Arg Ser Gln Leu Leu Leu Thr Phe Ala Ser
       130                 135                 140
   Ile Glu Ser Ala Phe Asp Tyr Glu Ile Lys Ala Lys Thr Ser Ser Ala
   145                 150                 155                 160
   Thr Gly Trp Phe Gln Phe Leu Thr Gly Thr Trp Lys Thr Met Ile Glu
                   165                 170                 175
   Asn Tyr Gly Met Lys Tyr Gly Val Leu Thr Asp Pro Thr Gly Ala Leu
               180                 185                 190
   Arg Lys Asp Pro Arg Ile Ser Ala Leu Met Gly Ala Glu Leu Ile Lys
           195                 200                 205
   Glu Asn Met Asn Ile Leu Arg Pro Val Leu Lys Arg Glu Pro Thr Asp
       210                 215                 220
   Thr Asp Leu Tyr Leu Ala His Phe Phe Gly Pro Gly Val Ala Arg Arg
   225                 230                 235                 240
   Phe Leu Thr Thr Gly Gln Asn Glu Leu Ala Ala Thr His Phe Pro Lys
                   245                 250                 255
   Glu Ala Gln Ala Asn Pro Thr Ile Phe Tyr Asn Lys Asp Gly Ser Pro
               260                 265                 270
   Lys Thr Ile Gln Glu Val Tyr Asn Leu Met Asp Gly Lys Val Ala Ala
           275                 280                 285
   His Arg Lys Leu Glu His His His His His His
       290                 295
<210> 138
   <211> 299
   <212> PRT
   <213> artificial sequence
<220>
   <223> mutated KZ144 with T82I, A206V, S232T, I122M and A160Tplus SMAP-29 and HisTag
<400> 138
   Met Arg Gly Leu Arg Arg Leu Gly Arg Lys Ile Ala His Gly Val Lys
   1               5                   10                  15
   Lys Tyr Gly Pro Thr Val Leu Arg Ile Ile Arg Ile Ala Gly Gly Ser
               20                  25                  30
   Lys Val Leu Arg Lys Gly Asp Arg Gly Asp Glu Val Cys Gln Leu Gln
           35                  40                  45
   Thr Leu Leu Asn Leu Cys Gly Tyr Asp Val Gly Lys Pro Asp Gly Ile
       50                  55                  60
   Phe Gly Asn Asn Thr Phe Asn Gln Val Val Lys Phe Gln Lys Asp Asn
   65                  70                  75                  80
   Cys Leu Asp Ser Asp Gly Ile Val Gly Lys Asn Thr Trp Ala Glu Leu
                   85                  90                  95
   Phe Ser Lys Tyr Ser Pro Pro Ile Pro Tyr Lys Thr Ile Pro Met Pro
               100                 105                 110
   Ile Ala Asn Lys Ser Arg Ala Ala Ala Thr Pro Val Met Asn Ala Val
           115                 120                 125
   Glu Asn Ala Thr Gly Val Arg Ser Gln Leu Leu Leu Thr Phe Ala Ser
       130                 135                 140
   Ile Glu Ser Ala Phe Asp Tyr Glu Met Lys Ala Lys Thr Ser Ser Ala
   145 150 155 160
   Thr Gly Trp Phe Gln Phe Leu Thr Gly Thr Trp Lys Thr Met Ile Glu
   165 170 175
   Asn Tyr Gly Met Lys Tyr Gly Val Leu Thr Asp Pro Thr Gly Thr Leu
   180 185 190
   Arg Lys Asp Pro Arg Ile Ser Ala Leu Met Gly Ala Glu Leu Ile Lys
   195 200 205
   Glu Asn Met Asn Ile Leu Arg Pro Val Leu Lys Arg Glu Pro Thr Asp
   210 215 220
   Thr Asp Leu Tyr Leu Ala His Phe Phe Gly Pro Gly Val Ala Arg Arg
   225 230 235 240
   Phe Leu Thr Thr Gly Gln Asn Glu Leu Ala Ala Thr His Phe Pro Lys
   245 250 255
   Glu Ala Gln Ala Asn Pro Thr Ile Phe Tyr Asn Lys Asp Gly Ser Pro
   260 265 270
   Lys Thr Ile Gln Glu Val Tyr Asn Leu Met Asp Gly Lys Val Ala Ala
   275 280 285
   His Arg Lys Leu Glu His His His His His His
   290 295
<210> 139
   <211> 299
   <212> PRT
   <213> artificial sequence
<220>
   <223> mutated KZ144 with C14S, C50S, I122M and A160Tplus SMAP-29 and HisTag
<400> 139
   Met Arg Gly Leu Arg Arg Leu Gly Arg Lys Ile Ala His Gly Val Lys
   1               5                   10                  15
   Lys Tyr Gly Pro Thr Val Leu Arg Ile Ile Arg Ile Ala Gly Gly Ser
               20                  25                  30
   Lys Val Leu Arg Lys Gly Asp Arg Gly Asp Glu Val Ser Gln Leu Gln
           35                  40                  45
   Thr Leu Leu Asn Leu Cys Gly Tyr Asp Val Gly Lys Pro Asp Gly Ile
   50 55 60
   Phe Gly Asn Asn Thr Phe Asn Gln Val Val Lys Phe Gln Lys Asp Asn
   65 70 75 80
   Ser Leu Asp Ser Asp Gly Ile Val Gly Lys Asn Thr Trp Ala Glu Leu
   85 90 95
   Phe Ser Lys Tyr Ser Pro Pro Ile Pro Tyr Lys Thr Ile Pro Met Pro
   100 105 110
   Thr Ala Asn Lys Ser Arg Ala Ala Ala Thr Pro Val Met Asn Ala Val
   115 120 125
   Glu Asn Ala Thr Gly Val Arg Ser Gln Leu Leu Leu Thr Phe Ala Ser
   130 135 140
   Ile Glu Ser Ala Phe Asp Tyr Glu Met Lys Ala Lys Thr Ser Ser Ala
   145 150 155 160
   Thr Gly Trp Phe Gln Phe Leu Thr Gly Thr Trp Lys Thr Met Ile Glu
   165 170 175
   Asn Tyr Gly Met Lys Tyr Gly Val Leu Thr Asp Pro Thr Gly Thr Leu
   180 185 190
   Arg Lys Asp Pro Arg Ile Ser Ala Leu Met Gly Ala Glu Leu Ile Lys
   195 200 205
   Glu Asn Met Asn Ile Leu Arg Pro Val Leu Lys Arg Glu Pro Thr Asp
   210 215 220
   Thr Asp Leu Tyr Leu Ala His Phe Phe Gly Pro Gly Ala Ala Arg Arg
   225 230 235 240
   Phe Leu Thr Thr Gly Gln Asn Glu Leu Ala Ala Thr His Phe Pro Lys
   245 250 255
   Glu Ala Gln Ala Asn Pro Ser Ile Phe Tyr Asn Lys Asp Gly Ser Pro
   260 265 270
   Lys Thr Ile Gln Glu Val Tyr Asn Leu Met Asp Gly Lys Val Ala Ala
   275 280 285
   His Arg Lys Leu Glu His His His His His His
   290 295
<210> 140
   <211> 299
   <212> PRT
   <213> artificial sequence
<220>
   <223> mutated KZ144 with C14S, C23S and C50Splus SMAP-29 and HisTag
<400> 140
   Met Arg Gly Leu Arg Arg Leu Gly Arg Lys Ile Ala His Gly Val Lys
   1               5                   10                  15
   Lys Tyr Gly Pro Thr Val Leu Arg Ile Ile Arg Ile Ala Gly Gly Ser
               20                  25                  30
   Lys Val Leu Arg Lys Gly Asp Arg Gly Asp Glu Val Ser Gln Leu Gln
           35                  40                  45
   Thr Leu Leu Asn Leu Ser Gly Tyr Asp Val Gly Lys Pro Asp Gly Ile
       50                  55                  60
   Phe Gly Asn Asn Thr Phe Asn Gln Val Val Lys Phe Gln Lys Asp Asn
   65                  70                  75                  80
   Ser Leu Asp Ser Asp Gly Ile Val Gly Lys Asn Thr Trp Ala Glu Leu
                   85                  90                  95
   Phe Ser Lys Tyr Ser Pro Pro Ile Pro Tyr Lys Thr Ile Pro Met Pro
               100                 105                 110
   Thr Ala Asn Lys Ser Arg Ala Ala Ala Thr Pro Val Met Asn Ala Val
           115                 120                 125
   Glu Asn Ala Thr Gly Val Arg Ser Gln Leu Leu Leu Thr Phe Ala Ser
       130                 135                 140
   Ile Glu Ser Ala Phe Asp Tyr Glu Ile Lys Ala Lys Thr Ser Ser Ala
   145                 150                 155                 160
   Thr Gly Trp Phe Gln Phe Leu Thr Gly Thr Trp Lys Thr Met Ile Glu
                   165                 170                 175
   Asn Tyr Gly Met Lys Tyr Gly Val Leu Thr Asp Pro Thr Gly Ala Leu
               180                 185                 190
   Arg Lys Asp Pro Arg Ile Ser Ala Leu Met Gly Ala Glu Leu Ile Lys
           195                 200                 205
   Glu Asn Met Asn Ile Leu Arg Pro Val Leu Lys Arg Glu Pro Thr Asp
   210 215 220
   Thr Asp Leu Tyr Leu Ala His Phe Phe Gly Pro Gly Ala Ala Arg Arg
   225 230 235 240
   Phe Leu Thr Thr Gly Gln Asn Glu Leu Ala Ala Thr His Phe Pro Lys
   245 250 255
   Glu Ala Gln Ala Asn Pro Ser Ile Phe Tyr Asn Lys Asp Gly Ser Pro
   260 265 270
   Lys Thr Ile Gln Glu Val Tyr Asn Leu Met Asp Gly Lys Val Ala Ala
   275 280 285
   His Arg Lys Leu Glu His His His His His His
   290 295
<210> 141
   <211> 299
   <212> PRT
   <213> artificial sequence
<220>
   <223> mutated KZ144 with T82I, A206V, S232T, I122M, A160T, C14S and C50Splus SMAP-29 and HisTag
<400> 141
   Met Arg Gly Leu Arg Arg Leu Gly Arg Lys Ile Ala His Gly Val Lys
   1               5                   10                  15
   Lys Tyr Gly Pro Thr Val Leu Arg Ile Ile Arg Ile Ala Gly Gly Ser
               20                  25                  30
   Lys Val Leu Arg Lys Gly Asp Arg Gly Asp Glu Val Ser Gln Leu Gln
           35                  40                  45
   Thr Leu Leu Asn Leu Cys Gly Tyr Asp Val Gly Lys Pro Asp Gly Ile
       50                  55                  60
   Phe Gly Asn Asn Thr Phe Asn Gln Val Val Lys Phe Gln Lys Asp Asn
   65                  70                  75                  80
   Ser Leu Asp Ser Asp Gly Ile Val Gly Lys Asn Thr Trp Ala Glu Leu
                   85                  90                  95
   Phe Ser Lys Tyr Ser Pro Pro Ile Pro Tyr Lys Thr Ile Pro Met Pro
               100                 105                 110
   Ile Ala Asn Lys Ser Arg Ala Ala Ala Thr Pro Val Met Asn Ala Val
                   115                 120                 125
   Glu Asn Ala Thr Gly Val Arg Ser Gln Leu Leu Leu Thr Phe Ala Ser
       130                 135                 140
   Ile Glu Ser Ala Phe Asp Tyr Glu Met Lys Ala Lys Thr Ser Ser Ala
   145                 150                 155                 160
   Thr Gly Trp Phe Gln Phe Leu Thr Gly Thr Trp Lys Thr Met Ile Glu
                   165                 170                 175
   Asn Tyr Gly Met Lys Tyr Gly Val Leu Thr Asp Pro Thr Gly Thr Leu
               180                 185                 190
   Arg Lys Asp Pro Arg Ile Ser Ala Leu Met Gly Ala Glu Leu Ile Lys
           195                 200                 205
   Glu Asn Met Asn Ile Leu Arg Pro Val Leu Lys Arg Glu Pro Thr Asp
       210                 215                 220
   Thr Asp Leu Tyr Leu Ala His Phe Phe Gly Pro Gly Val Ala Arg Arg
   225                 230                 235                 240
   Phe Leu Thr Thr Gly Gln Asn Glu Leu Ala Ala Thr His Phe Pro Lys
                   245                 250                 255
   Glu Ala Gln Ala Asn Pro Thr Ile Phe Tyr Asn Lys Asp Gly Ser Pro
               260                 265                 270
   Lys Thr Ile Gln Glu Val Tyr Asn Leu Met Asp Gly Lys Val Ala Ala
           275                 280                 285
   His Arg Lys Leu Glu His His His His His His
       290                 295
<210> 142
   <211> 299
   <212> PRT
   <213> artificial sequence
<220>
   <223> mutated KZ144 with T82I, A206N, S232T, I122M, A160T, C14S and C50Splus SMAP-29 and HisTag
<400> 142
   Met Arg Gly Leu Arg Arg Leu Gly Arg Lys Ile Ala His Gly Val Lys
   1               5                   10                  15
   Lys Tyr Gly Pro Thr Val Leu Arg Ile Ile Arg Ile Ala Gly Gly Ser
   20 25 30
   Lys Val Leu Arg Lys Gly Asp Arg Gly Asp Glu Val Ser Gln Leu Gln
   35 40 45
   Thr Leu Leu Asn Leu Cys Gly Tyr Asp Val Gly Lys Pro Asp Gly Ile
   50 55 60
   Phe Gly Asn Asn Thr Phe Asn Gln Val Val Lys Phe Gln Lys Asp Asn
   65 70 75 80
   Ser Leu Asp Ser Asp Gly Ile Val Gly Lys Asn Thr Trp Ala Glu Leu
   85 90 95
   Phe Ser Lys Tyr Ser Pro Pro Ile Pro Tyr Lys Thr Ile Pro Met Pro
   100 105 110
   Ile Ala Asn Lys Ser Arg Ala Ala Ala Thr Pro Val Met Asn Ala Val
   115 120 125
   Glu Asn Ala Thr Gly Val Arg Ser Gln Leu Leu Leu Thr Phe Ala Ser
   130 135 140
   Ile Glu Ser Ala Phe Asp Tyr Glu Met Lys Ala Lys Thr Ser Ser Ala
   145 150 155 160
   Thr Gly Trp Phe Gln Phe Leu Thr Gly Thr Trp Lys Thr Met Ile Glu
   165 170 175
   Asn Tyr Gly Met Lys Tyr Gly Val Leu Thr Asp Pro Thr Gly Thr Leu
   180 185 190
   Arg Lys Asp Pro Arg Ile Ser Ala Leu Met Gly Ala Glu Leu Ile Lys
   195 200 205
   Glu Asn Met Asn Ile Leu Arg Pro Val Leu Lys Arg Glu Pro Thr Asp
   210 215 220
   Thr Asp Leu Tyr Leu Ala His Phe Phe Gly Pro Gly Asn Ala Arg Arg
   225 230 235 240
   Phe Leu Thr Thr Gly Gln Asn Glu Leu Ala Ala Thr His Phe Pro Lys
   245 250 255
   Glu Ala Gln Ala Asn Pro Thr Ile Phe Tyr Asn Lys Asp Gly Ser Pro
   260 265 270
   Lys Thr Ile Gln Glu Val Tyr Asn Leu Met Asp Gly Lys Val Ala Ala
           275                 280                 285
   His Arg Lys Leu Glu His His His His His His
       290                 295
<210> 143
   <211> 298
   <212> PRT
   <213> artificial sequence
<220>
   <223> mutated KZ144 with C14S and C50S plus SMAP-29 and HisTag, w/o Met
<400> 143
   Arg Gly Leu Arg Arg Leu Gly Arg Lys Ile Ala His Gly Val Lys Lys
   1               5                   10                  15
   Tyr Gly Pro Thr Val Leu Arg Ile Ile Arg Ile Ala Gly Gly Ser Lys
               20                  25                  30
   Val Leu Arg Lys Gly Asp Arg Gly Asp Glu Val Ser Gln Leu Gln Thr
           35                  40                  45
   Leu Leu Asn Leu Cys Gly Tyr Asp Val Gly Lys Pro Asp Gly Ile Phe
       50                  55                  60
   Gly Asn Asn Thr Phe Asn Gln Val Val Lys Phe Gln Lys Asp Asn Ser
   65                  70                  75                  80
   Leu Asp Ser Asp Gly Ile Val Gly Lys Asn Thr Trp Ala Glu Leu Phe
                   85                  90                  95
   Ser Lys Tyr Ser Pro Pro Ile Pro Tyr Lys Thr Ile Pro Met Pro Thr
               100                 105                 110
   Ala Asn Lys Ser Arg Ala Ala Ala Thr Pro Val Met Asn Ala Val Glu
           115                 120                 125
   Asn Ala Thr Gly Val Arg Ser Gln Leu Leu Leu Thr Phe Ala Ser Ile
       130                 135                 140
   Glu Ser Ala Phe Asp Tyr Glu Ile Lys Ala Lys Thr Ser Ser Ala Thr
   145                 150                 155                 160
   Gly Trp Phe Gln Phe Leu Thr Gly Thr Trp Lys Thr Met Ile Glu Asn
                   165                 170                 175
   Tyr Gly Met Lys Tyr Gly Val Leu Thr Asp Pro Thr Gly Ala Leu Arg
                   180                 185                 190
   Lys Asp Pro Arg Ile Ser Ala Leu Met Gly Ala Glu Leu Ile Lys Glu
           195                 200                 205
   Asn Met Asn Ile Leu Arg Pro Val Leu Lys Arg Glu Pro Thr Asp Thr
       210                 215                 220
   Asp Leu Tyr Leu Ala His Phe Phe Gly Pro Gly Ala Ala Arg Arg Phe
   225                 230                 235                 240
   Leu Thr Thr Gly Gln Asn Glu Leu Ala Ala Thr His Phe Pro Lys Glu
                   245                 250                 255
   Ala Gln Ala Asn Pro Ser Ile Phe Tyr Asn Lys Asp Gly Ser Pro Lys
               260                 265                 270
   Thr Ile Gln Glu Val Tyr Asn Leu Met Asp Gly Lys Val Ala Ala His
           275                 280                 285
   Arg Lys Leu Glu His His His His His His
       290                 295
<210> 144
   <211> 298
   <212> PRT
   <213> artificial sequence
<220>
   <223> mutated KZ144 with T82I, A206V and S232 plus SMAP-29 and HisTag, w/o Met
<400> 144
   Arg Gly Leu Arg Arg Leu Gly Arg Lys Ile Ala His Gly Val Lys Lys
   1               5                   10                  15
   Tyr Gly Pro Thr Val Leu Arg Ile Ile Arg Ile Ala Gly Gly Ser Lys
               20                  25                  30
   Val Leu Arg Lys Gly Asp Arg Gly Asp Glu Val Cys Gln Leu Gln Thr
           35                  40                  45
   Leu Leu Asn Leu Cys Gly Tyr Asp Val Gly Lys Pro Asp Gly Ile Phe
       50                  55                  60
   Gly Asn Asn Thr Phe Asn Gln Val Val Lys Phe Gln Lys Asp Asn Cys
   65                  70                  75                  80
   Leu Asp Ser Asp Gly Ile Val Gly Lys Asn Thr Trp Ala Glu Leu Phe
                       85                  90                  95
   Ser Lys Tyr Ser Pro Pro Ile Pro Tyr Lys Thr Ile Pro Met Pro Ile
               100                 105                 110
   Ala Asn Lys Ser Arg Ala Ala Ala Thr Pro Val Met Asn Ala Val Glu
           115                 120                 125
   Asn Ala Thr Gly Val Arg Ser Gln Leu Leu Leu Thr Phe Ala Ser Ile
       130                 135                 140
   Glu Ser Ala Phe Asp Tyr Glu Ile Lys Ala Lys Thr Ser Ser Ala Thr
   145                 150                 155                 160
   Gly Trp Phe Gln Phe Leu Thr Gly Thr Trp Lys Thr Met Ile Glu Asn
                   165                 170                 175
   Tyr Gly Met Lys Tyr Gly Val Leu Thr Asp Pro Thr Gly Ala Leu Arg
               180                 185                 190
   Lys Asp Pro Arg Ile Ser Ala Leu Met Gly Ala Glu Leu Ile Lys Glu
           195                 200                 205
   Asn Met Asn Ile Leu Arg Pro Val Leu Lys Arg Glu Pro Thr Asp Thr
       210                 215                 220
   Asp Leu Tyr Leu Ala His Phe Phe Gly Pro Gly Val Ala Arg Arg Phe
   225                 230                 235                 240
   Leu Thr Thr Gly Gln Asn Glu Leu Ala Ala Thr His Phe Pro Lys Glu
                   245                 250                 255
   Ala Gln Ala Asn Pro Thr Ile Phe Tyr Asn Lys Asp Gly Ser Pro Lys
               260                 265                 270
   Thr Ile Gln Glu Val Tyr Asn Leu Met Asp Gly Lys Val Ala Ala His
           275                 280                 285
   Arg Lys Leu Glu His His His His His His
       290                 295
<210> 145
   <211> 298
   <212> PRT
   <213> artificial sequence
<220>
   <223> mutated KZ144 with T82I, A206V, S232T, I122M and A160Tplus SMAP-29 and HisTag, w/o Met
<400> 145
   Arg Gly Leu Arg Arg Leu Gly Arg Lys Ile Ala His Gly Val Lys Lys
   1 5 10 15
   Tyr Gly Pro Thr Val Leu Arg Ile Ile Arg Ile Ala Gly Gly Ser Lys
   20 25 30
   Val Leu Arg Lys Gly Asp Arg Gly Asp Glu Val Cys Gln Leu Gln Thr
   35 40 45
   Leu Leu Asn Leu Cys Gly Tyr Asp Val Gly Lys Pro Asp Gly Ile Phe
   50 55 60
   Gly Asn Asn Thr Phe Asn Gln Val Val Lys Phe Gln Lys Asp Asn Cys
   65 70 75 80
   Leu Asp Ser Asp Gly Ile Val Gly Lys Asn Thr Trp Ala Glu Leu Phe
   85 90 95
   Ser Lys Tyr Ser Pro Pro Ile Pro Tyr Lys Thr Ile Pro Met Pro Ile
   100 105 110
   Ala Asn Lys Ser Arg Ala Ala Ala Thr Pro Val Met Asn Ala Val Glu
   115 120 125
   Asn Ala Thr Gly Val Arg Ser Gln Leu Leu Leu Thr Phe Ala Ser Ile
   130 135 140
   Glu Ser Ala Phe Asp Tyr Glu Met Lys Ala Lys Thr Ser Ser Ala Thr
   145 150 155 160
   Gly Trp Phe Gln Phe Leu Thr Gly Thr Trp Lys Thr Met Ile Glu Asn
   165 170 175
   Tyr Gly Met Lys Tyr Gly Val Leu Thr Asp Pro Thr Gly Thr Leu Arg
   180 185 190
   Lys Asp Pro Arg Ile Ser Ala Leu Met Gly Ala Glu Leu Ile Lys Glu
   195 200 205
   Asn Met Asn Ile Leu Arg Pro Val Leu Lys Arg Glu Pro Thr Asp Thr
   210 215 220
   Asp Leu Tyr Leu Ala His Phe Phe Gly Pro Gly Val Ala Arg Arg Phe
   225 230 235 240
   Leu Thr Thr Gly Gln Asn Glu Leu Ala Ala Thr His Phe Pro Lys Glu
   245 250 255
   Ala Gln Ala Asn Pro Thr Ile Phe Tyr Asn Lys Asp Gly Ser Pro Lys
   260 265 270
   Thr Ile Gln Glu Val Tyr Asn Leu Met Asp Gly Lys Val Ala Ala His
   275 280 285
   Arg Lys Leu Glu His His His His His His
   290 295
<210> 146
   <211> 298
   <212> PRT
   <213> artificial sequence
<220>
   <223> mutated KZ144 with C14S, C50S, I122M and A160Tplus SMAP-29 and HisTag, w/o Met
<400> 146
   Arg Gly Leu Arg Arg Leu Gly Arg Lys Ile Ala His Gly Val Lys Lys
   1 5 10 15
   Tyr Gly Pro Thr Val Leu Arg Ile Ile Arg Ile Ala Gly Gly Ser Lys
   20 25 30
   Val Leu Arg Lys Gly Asp Arg Gly Asp Glu Val Ser Gln Leu Gln Thr
   35 40 45
   Leu Leu Asn Leu Cys Gly Tyr Asp Val Gly Lys Pro Asp Gly Ile Phe
   50 55 60
   Gly Asn Asn Thr Phe Asn Gln Val Val Lys Phe Gln Lys Asp Asn Ser
   65 70 75 80
   Leu Asp Ser Asp Gly Ile Val Gly Lys Asn Thr Trp Ala Glu Leu Phe
   85 90 95
   Ser Lys Tyr Ser Pro Pro Ile Pro Tyr Lys Thr Ile Pro Met Pro Thr
   100 105 110
   Ala Asn Lys Ser Arg Ala Ala Ala Thr Pro Val Met Asn Ala Val Glu
   115 120 125
   Asn Ala Thr Gly Val Arg Ser Gln Leu Leu Leu Thr Phe Ala Ser Ile
   130 135 140
   Glu Ser Ala Phe Asp Tyr Glu Met Lys Ala Lys Thr Ser Ser Ala Thr
   145 150 155 160
   Gly Trp Phe Gln Phe Leu Thr Gly Thr Trp Lys Thr Met Ile Glu Asn
   165 170 175
   Tyr Gly Met Lys Tyr Gly Val Leu Thr Asp Pro Thr Gly Thr Leu Arg
   180 185 190
   Lys Asp Pro Arg Ile Ser Ala Leu Met Gly Ala Glu Leu Ile Lys Glu
   195 200 205
   Asn Met Asn Ile Leu Arg Pro Val Leu Lys Arg Glu Pro Thr Asp Thr
   210 215 220
   Asp Leu Tyr Leu Ala His Phe Phe Gly Pro Gly Ala Ala Arg Arg Phe
   225 230 235 240
   Leu Thr Thr Gly Gln Asn Glu Leu Ala Ala Thr His Phe Pro Lys Glu
   245 250 255
   Ala Gln Ala Asn Pro Ser Ile Phe Tyr Asn Lys Asp Gly Ser Pro Lys
   260 265 270
   Thr Ile Gln Glu Val Tyr Asn Leu Met Asp Gly Lys Val Ala Ala His
   275 280 285
   Arg Lys Leu Glu His His His His His His
   290 295
<210> 147
   <211> 298
   <212> PRT
   <213> artificial sequence
<220>
   <223> mutated KZ144 with C14S, C23S and C50Splus SMAP-29 and HisTag, w/o Met
<400> 147
   Arg Gly Leu Arg Arg Leu Gly Arg Lys Ile Ala His Gly Val Lys Lys
   1 5 10 15
   Tyr Gly Pro Thr Val Leu Arg Ile Ile Arg Ile Ala Gly Gly Ser Lys
   20 25 30
   Val Leu Arg Lys Gly Asp Arg Gly Asp Glu Val Ser Gln Leu Gln Thr
   35 40 45
   Leu Leu Asn Leu Ser Gly Tyr Asp Val Gly Lys Pro Asp Gly Ile Phe
       50                  55                  60
   Gly Asn Asn Thr Phe Asn Gln Val Val Lys Phe Gln Lys Asp Asn Ser
   65                  70                  75                  80
   Leu Asp Ser Asp Gly Ile Val Gly Lys Asn Thr Trp Ala Glu Leu Phe
                   85                  90                  95
   Ser Lys Tyr Ser Pro Pro Ile Pro Tyr Lys Thr Ile Pro Met Pro Thr
               100                 105                 110
   Ala Asn Lys Ser Arg Ala Ala Ala Thr Pro Val Met Asn Ala Val Glu
           115                 120                 125
   Asn Ala Thr Gly Val Arg Ser Gln Leu Leu Leu Thr Phe Ala Ser Ile
       130                 135                 140
   Glu Ser Ala Phe Asp Tyr Glu Ile Lys Ala Lys Thr Ser Ser Ala Thr
   145                 150                 155                 160
   Gly Trp Phe Gln Phe Leu Thr Gly Thr Trp Lys Thr Met Ile Glu Asn
                   165                 170                 175
   Tyr Gly Met Lys Tyr Gly Val Leu Thr Asp Pro Thr Gly Ala Leu Arg
               180                 185                 190
   Lys Asp Pro Arg Ile Ser Ala Leu Met Gly Ala Glu Leu Ile Lys Glu
           195                 200                 205
   Asn Met Asn Ile Leu Arg Pro Val Leu Lys Arg Glu Pro Thr Asp Thr
       210                 215                 220
   Asp Leu Tyr Leu Ala His Phe Phe Gly Pro Gly Ala Ala Arg Arg Phe
   225                 230                 235                 240
   Leu Thr Thr Gly Gln Asn Glu Leu Ala Ala Thr His Phe Pro Lys Glu
                   245                 250                 255
   Ala Gln Ala Asn Pro Ser Ile Phe Tyr Asn Lys Asp Gly Ser Pro Lys
               260                 265                 270
   Thr Ile Gln Glu Val Tyr Asn Leu Met Asp Gly Lys Val Ala Ala His
           275                 280                 285
   Arg Lys Leu Glu His His His His His His
       290                 295
<210> 148
   <211> 298
   <212> PRT
   <213> artificial sequence
<220>
   <223> mutated KZ144 with T82I, A206V, S232T, I122M, A160T, C14S and C50Splus SMAP-29 and HisTag, w/o Met
<400> 148
   Arg Gly Leu Arg Arg Leu Gly Arg Lys Ile Ala His Gly Val Lys Lys
   1               5                   10                  15
   Tyr Gly Pro Thr Val Leu Arg Ile Ile Arg Ile Ala Gly Gly Ser Lys
               20                  25                  30
   Val Leu Arg Lys Gly Asp Arg Gly Asp Glu Val Ser Gln Leu Gln Thr
           35                  40                  45
   Leu Leu Asn Leu Cys Gly Tyr Asp Val Gly Lys Pro Asp Gly Ile Phe
       50                  55                  60
   Gly Asn Asn Thr Phe Asn Gln Val Val Lys Phe Gln Lys Asp Asn Ser
   65                  70                  75                  80
   Leu Asp Ser Asp Gly Ile Val Gly Lys Asn Thr Trp Ala Glu Leu Phe
                   85                  90                  95
   Ser Lys Tyr Ser Pro Pro Ile Pro Tyr Lys Thr Ile Pro Met Pro Ile
               100                 105                 110
   Ala Asn Lys Ser Arg Ala Ala Ala Thr Pro Val Met Asn Ala Val Glu
           115                 120                 125
   Asn Ala Thr Gly Val Arg Ser Gln Leu Leu Leu Thr Phe Ala Ser Ile
       130                 135                 140
   Glu Ser Ala Phe Asp Tyr Glu Met Lys Ala Lys Thr Ser Ser Ala Thr
   145                 150                 155                 160
   Gly Trp Phe Gln Phe Leu Thr Gly Thr Trp Lys Thr Met Ile Glu Asn
                   165                 170                 175
   Tyr Gly Met Lys Tyr Gly Val Leu Thr Asp Pro Thr Gly Thr Leu Arg
               180                 185                 190
   Lys Asp Pro Arg Ile Ser Ala Leu Met Gly Ala Glu Leu Ile Lys Glu
           195                 200                 205
   Asn Met Asn Ile Leu Arg Pro Val Leu Lys Arg Glu Pro Thr Asp Thr
       210                 215                 220
   Asp Leu Tyr Leu Ala His Phe Phe Gly Pro Gly Val Ala Arg Arg Phe
   225                 230                 235                 240
   Leu Thr Thr Gly Gln Asn Glu Leu Ala Ala Thr His Phe Pro Lys Glu
                   245                 250                 255
   Ala Gln Ala Asn Pro Thr Ile Phe Tyr Asn Lys Asp Gly Ser Pro Lys
               260                 265                 270
   Thr Ile Gln Glu Val Tyr Asn Leu Met Asp Gly Lys Val Ala Ala His
           275                 280                 285
   Arg Lys Leu Glu His His His His His His
       290                 295
<210> 149
   <211> 298
   <212> PRT
   <213> artificial sequence
<220>
   <223> mutated KZ144 with T82I, A206N, S232T, I122M, A160T, C14S and C50S plus SMAP-29 and HisTag, w/o Met
<400> 149
   Arg Gly Leu Arg Arg Leu Gly Arg Lys Ile Ala His Gly Val Lys Lys
   1               5                   10                  15
   Tyr Gly Pro Thr Val Leu Arg Ile Ile Arg Ile Ala Gly Gly Ser Lys
               20                  25                  30
   Val Leu Arg Lys Gly Asp Arg Gly Asp Glu Val Ser Gln Leu Gln Thr
           35                  40                  45
   Leu Leu Asn Leu Cys Gly Tyr Asp Val Gly Lys Pro Asp Gly Ile Phe
       50                  55                  60
   Gly Asn Asn Thr Phe Asn Gln Val Val Lys Phe Gln Lys Asp Asn Ser
   65                  70                  75                  80
   Leu Asp Ser Asp Gly Ile Val Gly Lys Asn Thr Trp Ala Glu Leu Phe
                   85                  90                  95
   Ser Lys Tyr Ser Pro Pro Ile Pro Tyr Lys Thr Ile Pro Met Pro Ile
               100                 105                 110
   Ala Asn Lys Ser Arg Ala Ala Ala Thr Pro Val Met Asn Ala Val Glu
   115 120 125
   Asn Ala Thr Gly Val Arg Ser Gln Leu Leu Leu Thr Phe Ala Ser Ile
   130 135 140
   Glu Ser Ala Phe Asp Tyr Glu Met Lys Ala Lys Thr Ser Ser Ala Thr
   145 150 155 160
   Gly Trp Phe Gln Phe Leu Thr Gly Thr Trp Lys Thr Met Ile Glu Asn
   165 170 175
   Tyr Gly Met Lys Tyr Gly Val Leu Thr Asp Pro Thr Gly Thr Leu Arg
   180 185 190
   Lys Asp Pro Arg Ile Ser Ala Leu Met Gly Ala Glu Leu Ile Lys Glu
   195 200 205
   Asn Met Asn Ile Leu Arg Pro Val Leu Lys Arg Glu Pro Thr Asp Thr
   210 215 220
   Asp Leu Tyr Leu Ala His Phe Phe Gly Pro Gly Asn Ala Arg Arg Phe
   225 230 235 240
   Leu Thr Thr Gly Gln Asn Glu Leu Ala Ala Thr His Phe Pro Lys Glu
   245 250 255
   Ala Gln Ala Asn Pro Thr Ile Phe Tyr Asn Lys Asp Gly Ser Pro Lys
   260 265 270
   Thr Ile Gln Glu Val Tyr Asn Leu Met Asp Gly Lys Val Ala Ala His
   275 280 285
   Arg Lys Leu Glu His His His His His His
   290 295
<210> 150
   <211> 190
   <212> PRT
   <213> Artificial Sequence
<220>
   <223> KZ144 fragment 71-260 with Y75A
<400> 150
   Pro Pro Ile Pro Ala Lys Thr Ile Pro Met Pro Thr Ala Asn Lys Ser
   1 5 10 15
   Arg Ala Ala Ala Thr Pro Val Met Asn Ala Val Glu Asn Ala Thr Gly
   20 25 30
   Val Arg Ser Gln Leu Leu Leu Thr Phe Ala Ser Ile Glu Ser Ala Phe
   35 40 45
   Asp Tyr Glu Ile Lys Ala Lys Thr Ser Ser Ala Thr Gly Trp Phe Gln
   50 55 60
   Phe Leu Thr Gly Thr Trp Lys Thr Met Ile Glu Asn Tyr Gly Met Lys
   65 70 75 80
   Tyr Gly Val Leu Thr Asp Pro Thr Gly Ala Leu Arg Lys Asp Pro Arg
   85 90 95
   Ile Ser Ala Leu Met Gly Ala Glu Leu Ile Lys Glu Asn Met Asn Ile
   100 105 110
   Leu Arg Pro Val Leu Lys Arg Glu Pro Thr Asp Thr Asp Leu Tyr Leu
   115 120 125
   Ala His Phe Phe Gly Pro Gly Ala Ala Arg Arg Phe Leu Thr Thr Gly
   130 135 140
   Gln Asn Glu Leu Ala Ala Thr His Phe Pro Lys Glu Ala Gln Ala Asn
   145 150 155 160
   Pro Ser Ile Phe Tyr Asn Lys Asp Gly Ser Pro Lys Thr Ile Gln Glu
   165 170 175
   Val Tyr Asn Leu Met Asp Gly Lys Val Ala Ala His Arg Lys
   180 185 190
<210> 151
   <211> 299
   <212> PRT
   <213> Artificial Sequence
<220>
   <223> KZ144 plus SMAP-29 and HisTag
<400> 151
   Met Arg Gly Leu Arg Arg Leu Gly Arg Lys Ile Ala His Gly Val Lys
   1               5                   10                  15
   Lys Tyr Gly Pro Thr Val Leu Arg Ile Ile Arg Ile Ala Gly Gly Ser
               20                  25                  30
   Lys Val Leu Arg Lys Gly Asp Arg Gly Asp Glu Val Cys Gln Leu Gln
   35 40 45
   Thr Leu Leu Asn Leu Cys Gly Tyr Asp Val Gly Lys Pro Asp Gly Ile
   50 55 60
   Phe Gly Asn Asn Thr Phe Asn Gln Val Val Lys Phe Gln Lys Asp Asn
   65 70 75 80
   Cys Leu Asp Ser Asp Gly Ile Val Gly Lys Asn Thr Trp Ala Glu Leu
   85 90 95
   Phe Ser Lys Tyr Ser Pro Pro Ile Pro Tyr Lys Thr Ile Pro Met Pro
   100 105 110
   Thr Ala Asn Lys Ser Arg Ala Ala Ala Thr Pro Val Met Asn Ala Val
   115 120 125
   Glu Asn Ala Thr Gly Val Arg Ser Gln Leu Leu Leu Thr Phe Ala Ser
   130 135 140
   Ile Glu Ser Ala Phe Asp Tyr Glu Ile Lys Ala Lys Thr Ser Ser Ala
   145 150 155 160
   Thr Gly Trp Phe Gln Phe Leu Thr Gly Thr Trp Lys Thr Met Ile Glu
   165 170 175
   Asn Tyr Gly Met Lys Tyr Gly Val Leu Thr Asp Pro Thr Gly Ala Leu
   180 185 190
   Arg Lys Asp Pro Arg Ile Ser Ala Leu Met Gly Ala Glu Leu Ile Lys
   195 200 205
   Glu Asn Met Asn Ile Leu Arg Pro Val Leu Lys Arg Glu Pro Thr Asp
   210 215 220
   Thr Asp Leu Tyr Leu Ala His Phe Phe Gly Pro Gly Ala Ala Arg Arg
   225 230 235 240
   Phe Leu Thr Thr Gly Gln Asn Glu Leu Ala Ala Thr His Phe Pro Lys
   245 250 255
   Glu Ala Gln Ala Asn Pro Ser Ile Phe Tyr Asn Lys Asp Gly Ser Pro
   260 265 270
   Lys Thr Ile Gln Glu Val Tyr Asn Leu Met Asp Gly Lys Val Ala Ala
   275 280 285
   His Arg Lys Leu Glu His His His His His His
   290 295
<210> 152
   <211> 256
   <212> PRT
   <213> Artificial sequence
<220>
   <223> KZ144 without N-terminal methionine, with selenomethionine instead of methionine residues
<220>
   <221> misc_feature
   <222> (79)..(79)
   <223> Xaa is selenomethionine
<220>
   <221> misc_feature
   <222> (93)..(93)
   <223> Xaa is selenomethionine
<220>
   <221> misc_feature
   <222> (142)..(142)
   <223> Xaa is selenomethionine
<220>
   <221> misc_feature
   <222> (148)..(148)
   <223> Xaa is selenomethionine
<220>
   <221> misc_feature
   <222> (170)..(170)
   <223> Xaa is selenomethionine
<220>
   <221> misc_feature
   <222> (179)..(179)
   <223> Xaa is selenomethionine
<220>
   <221> misc_feature
   <222> (250)..(250)
   <223> Xaa is selenomethionine
<400> 152
   Lys Val Leu Arg Lys Gly Asp Arg Gly Asp Glu Val Cys Gln Leu Gln
   1 5 10 15
   Thr Leu Leu Asn Leu Cys Gly Tyr Asp Val Gly Lys Pro Asp Gly Ile
   20 25 30
   Phe Gly Asn Asn Thr Phe Asn Gln Val Val Lys Phe Gln Lys Asp Asn
   35 40 45
   Cys Leu Asp Ser Asp Gly Ile Val Gly Lys Asn Thr Trp Ala Glu Leu
       50                  55                  60
   Phe Ser Lys Tyr Ser Pro Pro Ile Pro Tyr Lys Thr Ile Pro Xaa Pro
   65                  70                  75                  80
   Thr Ala Asn Lys Ser Arg Ala Ala Ala Thr Pro Val Xaa Asn Ala Val
                   85                  90                  95
   Glu Asn Ala Thr Gly Val Arg Ser Gln Leu Leu Leu Thr Phe Ala Ser
               100                 105                 110
   Ile Glu Ser Ala Phe Asp Tyr Glu Ile Lys Ala Lys Thr Ser Ser Ala
           115                 120                 125
   Thr Gly Trp Phe Gln Phe Leu Thr Gly Thr Trp Lys Thr Xaa Ile Glu
       130                 135                 140
   Asn Tyr Gly Xaa Lys Tyr Gly Val Leu Thr Asp Pro Thr Gly Ala Leu
   145                 150                 155                 160
   Arg Lys Asp Pro Arg Ile Ser Ala Leu Xaa Gly Ala Glu Leu Ile Lys
                   165                 170                 175
   Glu Asn Xaa Asn Ile Leu Arg Pro Val Leu Lys Arg Glu Pro Thr Asp
               180                 185                 190
   Thr Asp Leu Tyr Leu Ala His Phe Phe Gly Pro Gly Ala Ala Arg Arg
           195                 200                 205
   Phe Leu Thr Thr Gly Gln Asn Glu Leu Ala Ala Thr His Phe Pro Lys
       210                 215                 220
   Glu Ala Gln Ala Asn Pro Ser Ile Phe Tyr Asn Lys Asp Gly Ser Pro
   225                 230                 235                 240
   Lys Thr Ile Gln Glu Val Tyr Asn Leu Xaa Asn Ile Leu Arg Pro Val
                   245                 250                 255

## Description

The present invention relates to polypeptides comprising an amino acid sequence exhibiting at least 90% sequence identity with the sequence of SEQ ID NO: 1. Said polypeptides preferably degrade the peptidoglycan of Gram-negative bacteria, in particular of *Pseudomonas* and/or *Campylobacter* bacteria. In addition, the present invention relates to nucleic acids encoding such polypeptides, vectors comprising such nucleic acids, and corresponding host cells. Finally, the present invention relates to compositions comprising such polypeptides, nucleic acids, vectors, and/or host cells according to the present invention.

The giant, lytic Myoviridae bacteriophage φKZ (280 334 bp) infects *Pseudomonas aeruginosa,* an important opportunistic nosocomial pathogen resistant to many commonly used antibiotics, and is therefore the cause of considerable concern in hospital environments. In 2007, Briers et al. (Molecular Microbiology (2007) 65(5), 1334-1344) sequenced the genome of said bacteriophage and identified the endolysin KZ144, a highly lytic peptidoglycan hydrolase. Lopez et al., Drug Discovery Today: Therapeutic Strategies, 2004, 1 (4): 469-474)) discussed the peculiarities of endolysins and their application in anti-infective therapy. In a further publication (Briers et al., Biochem Biophys Res Commun. 2009 May 29;383(2):187-191) the high-affinity peptidoglycan binding domain of endolysin KZ144 was studied. Said peptidoglycan binding domain was used to improve the specific activity of the peptidoglycan hydrolase domain KMV36C. Fokine et al. (J Biol Chem. 2008 Mar 14;283(11):7242-7250) published the structure of KZ144 endolysin. In WO 2010/149792, a fusion protein comprising the sequence of said KZ 144 endolysin as enzymatic element has been proposed for use in degrading the cell wall of Gram-negative bacteria.

While said endolysin and fusion proteins are effective in general, it turned out, that for some technical applications the endolysin polypeptide exhibits suboptimal characteristics, in particular in terms of stability and processing. Thus, there was a need in the art for a further endolysin enzyme, which exhibits preferably improved characteristics in this respect. The problem of the present invention was thus to provide such polypeptide.

The problem is solved by the subject-matter as set forth in the appended claims.

In the following a brief description of the appended figures will be given. The figures are intended to illustrate the present invention in more detail. However, they are not intended to limit the subject matter of the invention to any extent.

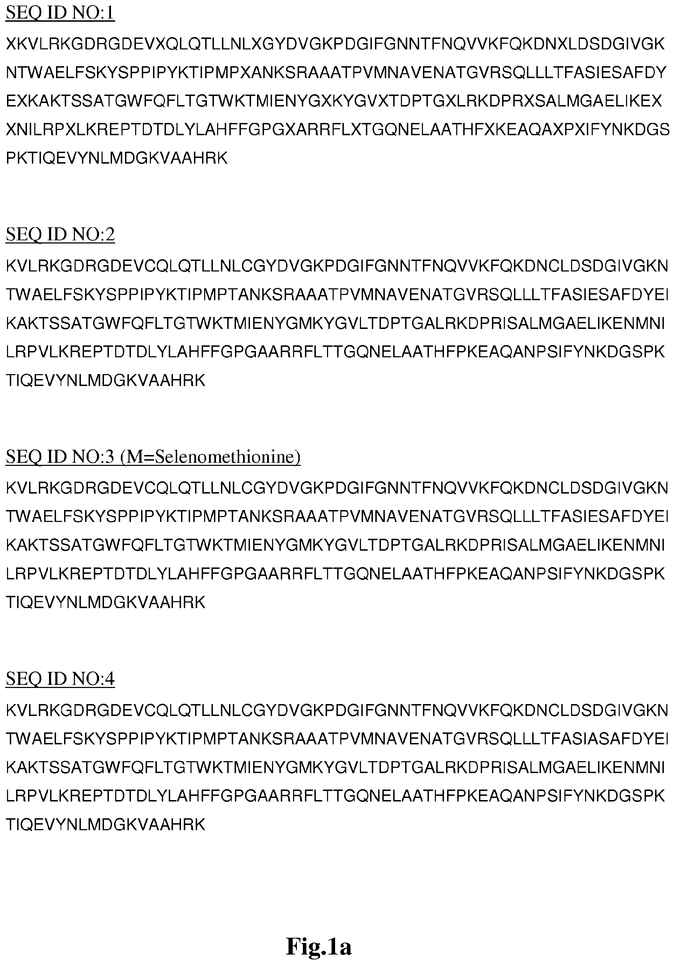

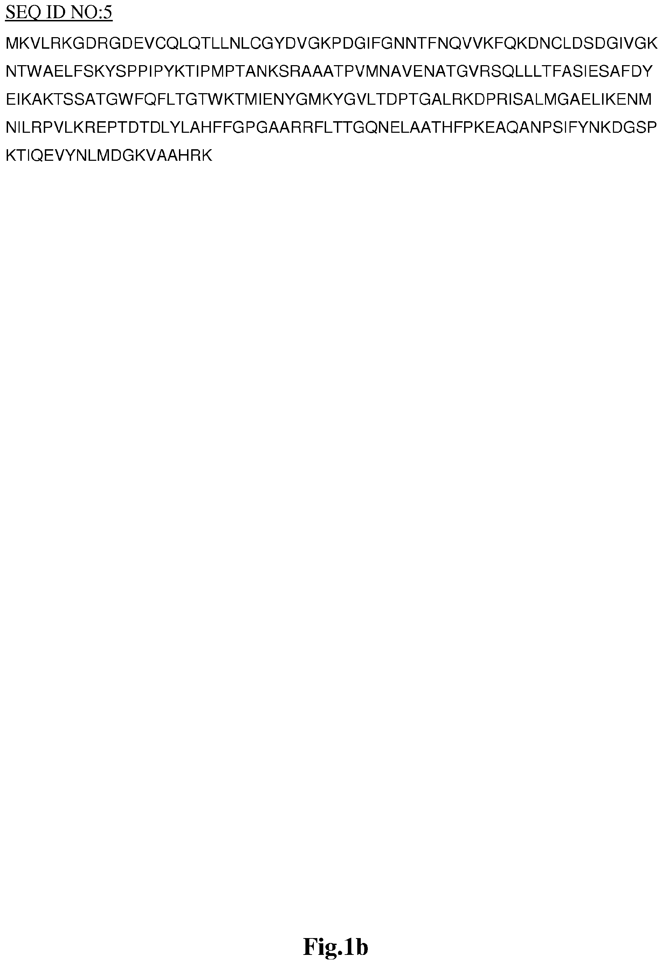

Fig. 1: illustrates:
   SEQ ID NO: 1, SEQ ID NO: 2 KZ144 endolysin without N-terminal methionine,
   SEQ ID NO: 3 KZ144 endolysin without N-terminal methionine and with selenomethionine residues instead of methionine residues,
   SEQ ID NO: 4 KZ144 endolysin with E115A mutation without N-terminal methionine, and
   SEQ ID NO: 5 KZ144 endolysin.

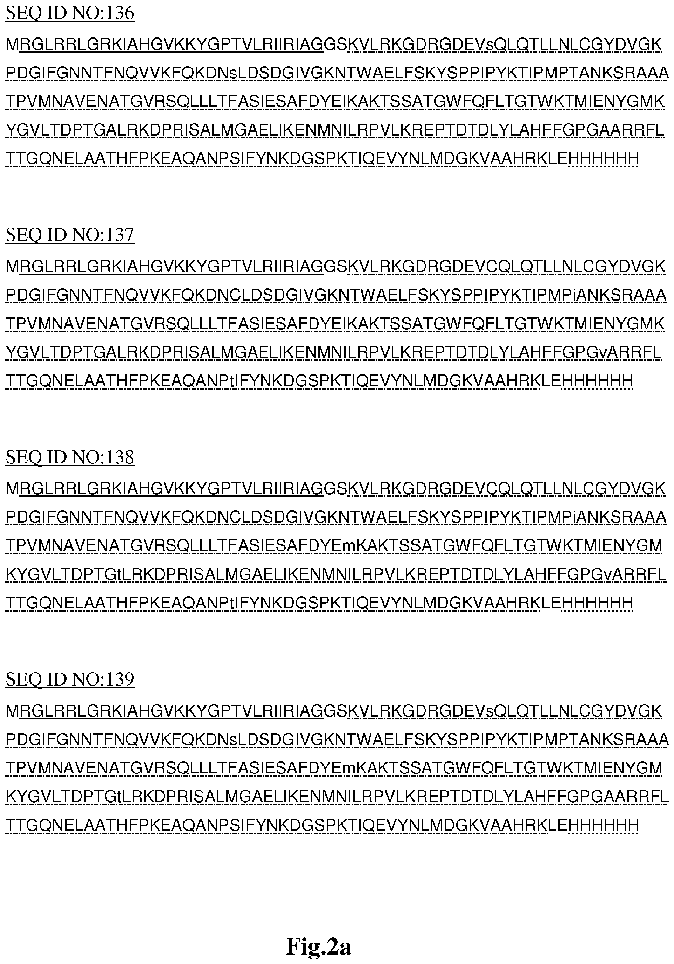

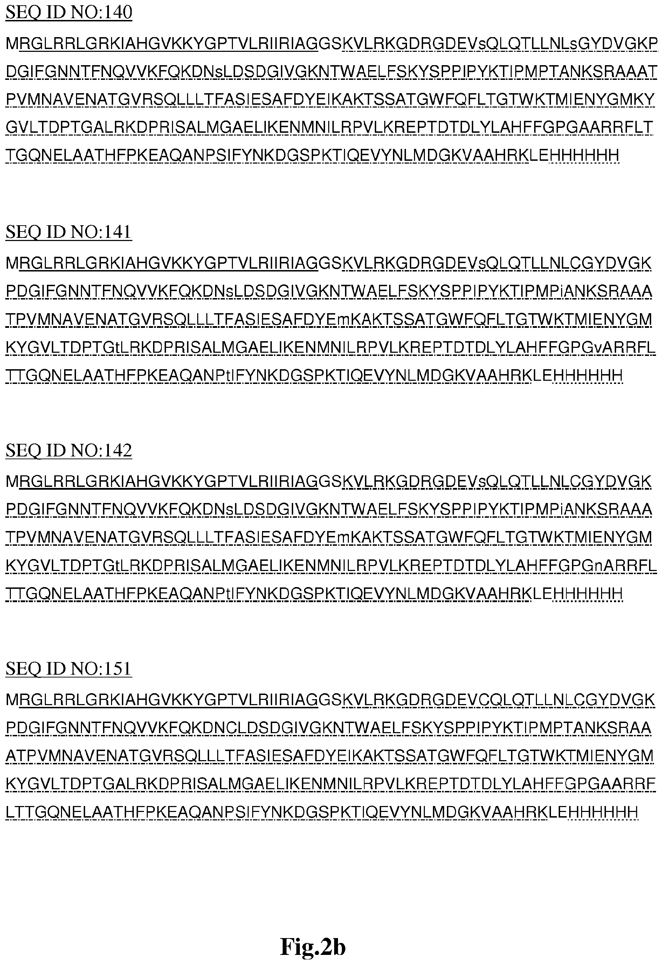

Fig. 2: illustrates:
   SEQ ID NO: 136 Fusion protein of SMAP-29 (underlined with solid line; SEQ ID NO: 76), modified KZ144 without N-terminal methionine and with C14S and C50S (underlined with semi-dotted/semisolid line; SEQ ID NO: 28) and His-tag (underlined with dotted line; SEQ ID NO: 135).
   SEQ ID NO: 137 Comparative example: Fusion protein of SMAP-29 (underlined with solid line; SEQ ID NO: 76), modified KZ144 without N-terminal methionine and with T82I, A206V and S232T (underlined with semi-dotted/semi-solid line; SEQ ID NO: 29) and His-tag (underlined with dotted line; SEQ ID NO: 135).
   SEQ ID NO: 138 Comparative example: Fusion protein of SMAP-29 (underlined with solid line; SEQ ID NO: 76), modified KZ144 without N-terminal methionine and with T82I, A206V, S232T, I122M; and A160T (underlined with semi-dotted/semi-solid line; SEQ ID NO: 30) and His-tag (underlined with dotted line; SEQ ID NO: 135).
   SEQ ID NO: 139 Fusion protein of SMAP-29 (underlined with solid line; SEQ ID NO: 76), modified KZ144 without N-terminal methionine and with C14S, C50S, I122M; and A160T (underlined with semi-dotted/semi-solid line; SEQ ID NO: 31) and His-tag (underlined with dotted line; SEQ ID NO: 135).
   SEQ ID NO: 140 Fusion protein of SMAP-29 (underlined with solid line; SEQ ID NO: 76), modified KZ144 without N-terminal methionine and with C14S, C23S and C50S (underlined with semi-dotted/semi-solid line; SEQ ID NO: 32) and His-tag (underlined with dotted line; SEQ ID NO: 135).
   SEQ ID NO: 141 Fusion protein of SMAP-29 (underlined with solid line; SEQ ID NO: 76), modified KZ144 without N-terminal methionine and with T82I, A206V, S232T, I122M; A160T, C14S and C50S (underlined with semi-dotted/semi-solid line; SEQ ID NO: 33) and His-tag (underlined with dotted line; SEQ ID NO: 135).
   SEQ ID NO: 142 Fusion protein of SMAP-29 (underlined with solid line; SEQ ID NO: 76), modified KZ144 without N-terminal methionine and with T82I, A206N, S232T, I122M; A160T C14S and C50S (underlined with semi-dotted/semi-solid line; SEQ ID NO: 49) and His-tag (underlined with dotted line; SEQ ID NO: 135).
   SEQ ID NO: 151 Comparative example: Fusion protein of SMAP-29 (underlined with solid line; SEQ ID NO: 76), KZ144 without N-terminal methionine (underlined with semi-dotted/semi-solid line; SEQ ID NO: 2) and His-tag (underlined with dotted line; SEQ ID NO: 135).

In a first aspect the present invention relates to a polypeptide comprising an amino acid sequence, said amino acid sequence exhibiting i) at least 90% sequence identity with the sequence of SEQ ID NO: 1, wherein SEQ ID NO: 1 is characterized by
X1 may be absent or any amino acid, in particular M,
X14 may be any amino acid, preferably S, R or N, more preferably S or R
X23 may be any amino acid, preferably S, R or N, more preferably S
X50 may be any amino acid, preferably S, R or N, more preferably S or N
X82 may be any amino acid, preferably T or I
X122 may be any amino acid, preferably I or M
X149 may be any amino acid, preferably M or P
X154 may be any amino acid, preferably L or T
X160 may be any amino acid, preferably A or T
X167 may be any amino acid, preferably I or L
X179 may be any amino acid, preferably N or F
X180 may be any amino acid, preferably M or E
X186 may be any amino acid, preferably V or Y
X206 may be any amino acid, preferably A, N or V
X212 may be any amino acid, preferably T or N
X224 may be any amino acid, preferably P or Q
X230 may be any amino acid, preferably N or Y
X232 may be any amino acid, preferably S or T;
and ii) at least one of the residues corresponding to position X14, X23 and X50 of SEQ ID NO: 1 is S; wherein the polypeptide does neither comprise the amino acid sequence of SEQ ID NO: 2, nor of SEQ ID NO: 3, nor of SEQ ID NO: 4, and wherein the polypeptide degrades the peptidoglycan of Pseudomonas and/or Campylobacter bacteria.

The term "polypeptide" as used herein refers in particular to a polymer of amino acid residues linked by peptide bonds in a specific sequence. The amino acid residues of a polypeptide may be modified by e.g. covalent attachments of various groups such as carbohydrates and phosphate. Other substances may be more loosely associated with the polypeptide, such as heme or lipid, giving rise to conjugated polypeptides which are also comprised by the term "polypeptide" as used herein. The term as used herein is intended to encompass also proteins. Thus, the term "polypeptide" also encompasses for example complexes of two or more amino acid polymer chains. The term "polypeptide " does encompass embodiments of polypeptides which exhibit optionally modifications typically used in the art, e.g. biotinylation, acetylation, pegylation, chemical changes of the amino-, SH- or carboxyl-groups (e.g. protecting groups) etc.. As will become apparent from the description below, the polypeptide according to the present invention may also be a fusion protein, i.e. linkage of at least two amino acid sequences which do not occur in this combination in nature. The term " polypeptide " as used herein is not limited to a specific length of the amino acid polymer chain, but typically the polypeptide will exhibit a length of more than about 50 amino acids, more than about 100 amino acids or even more than about 150 amino acids. Usually, but not necessarily, a typical polypeptide of the present invention will not exceed about 750 amino acids in length.

As used herein, the term "% sequence identity", has to be understood as follows: Two sequences to be compared are aligned to give a maximum correlation between the sequences. This may include inserting "gaps" in either one or both sequences, to enhance the degree of alignment. A % identity may then be determined over the whole length of each of the sequences being compared (so-called global alignment), that is particularly suitable for sequences of the same or similar length, or over shorter, defined lengths (so-called local alignment), that is more suitable for sequences of unequal length. In the above context, an amino acid sequence having a "sequence identity" of at least, for example, 95% to a query amino acid sequence, is intended to mean that the sequence of the subject amino acid sequence is identical to the query sequence except that the subject amino acid sequence may include up to five amino acid alterations per each 100 amino acids of the query amino acid sequence. In other words, to obtain an amino acid sequence having a sequence of at least 95% identity to a query amino acid sequence, up to 5% (5 of 100) of the amino acid residues in the subject sequence may be inserted or substituted with another amino acid or deleted. Methods for comparing the identity and homology of two or more sequences are well known in the art. The percentage to which two sequences are identical can for example be determined by using a mathematical algorithm. A preferred, but not limiting, example of a mathematical algorithm which can be used is the algorithm of Karlin et a/. (1993), PNAS USA, 90:5873-5877. Such an algorithm is integrated in the BLAST family of programs, e.g. BLAST or NBLAST program (see also Altschul et al., 1990, J. Mol. Biol. 215, 403-410 or Altschul et al. (1997), Nucleic Acids Res, 25:3389-3402), accessible through the home page of the NCBI at world wide web site ncbi.nlm.nih.gov) and FASTA (Pearson (1990), Methods Enzymol. 83, 63-98; Pearson and Lipman (1988), Proc. Natl. Acad. Sci. U. S. A 85, 2444-2448.). Sequences which are identical to other sequences to a certain extent can be identified by these programmes. Furthermore, programs available in the Wisconsin Sequence Analysis Package, version 9.1 (Devereux et al, 1984, Nucleic Acids Res., 387-395), for example the programs BESTFIT and GAP, may be used to determine the % identity between two polypeptide sequences. BESTFIT uses the "local homology" algorithm of (Smith and Waterman (1981), J. Mol. Biol. 147, 195-197.) and finds the best single region of similarity between two sequences. If herein reference is made to an amino acid sequence sharing a particular extent of sequence identity to a reference sequence, then said difference in sequence is preferably due to conservative amino acid substitutions. Preferably, such sequence retains the activity of the reference sequence, e.g. albeit maybe at a slower rate. In addition, if reference is made herein to a sequence sharing "at least" at certain percentage of sequence identity, then 100% sequence identity are preferably not encompassed.

"Conservative amino acid substitutions", as used herein, may occur within a group of amino acids which have sufficiently similar physicochemical properties, so that a substitution between members of the group will preserve the biological activity of the molecule (see e.g. Grantham, R. (1974), Science 185, 862-864). Particularly, conservative amino acid substitutions are preferably substitutions in which the amino acids originate from the same class of amino acids (e.g. basic amino acids, acidic amino acids, polar amino acids, amino acids with aliphatic side chains, amino acids with positively or negatively charged side chains, amino acids with aromatic groups in the side chains, amino acids the side chains of which can enter into hydrogen bridges, e.g. side chains which have a hydroxyl function, etc.). Conservative substitutions are in the present case for example substituting a basic amino acid residue (Lys, Arg, His) for another basic amino acid residue (Lys, Arg, His), substituting an aliphatic amino acid residue (Gly, Ala, Val, Leu, lie) for another aliphatic amino acid residue, substituting an aromatic amino acid residue (Phe, Tyr, Trp) for another aromatic amino acid residue, substituting threonine by serine or leucine by isoleucine. Further conservative amino acid exchanges will be known to the person skilled in the art.

The term "deletion" as used herein refers preferably to the absence of 1, 2, 3, 4, 5 (or even more than 5) continuous amino acid residues in the derivative sequence in comparison to the respective reference sequence, either intrasequentially or at the N- or C-terminus.

The term "insertion" as used herein refers preferably to the additional intrasequential presence of 1, 2, 3, 4, 5 (or even more than 5) continuous amino acid residues in the derivative sequence in comparison to the respective reference sequence.

The term "addition" as used herein refers preferably to the additional presence of 1, 2, 3, 4, 5 (or even more than 5) continuous amino acid residues at the N- and/or C-terminus of the derivative sequence in comparison to the respective reference sequence.

The term "substitution" as used herein refers to the presence of an amino acid residue at a certain position of the derivative sequence which is different from the amino acid residue which is present or absent at the corresponding position in the reference sequence. As mentioned above, preferably such substitutions are conservative substitutions.

The term "cell wall" as used herein refers to all components that form the outer cell enclosure of Gram-negative bacteria and thus guarantee their integrity. In particular, the term "cell wall" as used herein refers to peptidoglycan, the outer membrane of the Gram-negative bacteria with the lipopolysaccharide, the bacterial cell membrane, but also to additional layers deposited on the peptidoglycan as e.g. capsules, outer protein layers or slimes.

The term "amino acid sequence stretch" as used herein refers to a particular stretch of amino acid sequence in the amino acid sequence of the polypeptide of the invention. Said sequence refers to a sequence of a cationic peptide, a polycationic peptide, an amphiphatic peptide, a hydrophobic peptide, a sushi peptide and/or an antimicrobial peptide. The term does not refer to conventional tags like His-tags, such as His5-tags, His6-tags, His7-tags, His8-tags, His9-tags, His10-tags, His11-tags, His12-tags, His16-tags and His20-tags, Strep-tags, Avi-tags, Myc-tags, Gst-tags, JS-tags, cystein-tags, FLAG-tags or other tags known in the art, thioredoxin or maltose binding proteins (MBP). Preferably an amino acid sequence stretch as used herein as a length of about 6 to about 39 amino acid residues.

As used herein, the term "cationic peptide" refers preferably to a peptide having positively charged amino acid residues. Preferably a cationic peptide has a pKa-value of 9.0 or greater. Typically, at least four of the amino acid residues of the cationic peptide can be positively charged, for example, lysine or arginine. "Positively charged" refers to the side chains of the amino acid residues which have a net positive charge at about physiological conditions. The term "cationic peptide" as used herein refers also to polycationic peptides, but also includes cationic peptides which comprise for example less than 20%, preferably less than 10% positively charged amino acid residues.

The term "polycationic peptide" as used herein refers preferably to a peptide composed of mostly positively charged amino acid residues, in particular lysine and/or arginine residues. A peptide is composed of mostly positively charged amino acid residues if at least about 20, 30, 40, 50, 60, 70, 75, 80, 85, 90, 95 or about 100 % of the amino acid residues are positively charged amino acid residues, in particular lysine and/or arginine residues. The amino acid residues being not positively charged amino acid residues can be neutrally charged amino acid residues and/or negatively charged amino acid residues and/or hydrophobic amino acid residues. Preferably the amino acid residues being not positively charged amino acid residues are neutrally charged amino acid residues, in particular serine and/or glycine.

The term, "antimicrobial peptide" (AMP) as used herein refers preferably to any naturally occurring peptide that has microbicidal and/or microbistatic activity on for example bacteria, viruses, fungi, yeasts, mycoplasma and protozoa. Thus, the term "antimicrobial peptide" as used herein refers in particular to any peptide having anti-bacterial, anti-fungal, anti-mycotic, anti-parasitic, anti-protozoal, anti-viral, anti-infectious, anti-infective and/or germicidal, algicidal, amoebicidal, microbicidal, bactericidal, fungicidal, parasiticidal, protozoacidal, protozoicidal properties. Preferred are anti-bacterial peptides. The antimicrobial peptide may be a member of the RNase A super family, a defensin, cathelicidin, granulysin, histatin, psoriasin, dermicidine or hepcidin. The antimicrobial peptide may be naturally occurring in insects, fish, plants, arachnids, vertebrates or mammals. Preferably the antimicrobial peptide may be naturally occurring in insects, fish, plants, arachnids, vertebrates or mammals. Preferably the antimicrobial peptide may be naturally occurring in radish, silk moth, wolf spider, frog, preferably in Xenopus laevis, Rana frogs, more preferably in Rana catesbeiana, toad, preferably Asian toad Bufo bufo gargarizans, fly, preferably in Drosophila, more preferably in Drosophila melanogaster, in Aedes aegypti, in honey bee, bumblebee, preferably in Bombus pascuorum, flesh fly, preferably in Sarcophaga peregrine, scorpion, horseshoe crab, catfish, preferably in Parasilurus asotus, cow, pig, sheep, porcine, bovine, monkey and human. As used herein, an "antimicrobial peptide" (AMP) may in particular be a peptide which is not a cationic peptide, polycationic peptide, amphiphatic peptide, sushi peptide, defensins, and hydrophobic peptide, but nevertheless exhibits antimicrobial activity.

The term "sushi peptide" as used herein refers to complement control proteins (CCP) having short consensus repeats. The sushi module of sushi peptides functions as a protein-protein interaction domain in many different proteins. Peptides containing a Sushi domain have been shown to have antimicrobial activities. Preferably, sushi peptides are naturally occurring peptides.

The term "amphiphatic peptide" as used herein refers to synthetic peptides having both hydrophilic and hydrophobic functional groups. Preferably, the term "amphiphatic peptide" as used herein refers to a peptide having a defined arrangement of hydrophilic and hydrophobic groups e.g. amphiphatic peptides may be e.g. alpha helical, having predominantly non polar side chains along one side of the helix and polar residues along the rest of its surface.

The term "hydrophobic group" as used herein refers preferably to chemical groups such as amino acid side chains which are substantially water insoluble, but soluble in an oil phase, with the solubility in the oil phase being higher than that in water or in an aqueous phase. In water, amino acid residues having a hydrophobic side chain interact with one another to generate a non-aqueous environment. Examples of amino acid residues with hydrophobic side chains are valine, isoleucine, leucine, methionine, phenylalanine, tryptophan, cysteine, alanine, tyrosine, and proline residues

The term "hydrophobic peptide" as used herein refers to a hydrophobic peptide, which is preferably composed of mostly amino acid residues with hydrophobic groups. Such peptide is preferably composed of mostly hydrophobic amino acid residues, i.e. at least about 20, 30, 40, 50, 60, 70, 75, 80, 85, 90, 95 or at least about 100 % of the amino acid residues are hydrophobic amino acid residues. The amino acid residues being not hydrophobic are preferably neutral and preferably not hydrophilic.

As used herein, the term "tag" refers to an amino acid sequence, which is typically in the art fused to or included in another amino acid sequence for a) improving expression of the overall amino acid sequence or polypeptide, b) facilitating purification of the overall amino acid sequence or polypeptide, c) facilitating immobilisation of the overall amino acid sequence or polypeptide, and/or d) facilitating detection of the overall amino acid sequence or polypeptide. Examples for tags are His tags, such as His5-tags, His6-tags, His7-tags, His8-tags, His9-tags, His10-tags, His11-tags, His12-tags, His16-tags and His20-tags, Strep-tags, Avi-tags, Myc-tags, GST-tags, JS-tags, cystein-tags, FLAG-tags, HA-tags, thioredoxin or maltose binding proteins (MBP), CAT, GFP, YFP, etc. The person skilled in the art will know a vast number of tags suitable for different technical applications. The tag may for example make such tagged polypeptide suitable for e.g. antibody binding in different ELISA assay formats or other technical applications.

The term "comprising" as used herein shall not be construed as being limited to the meaning "consisting of" (i.e. excluding the presence of additional other matter). Rather, "comprising" implies that optionally additional matter may be present. The term "comprising" encompasses as particularly envisioned embodiments falling within its scope "consisting of" (i.e. excluding the presence of additional other matter) and "comprising but not consisting of" (i.e. requiring the presence of additional other matter), with the former being more preferred.

The polypeptide according to the present invention may exhibit in the amino acid sequence exhibiting at least 90% sequence identity with the sequence of SEQ ID NO: 1 at least one (e.g. 1, 2, 3, 4, 5, 6, 7, 8, 9, 10, 11, 12, 13, 14, 15, 16 or even all 17) of the following: X14 is not C; X23 is not C; X50 is not C; X82 is I; X122 is M; X149 is P; X154 is T, X160 is T; X167 is L; X179 is F; X180 is E; X186 is Y; X206 is N or V, X212 is N; X224 is Q; X230 is Y and/or X232 is T. It is understood that the number indicating the position of the respective amino acid residue indicates the relative position in the sequence corresponding to SEQ ID NO: 1, and not to the overall amino acid sequence of the polypeptide according to the present invention, which may be longer.

The inventive polypeptide exhibits said at least 90% sequence identity. The inventive polypeptide may thus for example exhibit a higher level of sequence identity, e.g. may exhibit at least about 95%, at least about 96%, at least about 97%, at least about 98%, at least about 98,5%, at least about 99% (e.g. less than 3 amino acids deviation), at least about 99,3% (e.g. less than 2 amino acids deviation), at least about 99,5%, at least about 99,6% or even 100% sequence identity with the sequence of SEQ ID NO: 1.

An inventive polypeptide comprising a sequence sharing a given level of sequence identity with the sequence of SEQ ID NO: 1 (or more specific sequences thereof, see below) can for example deviate from the reference sequence by addition, substitution, insertion or deletion of one or more amino acid residues and all possible combinations thereof. Only for the sake of clarity it is pointed out that such combinations refer to distinct positions in the sequence. A "deletion" followed by "addition", or "addition" followed by "deletion", of one or more amino acids, at the same relative position, is not a combination of an "addition" and "deletion" (or vice versa) but falls under the term "substitution". Preferably, the deviations in sequence from the sequence of SEQ ID NO: 1 (or more specific sequences thereof, see below) will be of conservative nature, e.g. conservative substitutions. Even more preferably the deviation in sequence is limited to those positions in SEQ ID NO: 1 (or more specific sequences thereof, see below), which have been identified to be non-critical for the enzymatic activity, i.e. X1, X14, X23, X50, X82, X122, X149; X160, X167, X179, X180, X186; X206; X212; X224; X230 and/or X232.

Preferably, the polypeptide according to the present invention exhibits in the amino acid sequence exhibiting at least 90% sequence identity with the sequence of SEQ ID NO: 1 a glutamic acid residue at position 115. As shown in the publication Briers et al. (Molecular Microbiology (2007) 65(5), 1334-1344), the mutation E115A led to a loss in activity of about 70% of the enzyme. Thus, while an inventive polypeptide comprising said mutation will thus not be a loss of function polypeptide and may still serve various technical purposes, it is certainly preferred if such mutation is not present in the sequence stretch corresponding to SEQ ID NO: 1 within the inventive polypeptide.

In a particular preferred embodiment according to the present invention the polypeptide of the present invention comprises the sequence of SEQ ID NO: 1.

The inventors of the present invention have found out that three cysteine residues in the amino acid sequence of SEQ ID NO: 5 (KZ144 endolysin sequence) are not essential for the enzymatic activity. In principle said amino acid residues X14, X23 and X50 can be deleted or substituted by any other amino acid, provided at least one of X14, X23 and X50 is S. Examples for such other amino acids are S, R and N. Thus, X14 may for example be S, N, or R; more preferably S or R; most preferably R; X23 may for example be S, N, or R, more preferably S; and X50 may for example be S, N, or R, more preferably S or N; most preferably N. X14, X23 and X50 may of course exhibit different amino acid substitutions, for example X14 may be R while X23 and X50 are S; or X14 and X23 are S, while X50 is N; X14 may be R while X23 is S and X50 is N etc.. Any other combination conceivable is also contemplated by the present invention. Conservative amino acid substitutions are preferred. Particularly preferred is a substitute of a serine residue for the cysteine residue. Of course, it is also possible that X14 and X23 are S, or that X14 and X50 are S, or that X23 and X50 are S. X14, X23 and X50 may also all three be S. Absence of one or more or even of all of these cysteine residue has the advantage that the risk of aggregation of the polypeptide according to the present invention, e.g. by undesired disulfide bridge formation, is reduced.

Aside of the dispensability of the above referenced cysteine residues, the inventors of the present invention have also elucidated that various other residues in the sequence of SEQ ID NO: 5 are also not essential and, moreover, may be replaced by other residues, thereby increasing for instance the temperature stability of the inventive polypeptide. Examples for such substitutions are X82I, X122M, X149P; X154T, X160T, X167L, X179F, X180E, X186Y, X206V, X206N, X212N, X230Y and X232T. These substitutions may be present alone or in any combination. A typical combination is the combination of X122M and X160T. Other examples of combinations are, without being limited thereto, X82I, X206V plus X232T; X82I, X122M, X160T, X206V, plus X232T; X82I, X122M, X160T, X206N, plus X232T; X82I, X122M, X206V, plus X232T; X82I, X122M, X149P, X160T, X206V, plus X232T; X82I, X122M, X160T, X180E, X206V, plus X232T; X82I, X122M, X160T, X186Y, X206V, plus X232T; X82I, X122M, X160T, X206V, X230Y, plus X232T; X82I, X122M, X149P, X206V, plus X232T; X82I, X122M, X149P, X160T, X206V, plus X232T; X82I, X122M, X149P, X206V, plus X232T; X82I, X122M, X149P, X167L, X206V, plus X232T; X82I, X122M, X149P, X179F, X206V, plus X232T; X82I, X122M, X149P, X206V, X212N plus X232T; X82I, X122M, X149P, X206V, X224Q plus X232T; X82I, X122M, X149P, X154T, X206V, plus X232T etc.. Of course, this second type of amino acid modifications may be combined with the above mentioned cysteine replacements in any type of combination conceivable. Examples of such combinations are, without being limited thereto, X14S, X50S, X122M and X160T; X14S, X50S, X82I, X122M, X160T, X206V, and X232T; X14S, X50S, X82I, X122M, X160T, X206N, and X232T; X14S, X50S, X82I, X122M, X206V, and X232T; X14S, X50S, X82I, X122M, X149P, X160T, X206V, and X232T; X14S, X50S, X82I, X122M, X160T, X180E, X206V, and X232T; X14S, X50S, X82I, X122M, X160T, X186Y, X206V, and X232T; X14S, X50S, X82I, X122M, X160T, X206V, X230Y, and X232T; X14R, X50S, X82I, X122M, X160T, X206V, and X232T; X14S, X50N, X82I, X122M, X160T, X206V, and X232T; X14R, X50S, X82I, X122M, X149P, X206V, and X232T; X14R, X50S, X82I, X122M, X149P, X160T, X206V, and X232T; X14R, X50N, X82I, X122M, X149P, X206V, and X232T; X14R, X50N, X82I, X122M, X149P, X167L, X206V, and X232T; X14R, X50N, X82I, X122M, X149P, X179F, X206V, and X232T; X14R, X50N, X82I, X122M, X149P, X206V, X212N, and X232T; X14R, X50N, X82I, X122M, X149P, X206V, X224Q and X232T; X14R, X50N, X82I, X122M, X149P, X154T, X206V, and X232T; etc..

In SEQ ID NO: 1 (consensus sequence of the present invention) the first amino acid residue is indicated as being either absent or any amino acid, in particular M. The results of the inventors, and of previous work (see WO 2010/149792) show, that the N-terminal methionine of KZ144 is dispensable. Thus, in some embodiments of the present invention the position of X1 in the sequence corresponding to SEQ ID NO: 1 in the inventive polypeptide is not M. If the polypeptide of the present invention exhibits for example N-terminally of the sequence corresponding to SEQ ID NO: 1 further sequence elements, it may for instance for the purpose of effective expression in a host cell be useful, if the methionine at position 1 of SEQ ID NO: 1 is eliminated or replaced by another amino acid in order to avoid a starting codon in the corresponding nucleic acid sequence, potentially leading to parallel expression of a polypeptide lacking the further sequence elements located more N-terminally. On the other hand, if there are no further N-terminal sequence elements in the inventive polypeptide, X1 is of course preferably methionine (e.g. for expression purposes). For the enzymatic activity X1 is however never required.

Sequences falling under the definition of SEQ ID NO: 1, which have been particularly tested by the inventors, are for instance SEQ ID NOs: 6-27 (and corresponding sequences without N-terminal methionine, SEQ ID NOs: 28-49).

It is understood that everything which has been set forth so far in terms of the generic sequence SEQ ID NO: 1 applies in similar manner also to more specific sequences. Thus, and only for the sake of clarity it is pointed out, that a polypeptide according to the present invention, comprising a sequence exhibiting at least 90% sequence identity with the generic sequence of SEQ ID NO: 1 as set out above, may in preferred embodiments certainly exhibit in analogous manner at least 90% sequence identity with more specific sequences of SEQ ID NO: 1 described or even particularly disclosed herein. Thus, in preferred embodiments of the present invention, the polypeptide of the present invention may for example comprise a sequence exhibiting at least 90% sequence identity with a sequence selected from any of SEQ ID NOs: 6, 9-28, 31-49, wherein the polypeptide does neither comprise the amino acid sequence of SEQ ID NO: 2, nor of SEQ ID NO: 3, nor of SEQ ID NO: 4.

The polypeptide according to the present invention may comprise aside of the enzymatic amino acid sequence, e.g. the sequence exhibiting at least 90% sequence identity with the sequence of SEQ ID NO: 1 (or other sequences falling under these definition), further amino acid sequence stretches, e.g. as already disclosed in similar fashion in WO 2010/149792. The polypeptide according to the present invention may for example comprise additionally at least one amino acid sequence stretch selected from the group consisting of amphiphatic peptide, cationic peptide, polycationic peptide, hydrophobic peptide, or naturally occurring antimicrobial peptide, like sushi peptide and defensin. Such additional amino acid sequence stretches may improve the antibacterial properties of the inventive polypeptide. In some embodiments, the inventive polypeptide may comprise at least two distinct amino acid sequence stretches selected from the group of amphiphatic peptide, cationic peptide, polycationic peptide, hydrophobic peptide, or naturally occurring antimicrobial peptide, like sushi peptide and defensin.

These one or more additional amino acid sequence stretches may be present N-terminally or C-terminally of the sequence exhibiting at least 90% sequence identity with the sequence of SEQ ID NO: 1. They may for example be located at the N- or C-terminus of the inventive polypeptide. Preferred examples of such additional amino acid sequence stretches (without being limited thereto), are the sequence KRK and SEQ ID NOs: 50-120, as set out in more detail below. The polypeptide according to the present invention may comprise at least one additional amino acid sequence stretch selected from this group. For further guidance, in particular with respect to the generic and specific nature of possible additional amino acid sequence stretches, see for example also WO 2010/023207, WO 2010/149792, WO 2010/149795 and WO 2012/085259.

Examples for cationic and polycationic amino acid sequence stretches are listed in the following table.

**Table 1:**

| **amino acid sequence stretch** | **length** | **SEQ ID NO:** |
|---|---|---|
| KRKKRK | 6 | SEQ ID NO: 50 |
| KRXKR | 5 | SEQ ID NO: 51 |
| KRSKR | 5 | SEQ ID NO: 52 |
| KRGSG | 5 | SEQ ID NO: 53 |
| KRKKRKKRK | 9 | SEQ ID NO: 54 |
| RRRRRRRRR | 9 | SEQ ID NO: 55 |
| KKKKKKKK | 8 | SEQ ID NO: 56 |
| KRKKRKKRKK | 10 | SEQ ID NO: 57 |
| KRKKRKKRKKRK | 12 | SEQ ID NO: 58 |
| KRKKRKKRKKRKKR | 14 | SEQ ID NO: 59 |
| KKKKKKKKKKKKKKKK | 16 | SEQ ID NO: 60 |
| KRKKRKKRKKRKKRKKRK | 18 | SEQ ID NO: 61 |
| KRKKRKKRKKRKKRKKRKK | 19 | SEQ ID NO: 62 |
| RRRRRRRRRRRRRRRRRRR | 19 | SEQ ID NO: 63 |
| KKKKKKKKKKKKKKKKKKK | 19 | SEQ ID NO: 64 |
| KRKKRKKRKRSKRKKRKKRK | 20 | SEQ ID NO: 65 |
| KRKKRKKRKRSKRKKRKKRKK | 21 | SEQ ID NO: 66 |
| KRKKRKKRKKRKKRKKRKKRK | 21 | SEQ ID NO: 67 |
| KRKKRKKRKRGSGKRKKRKKRK | 22 | SEQ ID NO: 68 |
| KRKKRKKRKRGSGSGKRKKRKKRK | 24 | SEQ ID NO: 69 |
| KRKKRKKRKKRKKRKKRKKRKKRKK | 25 | SEQ ID NO: 70 |
| KRKKRKKRKRSKRKKRKKRKRSKRKKRKKRK | 31 | SEQ ID NO: 71 |
| KRKKRKKRKRGSGSGKRKKRKKRKGSGSGKRKKRKKRK | 38 | SEQ ID NO: 72 |
| KRKKRKKRKKRKKRKKRKKRKKRKKRKKRKKRKKRKKRK | 39 | SEQ ID NO: 73 |
| KRKKRKKRKRSKRKKRKKRKRSKRKKRKKRKRSKRKKRKKRK | 42 | SEQ ID NO: 74 |

Examples for antimicrobial amino acid sequences which may be used in carrying out the present invention are listed in the following table.

**Table 2:**

| **Peptide** | **Sequence** | **SEQ ID NO** |
|---|---|---|
| LL-37 | LLGDFFRKSKEKIGKEFKRIVQRIKDFLRNLVPRTES | SEQ ID NO: 75 |
| SMAP-29 | RGLRRLGRKIAHGVKKYGPTVLRIIRIAG | SEQ ID NO: 76 |
| Indolicidin | ILPWKWPWWPWRR | SEQ ID NO: 77 |
| Protegrin | RGGRLCYCRRRFCVCVGR | SEQ ID NO: 78 |
| Cecropin P1 | SWLSKTAKKLENSAKKRISEGIAIAIQGGPR | SEQ ID NO: 79 |
| Magainin | GIGKFLHSAKKFGKAFVGEIMNS | SEQ ID NO: 80 |
| Pleurocidin | GWGSFFKKAAHVGKHVGKAALTHYL | SEQ ID NO: 81 |
| Cecropin A (A.aegypti) | GGLKKLGKKLEGAGKRVFNAAEKALPVVAGAKALRK | SEQ ID NO: 82 |
| Cecropin A (D. melanogaster) | | SEQ ID NO: 83 |
| Buforin II | TRSSRAGLQFPVGRVHRLLRK | SEQ ID NO: 84 |
| Sarcotoxin IA | | SEQ ID NO: 85 |
| Apidaecin | ANRPVYIPPPRPPHPRL | SEQ ID NO: 86 |
| Ascaphine 5 | GIKDWIKGAAKKLIKTVASHIANQ | SEQ ID NO: 87 |
| Nigrocine 2 | GLLSKVLGVGKKVLCGVSGLVC | SEQ ID NO: 88 |
| Pseudin 1 | GLNTLKKVFQGLHEAIKLINNHVQ | SEQ ID NO: 89 |
| Ranalexin | FLGGLIVPAMICAVTKKC | SEQ ID NO: 90 |
| Melittin | GIGAVLKVLTTGLPALISWIKRKRQQ | SEQ ID NO: 91 |
| Lycotoxin 1 | IWLTALKFLGKHAAKKLAKQQLSKL | SEQ ID NO: 92 |
| Parasin 1 | KGRGKQGGKVRAKAKTRSS | SEQ ID NO: 93 |
| Buforin I | | SEQ ID NO: 94 |
| Dermaseptin 1 | ALWKTMLKKLGTMALHAGKAALGAAADTISQGTQ | SEQ ID NO: 95 |
| Bactenecin 1 | RLCRIVVIRVCR | SEQ ID NO: 96 |
| Thanatin | GSKKPVPIIYCNRRTGKCQRM | SEQ ID NO: 97 |
| Brevinin 1T | VNPIILGVLPKVCLITKKC | SEQ ID NO: 98 |
| Ranateurin 1 | SMLSVLKNLGKVGLGFVACKINIKQC | SEQ ID NO: 99 |
| Esculentin 1 | | SEQ ID NO: 100 |
| Tachyplesin | RWCFRVCYRGICYRKCR | SEQ ID NO: 101 |
| Androctonin | RSVCRQIKICRRRGGCYYKCTNRPY | SEQ ID NO: 102 |
| alpha-defensin | DCYCRIPACIAGERRYGTCIYQGRLWAFCC | SEQ ID NO: 103 |
| beta-defensin | NPVSCVRNKGICVPIRCPGSMKQIGTCVGRAVKCCRKK | SEQ ID NO: 104 |
| theta-defensin | GFCRCLCRRGVCRCICTR | SEQ ID NO: 105 |
| defensin (sapecin A) | | SEQ ID NO: 106 |
| Thionin (crambin) | | SEQ ID NO: 107 |
| defensin from radish | | SEQ ID NO: 108 |
| Drosomycin | | SEQ ID NO: 109 |
| Hepcidin | DTHFPICIFCCGCCHRSKCGMCCKT | SEQ ID NO: 110 |
| Bac 5 | | SEQ ID NO: 111 |
| PR-39 | | SEQ ID NO: 112 |
| Pyrrhocoricin | VDKGSYLPRPTPPRPIYNRN | SEQ ID NO: 113 |
| Histatin 5 | DSHAKRHHGYKRKFHEKHHSHRGY | SEQ ID NO: 114 |

The at least one additional amino acid sequence stretch may be a sushi peptide which is described by Ding JL, Li P, Ho B Cell Mol Life Sci. 2008 Apr;65(7-8):1202-19. The Sushi peptides: structural characterization and mode of action against Gram-negative bacteria. Especially preferred is the sushi 1 peptide according to SEQ ID NO: 115. Other preferred sushi peptides are sushi peptides S1 and S3 and multiples thereof; FASEB J. 2000 Sep;14(12):1801-13.

Preferred hydrophobic peptides are Walmagh1 having the amino acid sequence according to SEQ ID NO: 116 and the hydrophobic peptide having the amino acid sequence Phe-Phe-Val-Ala-Pro (SEQ ID NO: 117).

Preferred amphiphatic peptides are α4-helix of T4 lysozyme according to SEQ ID NO: 118 and WLBU2-Variant having the amino acid sequence according to SEQ ID NO: 119 and Walmagh 2 according to SEQ ID NO: 120.

As mentioned above, a polypeptide according to the present invention may comprise at least one additional amino acid sequence stretch selected from the group consisting of: KRK and SEQ ID NOs: 50-120. Corresponding examples are for instance polypeptides comprising a sequence selected from the group consisting of SEQ ID NOs: 121 and 124-127 (and corresponding sequences without N-terminal methionine, SEQ ID NOs: 128 and 131-134.

A polypeptide according to the present invention comprises an amino acid sequence exhibiting at least 90% sequence identity with the sequence of SEQ ID NO: 1, wherein the polypeptide does neither comprise the amino acid sequence of SEQ ID NO: 2, nor of SEQ ID NO: 3, nor of SEQ ID NO: 4. Thus, a polypeptide of the present invention may also comprise an amino acid sequence exhibiting at least 91,5 % sequence identity with an amino acid sequence selected from any of SEQ ID NOs: 121, 124-128, and 131 - 134, wherein the polypeptide does neither comprise the amino acid sequence of SEQ ID NO: 2, nor of SEQ ID NO: 3, nor of SEQ ID NO: 4.

Such inventive polypeptide may thus for example comprise a sequence exhibiting a higher level of sequence identity than 91,5% with an amino acid sequence selected from any of SEQ ID NOs: 121, 124-128, and 131 - 134, e.g. may exhibit at least about 95%, at least about 96%, at least about 97%, at least about 98%, at least about 98,5%, at least about 98,75%, at least about 99% (e.g. less than 3 amino acids deviation), at least about 99,5% (e.g. less than 2 amino acids deviation), at least about 99,6% or even 100% sequence identity with an amino acid sequence selected from any of SEQ ID NOs: 121, 124-128, and 131 - 134.

In addition, and irrespective whether or not one or more additional amino acid sequence stretches as set out above are present in the inventive polypeptide, the polypeptide may comprise additionally one or more tag sequences. Such tag sequence may be present N-terminally or C-terminally of the sequence exhibiting at least 90% sequence identity with the sequence of SEQ ID NO: 1. They may for example be located at the N- or C-terminus of the inventive polypeptide. In a preferred embodiment, the one or more tag sequence is located C-terminally of the amino acid sequence exhibiting at least 90% sequence identity with the sequence of SEQ ID NO: 1.

The one or more tag sequences may for example be linked to the amino acid sequence exhibiting at least 90% sequence identity with the sequence of SEQ ID NO: 1 directly or via a short linker of 1 to 10 amino acid residues, preferably 1 to 5 amino acid residues, even more preferably 1 to 2 amino acids. Linker sequences are preferably flexible sequences, comprising one or more glycine residues. Numerous examples for tags are known in the art, some of which have already been mentioned above. In the context of the present invention a particularly preferred tag sequence is a His-tag, preferably a His tag according to SEQ ID NO: 135.

The length of the polypeptide according to present invention is in principle not limited, but preferably the length will not be excessively large. Preferably, a polypeptide according to the present invention has an overall length not exceeding about 320 amino acids, preferably not exceeding about 310 amino acids.

Specific examples of polypeptides according to the present invention can be selected from the group consisting of SEQ ID NOs: 136, and 139-142 (and corresponding sequences without N-terminal methionine, SEQ ID NOs: 143 and 146-149).

A polypeptide according to the present invention comprises an amino acid sequence exhibiting at least 90% sequence identity with the sequence of SEQ ID NO: 1, wherein the polypeptide does neither comprise the amino acid sequence of SEQ ID NO: 2, nor of SEQ ID NO: 3, nor of SEQ ID NO: 4. Thus, a polypeptide of the present invention may also comprise an amino acid sequence exhibiting at least 91,5 % sequence identity with an amino acid sequence selected from any of SEQ ID NOs: 136, 139-143, and 146 - 149, wherein the polypeptide does neither comprise the amino acid sequence of SEQ ID NO: 2, nor of SEQ ID NO: 3, nor of SEQ ID NO: 4.

Such inventive polypeptide may thus for example comprise a sequence exhibiting a higher level of sequence identity than 91,5% with an amino acid sequence selected from any of SEQ ID NOs: 136, 139-143, and 146 - 149, e.g. may exhibit at least about 95%, at least about 96%, at least about 97%, at least about 98%, at least about 98,5%, at least about 99%, at least about 99,25% (e.g. less than 3 amino acids deviation), at least about 99,5% (e.g. less than 2 amino acids deviation), at least about 99,6% or even 100% sequence identity with an amino acid sequence selected from any of SEQ ID NOs: 136, 139-143, and 146 - 149. Deviations from SEQ ID NOs: 136, 139-143, and 146 - 149 may in particular occur in the two sequences linking the components SMAP29 peptide, modified KZ144 endolysin and His-tag.

A polypeptide according to the present invention is preferably characterized by the ability to degrade the peptidoglycan of Gram-negative bacteria, in particular of *Pseudomonas* and/or *Campylobacter* bacteria. In particular, the polypeptide according to the present invention is preferably capable of degrading the peptidoglycan of *Pseudomonas aeroginosa,* in particular *Pseudomonas aeroginosa* PAO1, *Campylobacter jejuni* and/or *Campylobacter coli.*

The peptidoglycan degrading activity on gram negative bacteria can be measured by assays well known in the art, e.g. by muralytic assays in which the outer membrane of gram negative bacteria is permeabilized or removed (e.g. with chloroform) to allow the putative enzyme access to the peptidoglycan layer. If the enzyme is active, degradation of the peptidoglycan layer will lead to a drop of turbidity, which can be measured photometrically (see for example Briers et al., J. Biochem. Biophys Methods 70: 531-533, (2007*).*

In a further aspect the present invention relates to a nucleic acid encoding a polypeptide according to the present invention. A person skilled in the art, having the degeneracy of the genetic code in mind, will be aware of means to generate such nucleic acid.

In a further aspect, the present invention relates to a vector, such as an expression or cloning vector, which comprises a nucleic acid according to the present invention.

In a further aspect, the present invention relates to a host cell comprising a polypeptide according to the present invention, a nucleic acid according to the present invention, and/or a vector according to the present invention.

In a further aspect, the present invention relates to composition comprising a polypeptide according to the present invention, a nucleic acid according to the present invention, a vector according to the present invention, and/or a host cell according to the present invention. Preferably, said composition is a pharmaceutical composition comprising a pharmaceutical acceptable diluent, excipient or carrier. about 90% sequence identity with the sequence of SEQ ID NO: 1, wherein the polypeptide does neither comprise the amino acid sequence of SEQ ID NO: 2, nor of SEQ ID NO: 152, nor of SEQ ID NO: 4. Optionally, said polypeptide of this additional aspect does also not comprise the amino acid sequence of SEQ ID NO: 3. All embodiments and combinations disclosed above, in the examples or in the claims for the polypeptide of the invention, and respective nucleic acids, vectors, host cells and compositions, are specifically contemplated for this further aspect as well.

### Examples

In the following, specific examples illustrating various embodiments and aspects of the invention are presented. However, the present invention shall not to be limited in scope by the specific embodiments described herein. Indeed, various modifications of the invention in addition to those described herein will become readily apparent to those skilled in the art from the foregoing description, accompanying figures and the examples below. All such modifications fall within the scope of the appended claims.

### Example 1: Identification of mutations stabilizing KZ144 endolysin

For identification of advantageous sites of modification in endolysin KZ144 (SEQ ID NO: 5), the inventors used in a first step targeted destabilization of the target protein. For this purpose an N-terminally truncated KZ144 was generated (SEQ ID NO: 150) into which sequence mutations were introduced via random mutagenesis (error-prone PCR) followed by subsequent fusion and selection with a chloramphenicol assay (CAT assay).

The protein melting temperature of promising candidates was determined by circular dichroism (CD). Changes of ellipticity for the proteins were recorded at 220nm as a function of temperature using Jasco J-815 CD spectrometer and fitted to a simple sigmoid unfolding model using JASCO analysis software. The protein melting temperatures (Tmelt) were determined as midpoint of unfolding transition. The spectra were recorded at protein concentrations of 5.0-5.8µM with a heating rate of 1°C/min and incubation time of 3s in 410µl volume in a 1mm light path Hellma quartz cuvette. Measurements were performed in 50mM NaPh buffer, 300mM NaCl at pH of 7.4, 7.0, 6.2 and 5.7.

Some of the most promising candidates identified are illustrated in the following table:

**Table 3**

| **AA-Substitution*** | **Solubility** | **T_{M}** | **ΔT_{M}** |
|---|---|---|---|
| --- | + | 53,5 | 0 |
| S232T | + | 54,1 | + 0,6 |
| A206V | + | 55,8 | + 2,3 |
| T82I- | + | 54,3 | + 0,8 |
| I122M A160T | + | 56,5 | + 3,0 |

| | | | |
|---|---|---|---|
| * Please note, the position indicated refers to the position in the sequence of KZ144 sequence, SEQ ID NO: 5, and not to the position in SEQ ID NO: 150. | | | |

The thus identified stabilizing mutations can and were subsequently introduced into other sequences such as full length sequences, increasing stability there as well.

In a further step serine was used in some constructs for substitution of cysteine residues C14, C23 and/or C50 (position indicated with respect to SEQ ID NO: 5; conservative substitutions). Other substituents at said positions tested were N and R.

In a further round of experiments, various combinations of mutations were tested in the context of the full length endolysin KZ144 (SEQ ID NO: 5):

**Table 4**

| **AA-Substitution** | **#of mutations** | **ø T_{M}** | **Δ T_{M}*** |
|---|---|---|---|
| C14S C50S T82II122M A160T A206V S232T | 7 | 57,6 | +4,4 |
| C14S C50S T82I I122M A160T A206V N230Y S232T | 8 | 57,8 | +4,6 |
| C14S C50S T82I I122M A160T M180E A206V S232T | 8 | 57,7 | +4,5 |
| C14S C50S T82I I122M M149P A160T A206V S232T | 8 | 58,9 | +5,7 |
| C14S C50S T82I I122M A160T V186Y A206V S232T | 8 | 56,7 | +3,5 |
| C14R C50S T82I I122M A160T A206V S232T | 7 | 59,9 | +6,7 |
| C14S C50S T82I I122M T160A A206V S232T | 7 | 58,3 | +5,1 |
| C14S C50N T82I I122M A160T A206V S232T | 7 | 58,5 | +5,3 |
| C14R C50S T82I I122M M149P A160T A206V S232T | 8 | 61 | +7,9 |
| C14R C50S T82II122M A206V S232T M149P | 7 | 61,7 | +8,5 |
| C14R C50N T82I I122M M149P A206V S232T (comparative example) | 7 | 62,8 | +9,6 |
| C14R C50N T82I I122M M149P A206V S232T (comparative example) | 8 | 63,4 | +10,2 |
| C14R C50N T82I I122M M149P A206V S232T (comparative example) | 8 | 62,8 | +9,6 |
| C14R C50N T82I I122M M149P A206V S232T (comparative example) | 8 | 61,5 | +8,3 |
| C14R C50N T82I I122M M149P A206V S232T (comparative example) | 8 | 60,9 | +7,7 |

| | | | |
|---|---|---|---|
| * Δ TM vs. SEQ ID NO: 5 | | | |

### Example 2: Melting temperature of some polypeptides according to the present invention and MIC for selected bacterial strains

For the construction of polypeptides according to SEQ ID NO 151 (w/o mutations) and SEQ ID NOs: 136 -142 the lytic enzyme (gp144) of the Pseudomonas aeruginosa phage KZ was used. As peptide fusion partner SMAP-29 was chosen. SMAP-29 was found in sheep leukocytes and consists of 29 amino acids (RGLRRLGRKIAHGVKKYGPTVLRIIRIAG; molecular weight: 3.3 kDa, SEQ ID NO: 76). It is built up of two LPS-binding sites which are connected by a central hinge.

### Cloning

The nucleic acid molecules encoding the respective peptide and endolysin were constructed with a NdeI (5'-CAT ATG-3') restriction site at the 5'-end of the nucleic acid molecule and a XhoI (5'-CTC GAG-3') restriction site at the 3'-end of the nucleic acid molecule. Peptide and endolysin are connected via a BamHI (5'-GGA TCC-3').

Fusion proteins were constructed by linking at least two nucleic acid sequences using standard cloning techniques as described e.g. by Sambrook et al. 2001, Molecular Cloning: A Laboratory Manual. Therefore the nucleic acid molecule encoding the peptide stretch was cleaved in a digest with the respective restriction enzymes NdeI and BamHI. Subsequently the cleaved nucleic acids encoding the peptide stretch was ligated into the pET21 b expression vector (Novagen, Darmstadt, Germany), which was also cleaved in a digest with the respective restriction enzymes NdeI and BamHI before. Afterwards, the nucleic acid molecule encoding the endolysin was cleaved in a digest with the restriction enzyme BamHI and XhoI, so that the endolysin could be ligated into the pET21b expression vector (Novagen, Darmstadt, Germany), which was also cleaved in a digest with the respective restriction enzymes BamHI and XhoI before.

The sequence of the peptide-endolysin fusions was controlled via DNA sequencing and correct clones were transformed into *E.coli* BL21(DE3)pLysS (Novagen, Darmstadt, Germany) for protein expression.

### Purification

Recombinant expression of the fusion proteins was done in *E. coli* BL21(DE3)pLysS cells (Novagen, Darmstadt, Germany). The cells were grown until an optical density of OD₆₀₀ₙₘ = 0.5-0.8 was reached. Then the expression of the fusion protein was induced with 0.5 mM IPTG (isopropylthiogalactoside) and the expression was performed at 37°C for 4 h.

Cells were harvested by centrifugation for 20 min at 6000g and disrupted via sonication on ice. Soluble and insoluble fraction of the *E.coli* crude extract were separated by centrifugation (Sorvall, SS34, 30 min, 15 000 rpm). All proteins were purified by Ni²⁺ affinity chromatography (Äkta FPLC, GE Healthcare) using the C-terminal His₆ tag, encoded by the pET21b vector. Samples were microfiltrated (0.2 µm) before every chromatographic step.

The Ni²⁺ affinity chromatography is performed in 4 subsequent steps, all at room temperature:
*1. Equilibration* of the Histrap FF 5 ml column (GE Healthcare) with up to 10 column volumes of Washing Buffer (20mM imidazole, 1M NaCl and 20mM HEPES on pH 7.4) at a flow rate of 3-5 ml/min.
2. *Loading* of the total lysate with wanted target protein on the Histrap FF 5 ml column at a flow rate of 3-5 ml/min.
3. *Washing* of the column with up to 10 column volumes of Washing Buffer to remove unbound protein.
4. *Elution* of bounded target protein from the column with an increasing linear gradient of 15 column volumes of Elution Buffer (500mM imidazole, 500mM NaCl and 20mM HEPES on pH 7.4) to 100% at a flow rate of 3-5 ml/min.

The *Hydrophobic Interaction Chromatography* (HIC) is performed in 5 subsequent steps, all at room temperature:
*1. Equilibration* of the HiScreen Phenyl HP 5 ml column (GE Healthcare) with up to 5 column volumes of Washing Buffer (850mM ammonium sulfate, 500mM NaCl and 20mM HEPES on pH 7.4) at a flow rate of 1-2ml/min
*2. Preparation* of the sample (5mg per 1ml column volume of the protein pool from Ni²⁺ affinity step) starts by first setting the protein concentration to 0.5mg/ml by adding a predefined amount of Washing Buffer from the Ni²⁺ Affinity step. Followed by adjusting the ammonium sulfate concentration by stepwise adding of a predefined amount of ammonium sulfate stock solution (3.8M) to a final concentration of approx. 850mM.
3. *Loading* of the prepared sample on the HiScreen Phenyl HP 5 ml column at a flowrate of 1-2ml/min.
4. *Washing* of the column with 5 column volumes of Washing Buffer to remove unbound protein.
5. *Elution* of the target protein from the column with a step of 40% Elution Buffer (500mM NaCl and 20mM HEPES on pH 7.4) at a flow rate of 1-2 ml/min. The target protein is eluted in broad peak at this step.

### Buffer change by membrane dialysis:

The elution pool of the HIC step is dialyzed (membrane: regenerated cellulose with MWCO: 6000-8000D) into storage buffer (500 mM NaCl and 20mM HEPES; pH7.4) at 4°C. Dialysis factor is 160 - 250.

### Characterisation

Melting temperatures characterizing stability of the polypeptides according to SEQ ID NOs: 136 -142 at elevated temperatures were determined by circular dichroism spectroscopy (CD) as mentioned above.

Activity of polypeptides according to SEQ ID NOs: 136 -142 on P. aeroginasa, C. jejuni and C. coli was characterised by determination of minimal inhibitory concentration (MIC) on the respective strains.

### Determination of the Minimal Inhibitory Concentration (MIC)

In analogy to the determination of the "Minimum inhibitory concentration (MIC)" for antibiotics, the MIC was determined as a microdilution test. The test on Campylobacter species is performed completely at microaerophilic conditions and 42°C.

The setup of the experiment is the following:
The respective overnight culture was diluted 1:10. *Ps. aeruginosa* was incubated at 37°C up to OD600=0.6 (approx. 10⁹ cells/ml). *Campylobacter sp.* were incubated microaerophilic up to OD600=0.08 (approx. 2,5x10⁸ cells/ml). The bacterial culture was diluted to a concentration of 2x10⁵ to 8x10⁵ colony-forming-units per ml in Mueller-Hinton-broth (not cation-adjusted Mueller-Hinton-broth) and split in the required amount of tubes.

The polypeptide of interest was added in different concentrations (determined as µg/ml final concentration in the Mueller-Hinton-broth). In case of *Ps. aeruginosa* EDTA was added to a final concentration of 2 mM. In case of *Campylobacter sp* no EDTA was used.

The mixture was incubated overnight at 37°C for *Ps. Aeruginosa* and at 42°C for *Campylobacter* species. Bacterial growth was visibly determined by turbidity (in comparison to negative control). The MIC was defined as the concentration in the tube where no bacterial growth was observed. Positive (without polypeptide of interest and/or EDTA) and negative control (Mueller-Hinton-broth without bacteria) were included in the experiment.

The results are summarized in the following table:

**Table 5**

| | | | | **MIC on PAO1p S82 [µg/ml]** | **MIC on Camp. jejuni S371 [µg/ml]** | **MIC on Camp. coli S344 [µg/ml]** |
|---|---|---|---|---|---|---|
| **SEQ ID NO:** | **Mutations*** | **Concentration [by UV]** | **Tm [°C]** | **500 µM EDTA** | **no EDTA** | **no EDTA** |
| 151 (compara tive example) | | 5,5 µM | 44,14 | **5** | **5** | **4** |
| 136 | C14S C50S | 5,2 µM | 49,07 | **5** | **5** | **4** |
| 137 (compara tive example) | T82I A206V S232T | 5,4 µM | 45,4 | **5** | **4** | **3** |
| 138 (compara tive example) | T82I A206V S232T I122M A160T | 5,5 µM | 47,57 | **7** | **7** | **4** |
| 139 | C14S C50S I122M A160T | 5,6 µM | 51,42 | **3-4** | **3** | **4** |
| 140 | C14S C23S C50S | 5,3 µM | 50,31 | **5** | **3-4** | **2** |
| 141 | T82I A206V S232T I122M A160T C14S C50S | 5,9 µM | 51,68 | **3-6** | **5-7** | **5** |
| 142 | T82I A206N S232T I122M A160T C14S | 5,3 µM | 50,64 | **3** | **5-7** | **5** |
| | C50S | | | | | |

| | | | | | | |
|---|---|---|---|---|---|---|
| Melting temperatures measured by CD, buffer: 50mM NaPh, pH 7.45 300mM NaCl | | | | | | |

Please note again, that the position indicated refers to the position in the sequence of KZ144 sequence, SEQ ID NO: 5, and not to the position of the SEQ ID NO: indicated in the table.

The mutations introduced did thus not only increase melting temperature of the polypeptides of SEQ ID NOs: 136 -142 vs the polypeptide of SEQ ID NO: 151, but did also not affect activity of the polypeptide, not even the sevenfold mutation of SEQ ID NO: 141.

### Example 3: Temperature stability of the polypeptide according to SEQ ID NO: 139 and SEQ ID NO: 141

In order to illustrate that the increased melting temperature does indeed affect temperature stability of the respective mutated polypeptides, the inventors exposed exemplarily the mutated polypeptides of SEQ ID NO: 139 and SEQ ID NO: 141 to temperatures clearly exceeding the melting temperature of the native, non-mutated reference polypeptide (SEQ ID NO: 151).

For this purpose both polypeptides were subjected to prolonged direct heating at temperatures of 51°C and 52°C. Subsequently, an activity test was performed on *Pseudomonas aeruginosa* strain as a model system at adapted conditions.

The results are summarized in the following table:

**Table 6**

| **SEQ ID NO:** | **Mutations** | **Concentration [by UV]** | **Heating** | **MIC on PAO1p S82 [µg/ml] 500 µM EDTA** |
|---|---|---|---|---|
| 139 | C14S C50S I122M A160T | | 0 | **3-4** |
| | | 5,3 µM | 1min 51°C | **12,5** |
| | | | 2min 51°C | **15** |
| | | | 2min 52°C | **17,6** |
| 141 | T82I A206V S232T I122M A160T C14S C50S | | 0 | **3-6** |
| | | 5,45 µM | 1min 51°C | **8** |
| | | | 2min 51°C | **10** |
| | | | 2min 52°C | **12,5** |

| | | | | |
|---|---|---|---|---|
| Protein Buffer:50mM NaPh, pH 7.45 300mM NaCl | | | | |

As previously, the position indicated refers to the position within the sequence portion corresponding to the KZ144 sequence, SEQ ID NO: 5, and not to the position within the full-length sequence of the SEQ ID NO: indicated in the table.

## Claims

1. Polypeptide comprising an amino acid sequence, said amino acid sequence exhibiting
i) at least 90% sequence identity with the sequence of SEQ ID NO: 1, wherein residue X1 of SEQ ID NO: 1 may be absent or any amino acid, and each residue of residues X14, X23, X50, X82, X122, X149, X154, X160, X167, X179, X180, X186, X206, X212, X224, X230, and X232 of SEQ ID NO: 1 may be any amino acid; and
ii) at least one of the residues corresponding to position X14, X23 and X50 of SEQ ID NO: 1 is S;
wherein the polypeptide does neither comprise the amino acid sequence of SEQ ID NO: 2, nor SEQ ID NO:3 nor SEQ ID NO: 4, and wherein the polypeptide degrades the peptidoglycan of Pseudomonas and/or Campylobacter bacteria.

2. Polypeptide according to claim 1, wherein:
X14 is S, R or N,
X23 is S, R or N,
X50 is S, R or N,
X82 is T or I
X122 is I or M
X149 is M or P
X154 is L or T
X160 is A or T
X167 is I or L
X179 is N or F
X180 is M or E
X186 is V or Y
X206 is A, N or V
X212 is T or N
X224 is P or Q
X230 is N or Y
X232 is S or T.

3. The polypeptide according to any one of the preceding claims, wherein the sequence exhibiting at least 90% sequence identity with the sequence of SEQ ID NO: 1 deviates from the sequence of SEQ ID NO: 1 as defined in claim 1 or 2 only at one or more residues selected from X1, X14, X23, X50, X82, X122, X149, X154, X160, X167, X179, X180, X186, X206, X212, X224, X230 and/or X232.

4. The polypeptide according to claim 1 or 2, wherein the polypeptide exhibits in the amino acid sequence exhibiting at least 90% sequence identity with the sequence of SEQ ID NO: 1 a glutamic acid residue at position 115.

5. The polypeptide according to any one of the preceding claims, wherein SEQ ID NO: 1 is a sequence selected from the group consisting of SEQ ID NOs: 6-49.

6. The polypeptide according to any one of the preceding claims, wherein the polypeptide comprises a sequence selected from the group consisting of SEQ ID NOs: 6, 9-28, 31-49.

7. The polypeptide according to any one of the preceding claims, wherein the polypeptide comprises at least one additional amino acid sequence stretch selected from the group consisting of: KRK and SEQ ID NOs: 50 - 120.

8. The polypeptide according to claim 7, wherein the polypeptide comprises at least one additional amino acid sequence stretch having the amino acid sequence of SMAP-29 as shown in SEQ ID NO: 76.

9. The polypeptide according to any one of the preceding claims, wherein the polypeptide comprises an amino acid sequence exhibiting at least 91,5% sequence identity with an amino acid sequence selected from any of SEQ ID NOs: 121, 124-128, and 131-134, wherein the polypeptide does neither comprise the amino acid sequence of SEQ ID NO: 2, nor SEQ ID NO: 3, nor SEQ ID NO: 4.

10. The polypeptide according to any one of the preceding claims, wherein the polypeptide comprises a sequence selected from from the group consisting of SEQ ID NOs: 121, 124-128, and 131-134 or comprises a sequence selected from the group consisting of SEQ ID NOs: 136, 139-143, and 146-149.

11. Nucleic acid encoding a polypeptide according to any one of claims 1 to 10.

12. Composition comprising a polypeptide according to any one of claims 1 to 10 and/or a nucleic acid according to claim 11.

## Patentansprüche

1. Polypeptid umfassend eine Aminosäuresequenz, die Aminosäuresequenz aufweisend
i) mindestens 90% Sequenzidentität mit der Sequenz SEQ ID NO: 1, wobei Rest X1 der SEQ ID NO: 1 fehlen oder irgendeine Aminosäure sein kann, und jeder Rest der Reste X14, X23, X50, X82, X122, X149, X154, X160, X167, X179, X180, X186, X206, X212, X224, X230, und X232 von SEQ ID NO: 1 irgendeine Aminosäure sein kann; und
ii) mindestens einer der Reste korrespondierend zur Position X14, X23 und X50 der SEQ ID NO: 1 S ist;
wobei das Polypeptide weder die Aminosäuresequenz der SEQ ID NO: 2 umfasst, noch SEQ ID NO:3, noch SEQ ID NO: 4, und wobei das Polypeptid das Peptidoglykan von Pseudomonas und/oder Campylobacter Bakterien abbaut.

2. Polypeptid nach Anspruch 1, wobei:
X14 S, R oder N ist,
X23 S, R oder N ist,
X50 S, R oder N ist,
X82 T oder I ist
X122 I oder Mist
X149 M oder P ist
X154 L oder T ist
X160 A oder T ist
X167 I oder List
X179 N oder F ist
X180 M oder Eist
X186 V oder Y ist
X206 A, N oder V ist
X212 T oder N ist
X224 P oder Q ist
X230 N oder Y ist
X232 S oder T ist.

3. Das Polypeptid nach einem der vorhergehenden Ansprüche, wobei die Sequenz, die mindestens 90% Sequenzidentität mit der Sequenz der SEQ ID NO: 1 aufweist, von der Sequenz der SEQ ID NO: 1 wie in Anspruch 1 oder 2 definiert nur an einem oder mehreren Resten ausgewählt aus X1, X14, X23, X50, X82, X122, X149, X154, X160, X167, X179, X180, X186, X206, X212, X224, X230 und/oder X232 abweicht.

4. Das Polypeptid nach Anspruch 1 oder 2, wobei das Polypeptid in der Aminosäuresequenz, die mindestens 90% Sequenzidentiät mit der Sequenz der SEQ ID NO: 1 aufweist, ein Glutaminsäure-Rest an Position 115 aufweist.

5. Das Polypeptid nach einem der vorhergehenden Ansprüche, wobei SEQ ID NO: 1 eine Sequenz ausgewählt aus der Gruppe bestehend aus SEQ ID NOs: 6-49 ist.

6. Das Polypeptid nach einem der vorhergehenden Ansprüche, wobei das Polypeptid eine Sequenz ausgewählt aus der Gruppe bestehend aus SEQ ID NOs: 6, 9-28, 31-49 umfasst.

7. Das Polypeptid nach einem der vorhergehenden Ansprüche, wobei das Polypeptid mindestens einen zusätzlichen Aminosäuresequenzbereich ausgewählt aus der Gruppe bestehend aus: KRK und SEQ ID NOs: 50 - 120 umfasst.

8. Das Polypeptid nach Anspruch 7, wobei das Polypeptid mindestens einen zusätzlichen Aminosäuresequenzbereich umfasst, der die Aminosäuresequenz von SMAP-29 wie in SEQ ID NO: 76 gezeigt, hat.

9. Das Polypeptid nach einem der vorhergehenden Ansprüche, wobei das Polypeptid eine Aminosäuresequenz umfasst, die mindestens 91,5% Sequenzidentität mit einer Aminosäureseqenz ausgewählt aus einer der SEQ ID NOs: 121, 124-128 und 131-134 aufweist, wobei das Polypeptid weder die Aminosäuresequenz der SEQ ID NO: 2, noch SEQ ID NO: 3, noch SEQ ID NO: 4 umfasst.

10. Das Polypeptid nach einem der vorhergehenden Ansprüche, wobei das Polypeptid eine Sequenz ausgewählt aus der Gruppe bestehend aus SEQ ID NOs: 121, 124-128 und 131-134 umfasst oder eine Sequenz ausgewählt aus der Gruppe bestehend aus SEQ ID NOs: 136, 139-143 und 146-149 umfasst.

11. Nukleinsäure kodierend ein Polypeptid nach einem der Ansprüche 1 bis 10.

12. Zusammensetzung umfassend ein Polypeptid nach einem der Ansprüche 1 bis 10 und/oder eine Nukleinsäure nach Anspruch 11.

## Revendications

1. Polypeptide comprenant une séquence d'acides aminés, ladite séquence d'acides aminés présentant
i) une identité de séquence d'au moins 90 % avec la séquence de la SEQ ID NO : 1, dans laquelle le résidu X1 de la SEQ ID NO : 1 peut être absent ou être n'importe quel acide aminé, et chaque résidu parmi les résidus X14, X23, X50, X82, X122, X149, X154, X160, X167, X179, X180, X186, X206, X212, X224, X230 et X232 de la SEQ ID NO : 1 peut être n'importe quel acide aminé ; et
ii) au moins l'un des résidus correspondant aux positions X14, X23 et X50 de la SEQ ID NO: 1 est S ;
dans lequel ledit polypeptide ne comprend pas la séquence d'acides aminés de SEQ ID NO: 2, de SEQ ID NO : 3 et de SEQ ID NO : 4, et dans lequel ledit polypeptide dégrade le peptidoglycane de bactéries Pseudomonas et/ou Campylobacter.

2. Polypeptide selon la revendication 1, dans lequel :
X14 est S, R ou N,
X23 est S, R ou N,
X50 est S, R ou N,
X82 est T ou I,
X122 est I ou M,
X149 est M ou P,
X154 est L ou T,
X160 est A ou T,
X167 est I ou L,
X179 est N ou F,
X180 est M ou E,
X186 est V ou Y,
X206 est A, N ou V,
X212 est T ou N,
X224 est P ou Q,
X230 est N ou Y,
X232 est S ou T.

3. Polypeptide selon l'une quelconque des revendications précédentes, dans lequel la séquence présentant une identité de séquence d'au moins 90 % avec la séquence de la SEQ ID NO : 1 s'écarte de la séquence de la SEQ ID NO : 1 telle que définie dans la revendication 1 ou 2 uniquement d'un ou plusieurs résidus choisis parmi X1, X14, X23, X50, X82, X122, X149, X154, X160, X167, X179, X180, X186, X206, X212, X224, X230 et/ou X232.

4. Polypeptide selon la revendication 1 ou 2, lequel polypeptide présente, dans la séquence d'acides aminés présentant une identité de séquence d'au moins 90 % avec la séquence de la SEQ ID NO : 1, un résidu d'acide glutamique à la position 115.

5. Polypeptide selon l'une quelconque des revendications précédentes, dans lequel la SEQ ID NO : 1 est une séquence choisie dans le groupe constitué par les SEQ ID NO: 6 à 49.

6. Polypeptide selon l'une quelconque des revendications précédentes, lequel polypeptide comprend une séquence choisie dans le groupe constitué par les SEQ ID NO : 6, 9 à 28, et 31 à 49.

7. Polypeptide selon l'une quelconque des revendications précédentes, lequel polypeptide comprend au moins un étirement de séquence d'acides aminés supplémentaire choisi dans le groupe constitué par : KRK et les SEQ ID NO : 50 à 120.

8. Polypeptide selon la revendication 7, lequel polypeptide comprend au moins un étirement de séquence d'acides aminés supplémentaire ayant la séquence d'acides aminés de SMAP-29 telle qu'indiquée dans la SEQ ID NO: 76.

9. Polypeptide selon l'une quelconque des revendications précédentes, lequel polypeptide comprend une séquence d'acides aminés présentant une identité de séquence d'au moins 91,5 % avec une séquence d'acides aminés choisie parmi l'une quelconque des SEQ ID NO: 121, 124 à 128, et 131 à 134, lequel polypeptide ne comprend la séquence d'acides aminés ni de la SEQ ID NO: 2 ni de la SEQ ID NO: 3 ni de la SEQ ID NO: 4.

10. Polypeptide selon l'une quelconque des revendications précédentes, lequel polypeptide comprend une séquence choisie dans le groupe constitué par les SEQ ID NO : 121, 124 à 128, et 131 à 134, ou comprend une séquence choisie dans le groupe constitué par les SEQ ID NO: 136, 139 à 143, et 146 à 149.

11. Acide nucléique codant un polypeptide selon l'une quelconque des revendications 1 à 10.

12. Composition comprenant un polypeptide selon l'une quelconque des revendications 1 à 10 et/ou un acide nucléique selon la revendication 11.
